# EUROPEAN PATENT APPLICATION

(11) **EP 4 375 277 A1**
(43) Date of publication of application: **29.05.2024**
(21) Application number: 22845342.9
(22) Date of filing: 20.07.2022
(51) Int. Cl.: C07D 401/04, C07D 401/14, A61K 31/4439, A61P 1/16, A61P 3/00, A61P 9/00, A61P 11/00, A61P 13/00, A61P 25/00, A61P 27/00, A61P 35/00

(54) **LPA1 SMALL MOLECULE ANTAGONIST**

(30) Priority: 20.07.2021 CN 202110820727; 17.08.2021 CN 202110945201; 02.11.2021 CN 202111287042
(71) Applicant: Tuojie Biotech (Shanghai) Co., Ltd., Shanghai 201203 (CN)
(72) Inventor: LI, Yunfei, Shanghai 201203 (CN); TAN, Liang, Shanghai 201203 (CN); MO, Mingguang, Shanghai 201203 (CN); ZHANG, Zhen, Shanghai 201203 (CN); LIN, Xiaoyan, Shanghai 201203 (CN); GU, Xiaowen, Shanghai 201203 (CN); LI, Jiao, Shanghai 201203 (CN)
(74) Representative: Potter Clarkson
(86) International application number: PCT/CN2022/106706
(87) International publication number: WO 2023/001177

(57) **Abstract**

A triazolopyridine compound represented by formula I-a and a preparation method therefor, a pharmaceutical composition containing the compound, and a use of the compound as an LPA1 receptor inhibitor in the treatment of LPA1 related diseases.

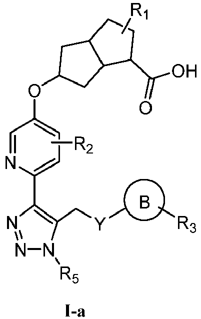

## Description

### TECHNICAL FIELD

The present disclosure belongs to the field of pharmaceutics and relates to an LPA1 small-molecule antagonist.

### BACKGROUND

Blood phospholipids are membrane-derived bioactive lipid mediators, and one of the most important blood phospholipids in medicine is lysophosphatidic acid (LPA). Lysophospholipids affect fundamental cellular functions including proliferation, differentiation, survival, migration, adhesion, invasion and morphogenesis. These functions influence many biological processes that include, but are not limited to, neurogenesis, angiogenesis, wound healing, fibrosis, immunity and carcinogenesis. LPA is not a single molecular entity but a group of endogenous structural variants of fatty acids that vary in length and saturation. The structural backbone of LPA is derived from a glycerol-based phospholipid, such as phosphatidylcholine (PC) or phosphatidic acid (PA). Lysophosphatidic acid (LPA) is a lysophospholipid that acts through sets of specific G protein-coupled receptors (GPCRs) in an autocrine and paracrine fashion. LPA binds to its cognate GPCRs (LPA1, LPA2, LPA3, LPA4, LPA5 and LPA6), activating intracellular signaling pathways to produce a variety of biological responses. Antagonists of the LPA receptors find use in the treatment of diseases, disorders or conditions in which LPA plays a role.

### SUMMARY

In a first aspect, the present disclosure provides a compound represented by formula I or a pharmaceutically acceptable salt thereof, 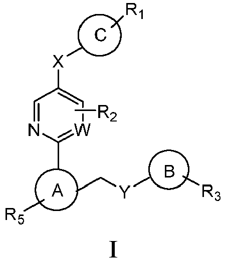
wherein ring C is selected from 8-15 membered cycloalkyl substituted with carboxyl or P(=O)(OH)₂;
R₁ is selected from the group consisting of hydrogen, C₁₋₆ alkyl, 3-6 membered cycloalkyl, C₁₋₆ alkoxy, halogen, cyano, nitro, hydroxy, 3-6 membered heterocyclyl, 5-6 membered heteroaryl, -O-3-6 membered heterocyclyl and -O-5-6 membered heteroaryl, wherein the C₁₋₆ alkyl, 3-6 membered cycloalkyl, C₁₋₆ alkoxy, 3-6 membered heterocyclyl, 5-6 membered heteroaryl, -O-3-6 membered heterocyclyl or -O-5-6 membered heteroaryl is optionally substituted with one or more R₄ₐ;
-X- is selected from the group consisting of -CO-NR₀-, -CH₂-, -Y₁- NR₀-, -NR₀-Y₁-, -Y₁-O-, -O-Y₁-, -S-Y₁-, -S(=O)₂-Y₁- and -O-C(=O)-NR₀-; the Y₁ is selected from the group consisting of a single bond, alkylene and alkenylene; the alkylene or alkenylene is optionally substituted with one or more oxo, hydroxy, cyano or halogens; the R₀ is selected from the group consisting of hydrogen and C₁₋₆ alkyl, preferably hydrogen and C₁₋₆ alkyl;
W is selected from the group consisting of a nitrogen atom and a carbon atom;
R₂ is selected from the group consisting of hydrogen, C₁₋₆ alkyl, C₁₋₆ alkoxy, hydroxy, halogen, nitro, cyano, amino, carboxyl, 3-6 membered cycloalkyl, 3-6 membered heterocyclyl, 5-6 membered heteroaryl, -O-3-6 membered heterocyclyl and -O-5-6 membered heteroaryl; the C₁₋₆ alkyl, C₁₋₆ alkoxy, 3-6 membered cycloalkyl, 3-6 membered heterocyclyl, 5-6 membered heteroaryl, -O-3-6 membered heterocyclyl or -O-5-6 membered heteroaryl is optionally substituted with one or more R_{4b};
ring A is selected from 5-6 membered heteroaryl;
Rs is substituted with a substituent selected from the group consisting of C₁₋₆ alkyl, C₁₋₆ alkoxy, methylenecyclopropyl, halogen, nitro and cyano, wherein the C₁₋₆ alkyl, C₁₋₆ alkoxy or methylenecyclopropyl is optionally substituted with one or more substituents selected from the group consisting of halogen, hydroxy, cyano and nitro;
Y is selected from the group consisting of an oxygen atom and a nitrogen atom;
ring B is selected from the group consisting of 5-6 membered heteroaryl; the 5-6 membered heteroaryl is optionally substituted with 1-3 substituents selected from the group consisting of C₁₋₆ alkyl, C₁₋₆ alkoxy, methylenecyclopropyl, halogen, nitro and cyano, wherein the C₁₋₆ alkyl, C₁₋₆ alkoxy or methylenecyclopropyl is optionally substituted with one or more substituents selected from the group consisting of halogen, hydroxy, cyano and nitro;
R₃ is selected from the group consisting of hydrogen, C₁₋₆ alkyl, C₁₋₆ alkoxy, 3-6 membered cycloalkyl, 3-6 membered heterocyclyl, 5-6 membered heteroaryl, -O-3-6 membered heterocyclyl, -O-5-6 membered heteroaryl, hydroxy, halogen, nitro, cyano, amino and carboxyl; the C₁₋₆ alkyl, C₁₋₆ alkoxy, 3-6 membered cycloalkyl, 3-6 membered heterocyclyl, 5-6 membered heteroaryl, -O-3-6 membered heterocyclyl or -O-5-6 membered heteroaryl is optionally substituted with one or more R_{4c};
R₄ₐ, R_{4b} and R_{4c} are each independently selected from the group consisting of halogen, hydroxy, oxo, nitro, cyano, amino, C₁₋₆ alkyl, C₁₋₆ haloalkyl, C₁₋₆ alkoxy, C₁₋₆ haloalkoxy, C₂₋₆ alkenyloxy, C₂₋₆ alkynyloxy, 3-6 membered cycloalkyl, 3-6 membered heterocyclyl, 3-6 membered heterocycloalkoxy, C₃₋₈ cycloalkenyloxy, 5-6 membered aryl and 3-6 membered heteroaryl.

In some embodiments, in the compound represented by formula I or the pharmaceutically acceptable salt thereof, ring C is selected from the group consisting of 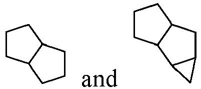 substituted with carboxyl or P(=O)(OH)₂-.

In some embodiments, in the compound represented by formula I or the pharmaceutically acceptable salt thereof, ring C is selected from the group consisting of 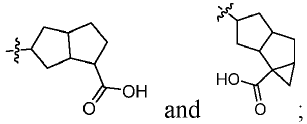 preferably, ring C is selected from

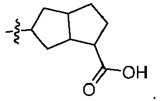

In some embodiments, in the compound represented by formula I or the pharmaceutically acceptable salt thereof, -X- is selected from the group consisting of -CO-NR₀-, -CH₂-, -Y₁-NR₀-, -NR₀-Y₁-, -Y₁-O-, -O-Y₁-, -S-Y₁- , -S(=O)₂-Y₁- and -O-C(=O)-NR₀-;
the Y₁ is selected from the group consisting of a single bond, alkylene and alkenylene; the alkylene or alkenylene is optionally substituted with one or more oxo, hydroxy, cyano or halogens;
the R₀ is selected from the group consisting of hydrogen and C₁₋₆ alkyl.

In some embodiments, in the compound represented by formula I or the pharmaceutically acceptable salt thereof, -X- is selected from the group consisting of -O-, -N-, -S- and -SO₂-.

In some embodiments, in the compound represented by formula I or the pharmaceutically acceptable salt thereof, W is a nitrogen atom.

In some embodiments, in the compound represented by formula I or the pharmaceutically acceptable salt thereof, W is a carbon atom.

In some embodiments, in the compound represented by formula I or the pharmaceutically acceptable salt thereof, ring A is selected from 5-6 membered heteroaryl;
Rs is selected from the group consisting of C₁₋₆ alkyl, C₁₋₆ alkoxy and methylenecyclopropyl, wherein the C₁₋₆ alkyl, C₁₋₆ alkoxy or methylenecyclopropyl is optionally substituted with one or more substituents selected from the group consisting of halogen, hydroxy, cyano and nitro.

In some embodiments, in the compound represented by formula I or the pharmaceutically acceptable salt thereof, R₅ is selected from C₁₋₆ alkyl, wherein the C₁₋₆ alkyl is optionally substituted with one or more halogens.

In some embodiments, in the compound represented by formula I or the pharmaceutically acceptable salt thereof, R₅ is selected from methyl, wherein the methyl is optionally substituted with 1-3 halogens.

In some embodiments, in the compound represented by formula I or the pharmaceutically acceptable salt thereof, ring A is selected from 5-6 membered heteroaryl; R₅ is selected from the group consisting of halogen, nitro and cyano.

In a second aspect, the present disclosure provides compounds represented by formulas I-a to I-l or pharmaceutically acceptable salts thereof, 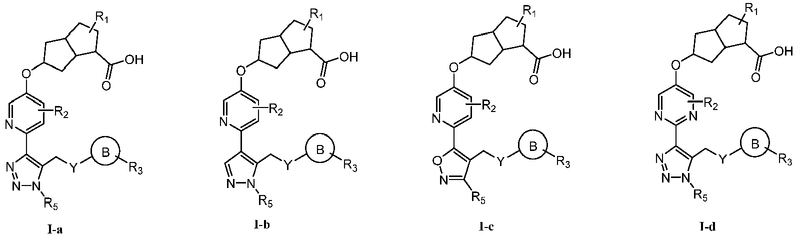 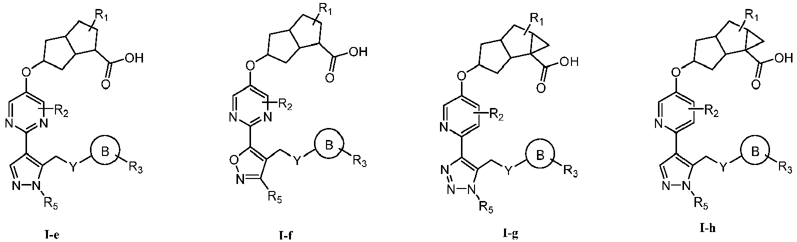 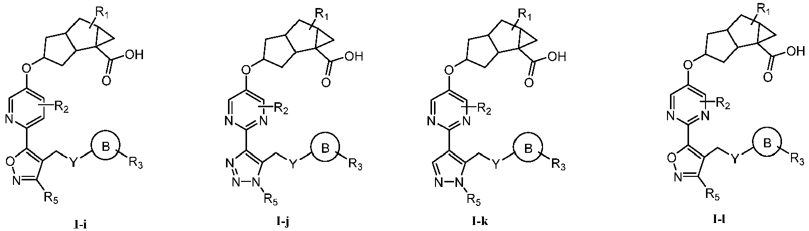 wherein R₁, R₂, R₅, Y, ring B and R₃ are as defined in formula I.

In some embodiments, in the compound represented by formula I, I-a, I-b, I-c, I-d, I-e, I-f, I-g, I-h, I-i, I-j, I-k or I-l or the pharmaceutically acceptable salt thereof, R₁ is selected from the group consisting of hydrogen, C₁₋₆ alkyl, 3-6 membered cycloalkyl, C₁₋₆ alkoxy, halogen, cyano, nitro and hydroxy, wherein the C₁₋₆ alkyl, 3-6 membered cycloalkyl or C₁₋₆ alkoxy is optionally substituted with one or more R₄ₐ.

In some embodiments, in the compound represented by formula I, I-a, I-b, I-c, I-d, I-e, I-f, I-g, I-h, I-i, I-j, I-k or I-l or the pharmaceutically acceptable salt thereof, R₁ is selected from the group consisting of hydrogen, 3-6 membered heterocyclyl, 5-6 membered heteroaryl, -O-3-6 membered heterocyclyl and -O-5-6 membered heteroaryl, wherein the 3-6 membered heterocyclyl, 5-6 membered heteroaryl, -O-3-6 membered heterocyclyl or -O-5-6 membered heteroaryl is optionally substituted with one or more R4a.

In some embodiments, in the compound represented by formula I, I-a, I-b, I-c, I-d, I-e, I-f, I-g, I-h, I-i, I-j, I-k or I-l or the pharmaceutically acceptable salt thereof, R₁ is selected from the group consisting of hydrogen, C₁₋₆ alkyl, 3-6 membered cycloalkyl and C₁₋₆ alkoxy, wherein the C₁₋₆ alkyl, 3-6 membered cycloalkyl or C₁₋₆ alkoxy is optionally substituted with one or more R₄ₐ.

In some embodiments, in the compound represented by formula I, I-a, I-b, I-c, I-d, I-e, I-f, I-g, I-h, I-i, I-j, I-k or I-l or the pharmaceutically acceptable salt thereof, R₁ is selected from the group consisting of hydrogen, methyl, trifluoromethyl and halogen.

In some embodiments, in the compound represented by formula I, I-a, I-b, I-c, I-d, I-e, I-f, I-g, I-h, I-i, I-j, I-k or I-l or the pharmaceutically acceptable salt thereof, R₁ is selected from hydrogen.

In some embodiments, in the compound represented by formula I, I-a, I-b, I-c, I-d, I-e, I-f, I-g, I-h, I-i, I-j, I-k or I-l or the pharmaceutically acceptable salt thereof, R₂ is selected from the group consisting of hydrogen, C₁₋₆ alkyl, C₁₋₆ alkoxy, hydroxy, halogen, nitro, cyano, amino, carboxyl and 3-6 membered cycloalkyl; the C₁₋₆ alkyl, C₁₋₆ alkoxy or 3-6 membered cycloalkyl is optionally substituted with one or more R_{4b}. In some embodiments, in the compound represented by formula I, I-a, I-b, I-c, I-d, I-e, I-f, I-g, I-h, I-i, I-j, I-k or I-l or the pharmaceutically acceptable salt thereof, R₂ is selected from the group consisting of hydrogen, 3-6 membered heterocyclyl, 5-6 membered heteroaryl, -O-3-6 membered heterocyclyl and -O-5-6 membered heteroaryl; the 3-6 membered heterocyclyl, 5-6 membered heteroaryl, -O-3-6 membered heterocyclyl or -O-5-6 membered heteroaryl is optionally substituted with one or more R4b.

In some embodiments, in the compound represented by formula I, I-a, I-b, I-c, I-d, I-e, I-f, I-g, I-h, I-i, I-j, I-k or I-l or the pharmaceutically acceptable salt thereof, R₂ is selected from the group consisting of hydrogen, C₁₋₆ alkyl and halogen; the C₁₋₆ alkyl is optionally substituted with one or more R_{4b}.

In some embodiments, in the compound represented by formula I, I-a, I-b, I-c, I-d, I-e, I-f, I-g, I-h, I-i, I-j, I-k or I-l or the pharmaceutically acceptable salt thereof, R₂ is selected from the group consisting of C₁₋₆ alkyl; the C₁₋₆ alkyl is optionally substituted with one or more R_{4b}.

In some embodiments, in the compound represented by formula I, I-a, I-b, I-c, I-d, I-e, I-f, I-g, I-h, I-i, I-j, I-k or I-l or the pharmaceutically acceptable salt thereof, R₂ is selected from the group consisting of hydrogen, hydroxy, halogen, nitro, cyano and amino.

In some embodiments, in the compound represented by formula I, I-a, I-b, I-c, I-d, I-e, I-f, I-g, I-h, I-i, I-j, I-k or I-l or the pharmaceutically acceptable salt thereof, R₂ is selected from methyl.

In a third aspect, the present disclosure further provides a compound represented by formula I'-a, I'-b, I'-c, I'-d, I'-e, I'-f, I'-g, I'-h, I'-i, I'-j, I'-k or I'-l or a pharmaceutically acceptable salt thereof: 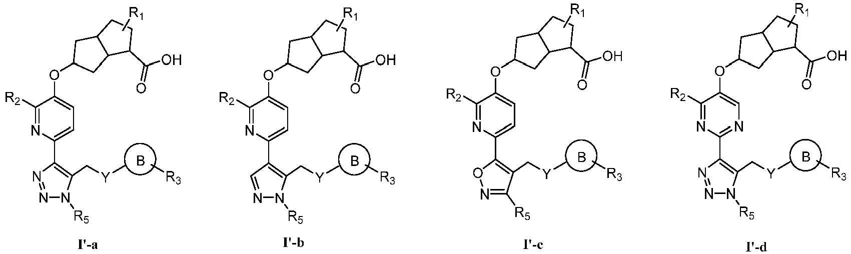 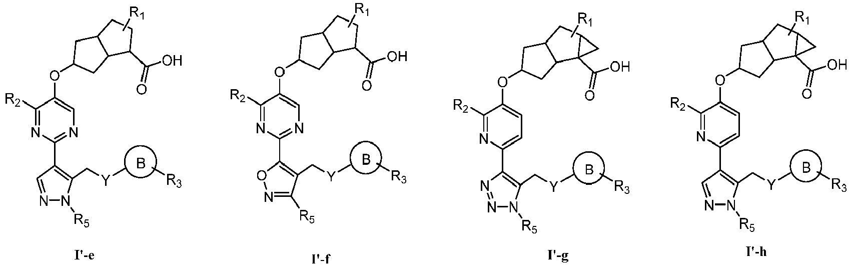 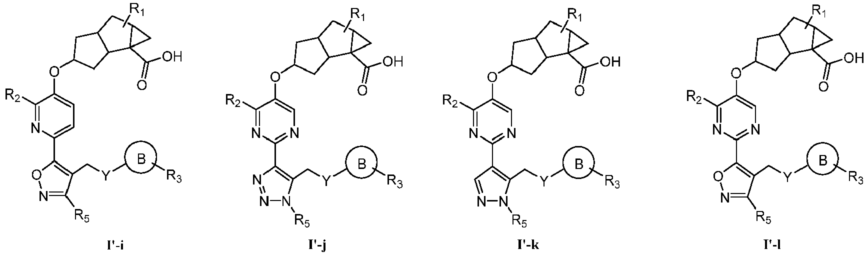 wherein R₁, R₂, R₅, Y, ring B and R₃ are as defined in formula I.

In some embodiments, in the compound represented by formula I, I-a, I-b, I-c, I-d, I-e, I-f, I-g, I-h, I-i, I-j, I-k, I-l, I'-a, I'-b, I'-c, I'-d, I'-e, I'-f, I'-g, I'-h, I'-i, I'-j, I'-k or I'-l or the pharmaceutically acceptable salt thereof, Y is an oxygen atom.

In some embodiments, in the compound represented by formula I, I-a, I-b, I-c, I-d, I-e, I-f, I-g, I-h, I-i, I-j, I-k, I-l, I'-a, I'-b, I'-c, I'-d, I'-e, I'-f, I'-g, I'-h, I'-i, I'-j, I'-k or I'-l or the pharmaceutically acceptable salt thereof, Y is a nitrogen atom.

In some embodiments, in the compound represented by formula I, I-a, I-b, I-c, I-d, I-e, I-f, I-g, I-h, I-i, I-j, I-k, I-l, I'-a, I'-b, I'-c, I'-d, I'-e, I'-f, I'-g, I'-h, I'-i, I'-j, I'-k or I'-l or the pharmaceutically acceptable salt thereof, ring B is selected from the group consisting of 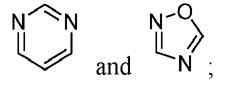 the 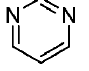 is optionally substituted with 1-3 substituents selected from the group consisting of C₁₋₆ alkyl, C₁₋₆ alkoxy, methylenecyclopropyl, halogen, nitro and cyano, wherein the C₁₋₆ alkyl, C₁₋₆ alkoxy or methylenecyclopropyl is optionally substituted with one or more substituents selected from the group consisting of halogen, hydroxy, cyano and nitro.

In some embodiments, in the compound represented by formula I, I-a, I-b, I-c, I-d, I-e, I-f, I-g, I-h, I-i, I-j, I-k, I-l, I'-a, I'-b, I'-c, I'-d, I'-e, I'-f, I'-g, I'-h, I'-i, I'-j, I'-k or I'-l or the pharmaceutically acceptable salt thereof, ring B is selected from the group consisting of 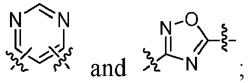 the 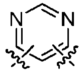 is optionally substituted with 1-3 substituents selected from the group consisting of C₁₋₆ alkyl, C₁₋₆ alkoxy, methylenecyclopropyl, halogen, nitro and cyano, wherein the C₁₋₆ alkyl, C₁₋₆ alkoxy or methylenecyclopropyl is optionally substituted with one or more substituents selected from the group consisting of halogen, hydroxy, cyano and nitro.

In some embodiments, in the compound represented by formula I, I-a, I-b, I-c, I-d, I-e, I-f, I-g, I-h, I-i, I-j, I-k, I-l, I'-a, I'-b, I'-c, I'-d, I'-e, I'-f, I'-g, I'-h, I'-i, I'-j, I'-k or I'-l or the pharmaceutically acceptable salt thereof, ring B is selected from 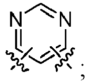 the 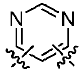 is optionally substituted with 1-3 substituents selected from the group consisting of C₁₋₆ alkyl, halogen and cyano, wherein the C₁₋₆ alkyl is optionally substituted with one or more substituents selected from the group consisting of halogen, hydroxy, cyano and nitro.

In some embodiments, in the compound represented by formula I, I-a, I-b, I-c, I-d, I-e, I-f, I-g, I-h, I-i, I-j, I-k, I-l, I'-a, I'-b, I'-c, I'-d, I'-e, I'-f, I'-g, I'-h, I'-i, I'-j, I'-k or I'-l or the pharmaceutically acceptable salt thereof, ring B is selected from the group consisting of 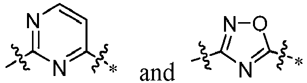 , and R₃ is attached to the * end.

In some embodiments, in the compound represented by formula I, I-a, I-b, I-c, I-d, I-e, I-f, I-g, I-h, I-i, I-j, I-k, I-l, I'-a, I'-b, I'-c, I'-d, I'-e, I'-f, I'-g, I'-h, I'-i, I'-j, I'-k or I'-l or the pharmaceutically acceptable salt thereof, ring B is selected from 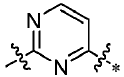 , and R₃ is attached to the * end.

In some embodiments, in the compound represented by formula I, I-a, I-b, I-c, I-d, I-e, I-f, I-g, I-h, I-i, I-j, I-k, I-l, I'-a, I'-b, I'-c, I'-d, I'-e, I'-f, I'-g, I'-h, I'-i, I'-j, I'-k or I'-l or the pharmaceutically acceptable salt thereof, ring B is selected from 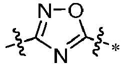 , and R₃ is attached to the * end.

In some embodiments, in the compound represented by formula I, I-a, I-b, I-c, I-d, I-e, I-f, I-g, I-h, I-i, I-j, I-k, I-l, I'-a, I'-b, I'-c, I'-d, I'-e, I'-f, I'-g, I'-h, I'-i, I'-j, I'-k or I'-l or the pharmaceutically acceptable salt thereof, ring B is selected from 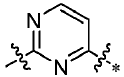 , and R₃ is attached to the * end; the 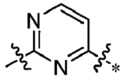 is optionally substituted with 1-3 substituents selected from the group consisting of C₁₋₆ alkyl, halogen and cyano, wherein the C₁₋₆ alkyl is optionally substituted with one or more substituents selected from the group consisting of halogen, hydroxy, cyano and nitro. In some embodiments, in the compound represented by formula I, I-a, I-b, I-c, I-d, I-e, I-f, I-g, I-h, I-i, I-j, I-k, I-l, I'-a, I'-b, I'-c, I'-d, I'-e, I'-f, I'-g, I'-h, I'-i, I'-j, I'-k or I'-l or the pharmaceutically acceptable salt thereof, R₃ is selected from the group consisting of hydrogen, C₁₋₆ alkyl, C₁₋₆ alkoxy, 3-6 membered cycloalkyl, 3-6 membered heterocyclyl, 5-6 membered heteroaryl, -O-3-6 membered heterocyclyl and -O-5-6 membered heteroaryl; the C₁₋₆ alkyl, C₁₋₆ alkoxy, 3-6 membered cycloalkyl, 3-6 membered heterocyclyl, 5-6 membered heteroaryl, -O-3-6 membered heterocyclyl or -O-5-6 membered heteroaryl is optionally substituted with one or more R_{4c}.

In some embodiments, in the compound represented by formula I, I-a, I-b, I-c, I-d, I-e, I-f, I-g, I-h, I-i, I-j, I-k, I-l, I'-a, I'-b, I'-c, I'-d, I'-e, I'-f, I'-g, I'-h, I'-i, I'-j, I'-k or I'-l or the pharmaceutically acceptable salt thereof, R₃ is selected from the group consisting of hydrogen, hydroxy, halogen, nitro, cyano, amino and carboxyl.

In some embodiments, in the compound represented by formula I, I-a, I-b, I-c, I-d, I-e, I-f, I-g, I-h, I-i, I-j, I-k, I-l, I'-a, I'-b, I'-c, I'-d, I'-e, I'-f, I'-g, I'-h, I'-i, I'-j, I'-k or I'-l or the pharmaceutically acceptable salt thereof, R₃ is selected from the group consisting of hydrogen, C₁₋₆ alkyl, C₁₋₆ alkoxy and 3-6 membered cycloalkyl; the C₁₋₆ alkyl, C₁₋₆ alkoxy or 3-6 membered cycloalkyl is optionally substituted with one or more R_{4c}.

In some embodiments, in the compound represented by formula I, I-a, I-b, I-c, I-d, I-e, I-f, I-g, I-h, I-i, I-j, I-k, I-l, I'-a, I'-b, I'-c, I'-d, I'-e, I'-f, I'-g, I'-h, I'-i, I'-j, I'-k or I'-l or the pharmaceutically acceptable salt thereof, R₃ is selected from the group consisting of methyl, methoxy, ethyl, propyl, ethoxy, cyclopropyloxy, cyclobutyl and cyclobutyloxy; the methyl, methoxy, ethyl, propyl, ethoxy, cyclopropyloxy, cyclobutyl or cyclobutyloxy is optionally substituted with 1-3 R_{4c}.

In some embodiments, in the compound represented by formula I, I-a, I-b, I-c, I-d, I-e, I-f, I-g, I-h, I-i, I-j, I-k, I-l, I'-a, I'-b, I'-c, I'-d, I'-e, I'-f, I'-g, I'-h, I'-i, I'-j, I'-k or I'-l or the pharmaceutically acceptable salt thereof, R₃ is selected from the group consisting of methyl, ethyl, propyl and cyclobutyl; the methyl, ethyl, propyl or cyclobutyl is optionally substituted with 1-3 R_{4c}.

In some embodiments, in the compound represented by formula I, I-a, I-b, I-c, I-d, I-e, I-f, I-g, I-h, I-i, I-j, I-k, I-l, I'-a, I'-b, I'-c, I'-d, I'-e, I'-f, I'-g, I'-h, I'-i, I'-j, I'-k or I'-l or the pharmaceutically acceptable salt thereof, R₃ is selected from C₁₋₆ alkyl; the C₁₋₆ alkyl is substituted with one or more R_{4c}.

In some embodiments, in the compound represented by formula I, I-a, I-b, I-c, I-d, I-e, I-f, I-g, I-h, I-i, I-j, I-k, I-l, I'-a, I'-b, I'-c, I'-d, I'-e, I'-f, I'-g, I'-h, I'-i, I'-j, I'-k or I'-l or the pharmaceutically acceptable salt thereof, R₃ is selected from methyl; the methyl is substituted with one or more R_{4c}.

In some embodiments, in the compound represented by formula I, I-a, I-b, I-c, I-d, I-e, I-f, I-g, I-h, I-i, I-j, I-k, I-l, I'-a, I'-b, I'-c, I'-d, I'-e, I'-f, I'-g, I'-h, I'-i, I'-j, I'-k or I'-l or the pharmaceutically acceptable salt thereof, R₃ is selected from the group consisting of trifluoromethyl, ethyl, isopropyl, difluoroethoxy, ethoxy, cyclopropyloxy, cyclobutyl, -CH₂OCH₃,

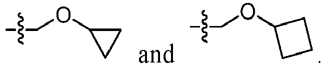

In some embodiments, in the compound represented by formula I, I-a, I-b, I-c, I-d, I-e, I-f, I-g, I-h, I-i, I-j, I-k, I-l, I'-a, I'-b, I'-c, I'-d, I'-e, I'-f, I'-g, I'-h, I'-i, I'-j, I'-k or I'-l or the pharmaceutically acceptable salt thereof, R₃ is selected from the group consisting of trifluoromethyl, difluoromethyl, ethyl, cyclopropyl, -CH₂OCH₃, -CH₂OCHF₂, -CH₂OCH₂CH₃,

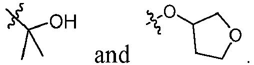

In some embodiments, in the compound represented by formula I, I-a, I-b, I-c, I-d, I-e, I-f, I-g, I-h, I-i, I-j, I-k, I-l, I'-a, I'-b, I'-c, I'-d, I'-e, I'-f, I'-g, I'-h, I'-i, I'-j, I'-k or I'-l or the pharmaceutically acceptable salt thereof, R₅ is selected from C₁₋₆ alkyl, wherein the C₁₋₆ alkyl is optionally substituted with one or more halogens.

In some embodiments, in the compound represented by formula I, I-a, I-b, I-c, I-d, I-e, I-f, I-g, I-h, I-i, I-j, I-k, I-l, I'-a, I'-b, I'-c, I'-d, I'-e, I'-f, I'-g, I'-h, I'-i, I'-j, I'-k or I'-l or the pharmaceutically acceptable salt thereof, R₅ is selected from methyl, wherein the methyl is optionally substituted with 1-3 halogens.

In some embodiments, in the compound represented by formula I, I-a, I-b, I-c, I-d, I-e, I-f, I-g, I-h, I-i, I-j, I-k, I-l, I'-a, I'-b, I'-c, I'-d, I'-e, I'-f, I'-g, I'-h, I'-i, I'-j, I'-k or I'-l or the pharmaceutically acceptable salt thereof, each R₄ₐ is independently selected from the group consisting of halogen, hydroxy, oxo, nitro, cyano and amino.

In some embodiments, in the compound represented by formula I, I-a, I-b, I-c, I-d, I-e, I-f, I-g, I-h, I-i, I-j, I-k, I-l, I'-a, I'-b, I'-c, I'-d, I'-e, I'-f, I'-g, I'-h, I'-i, I'-j, I'-k or I'-l or the pharmaceutically acceptable salt thereof, each R₄ₐ is independently selected from the group consisting of C₁₋₆ alkyl, C₁₋₆ alkoxy, 3-6 membered cycloalkyl, 3-6 membered heterocyclyl and 3-6 membered heterocycloalkoxy.

In some embodiments, in the compound represented by formula I, I-a, I-b, I-c, I-d, I-e, I-f, I-g, I-h, I-i, I-j, I-k, I-l, I'-a, I'-b, I'-c, I'-d, I'-e, I'-f, I'-g, I'-h, I'-i, I'-j, I'-k or I'-l or the pharmaceutically acceptable salt thereof, each R₄ₐ is independently selected from the group consisting of C₂₋₆ alkenyloxy, C₂₋₆ alkynyloxy, 3-6 membered cycloalkyl, 3-6 membered heterocyclyl, 3-6 membered heterocycloalkoxy, C₃₋₈ cycloalkenyloxy, 5-6 membered aryl and 3-6 membered heteroaryl.

In some embodiments, in the compound represented by formula I, I-a, I-b, I-c, I-d, I-e, I-f, I-g, I-h, I-i, I-j, I-k, I-l, I'-a, I'-b, I'-c, I'-d, I'-e, I'-f, I'-g, I'-h, I'-i, I'-j, I'-k or I'-l or the pharmaceutically acceptable salt thereof, R₄ₐ is independently selected from the group consisting of halogen, methyl, ethyl, methoxy, ethoxy, cyclopropoxy and cyclobutoxy. In some embodiments, in the compound represented by formula I, I-a, I-b, I-c, I-d, I-e, I-f, I-g, I-h, I-i, I-j, I-k, I-l, I'-a, I'-b, I'-c, I'-d, I'-e, I'-f, I'-g, I'-h, I'-i, I'-j, I'-k or I'-l or the pharmaceutically acceptable salt thereof, each R_{4b} is independently selected from the group consisting of halogen, hydroxy, oxo, nitro, cyano and amino.

In some embodiments, in the compound represented by formula I, I-a, I-b, I-c, I-d, I-e, I-f, I-g, I-h, I-i, I-j, I-k, I-l, I'-a, I'-b, I'-c, I'-d, I'-e, I'-f, I'-g, I'-h, I'-i, I'-j, I'-k or I'-l or the pharmaceutically acceptable salt thereof, each R_{4b} is independently selected from the group consisting of C₁₋₆ alkyl, C₁₋₆ alkoxy, 3-6 membered cycloalkyl, 3-6 membered heterocyclyl and 3-6 membered heterocycloalkoxy.

In some embodiments, in the compound represented by formula I, I-a, I-b, I-c, I-d, I-e, I-f, I-g, I-h, I-i, I-j, I-k, I-l, I'-a, I'-b, I'-c, I'-d, I'-e, I'-f, I'-g, I'-h, I'-i, I'-j, I'-k or I'-l or the pharmaceutically acceptable salt thereof, each R_{4b} is independently selected from the group consisting of C₂₋₆ alkenyloxy, C₂₋₆ alkynyloxy, 3-6 membered cycloalkyl, 3-6 membered heterocyclyl, 3-6 membered heterocycloalkoxy, C₃₋₈ cycloalkenyloxy, 5-6 membered aryl and 3-6 membered heteroaryl.

In some embodiments, in the compound represented by formula I, I-a, I-b, I-c, I-d, I-e, I-f, I-g, I-h, I-i, I-j, I-k, I-l, I'-a, I'-b, I'-c, I'-d, I'-e, I'-f, I'-g, I'-h, I'-i, I'-j, I'-k or I'-l or the pharmaceutically acceptable salt thereof, each R_{4b} is independently selected from the group consisting of halogen, methyl, ethyl, methoxy, ethoxy, cyclopropoxy and cyclobutoxy.

In some embodiments, in the compound represented by formula I, I-a, I-b, I-c, I-d, I-e, I-f, I-g, I-h, I-i, I-j, I-k, I-l, I'-a, I'-b, I'-c, I'-d, I'-e, I'-f, I'-g, I'-h, I'-i, I'-j, I'-k or I'-l or the pharmaceutically acceptable salt thereof, each R_{4c} is independently selected from the group consisting of halogen, hydroxy, oxo, nitro, cyano and amino.

In some embodiments, in the compound represented by formula I, I-a, I-b, I-c, I-d, I-e, I-f, I-g, I-h, I-i, I-j, I-k, I-l, I'-a, I'-b, I'-c, I'-d, I'-e, I'-f, I'-g, I'-h, I'-i, I'-j, I'-k or I'-l or the pharmaceutically acceptable salt thereof, each R_{4c} is independently selected from the group consisting of C₁₋₆ alkyl, C₁₋₆ haloalkyl, C₁₋₆ alkoxy, C₁₋₆ haloalkoxy, 3-6 membered cycloalkyl, 3-6 membered heterocyclyl and 3-6 membered heterocycloalkoxy.

In some embodiments, in the compound represented by formula I, I-a, I-b, I-c, I-d, I-e, I-f, I-g, I-h, I-i, I-j, I-k, I-l, I'-a, I'-b, I'-c, I'-d, I'-e, I'-f, I'-g, I'-h, I'-i, I'-j, I'-k or I'-l or the pharmaceutically acceptable salt thereof, each R_{4c} is independently selected from the group consisting of C₂₋₆ alkenyloxy, C₂₋₆ alkynyloxy, 3-6 membered cycloalkyl, 3-6 membered heterocyclyl, 3-6 membered heterocycloalkoxy, C₃₋₈ cycloalkenyloxy, 5-6 membered aryl and 3-6 membered heteroaryl.

In some embodiments, in the compound represented by formula I, I-a, I-b, I-c, I-d, I-e, I-f, I-g, I-h, I-i, I-j, I-k, I-l, I'-a, I'-b, I'-c, I'-d, I'-e, I'-f, I'-g, I'-h, I'-i, I'-j, I'-k or I'-l or the pharmaceutically acceptable salt thereof, each R_{4c} is independently selected from the group consisting of halogen, methyl, ethyl, methoxy, ethoxy, cyclopropoxy and cyclobutoxy.

In a fourth aspect, the present disclosure further provides a compound as shown below or a pharmaceutically acceptable salt thereof:

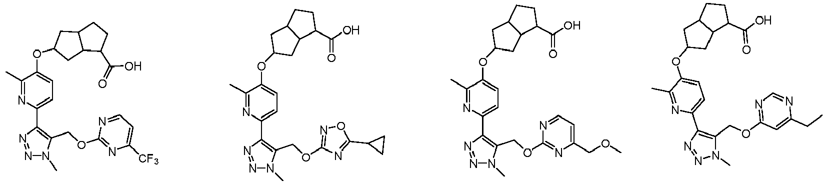

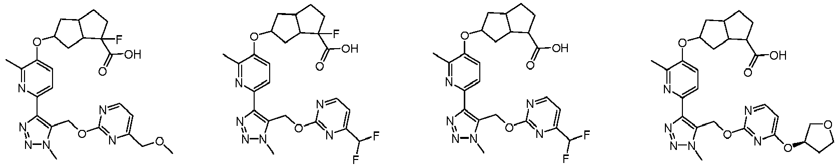

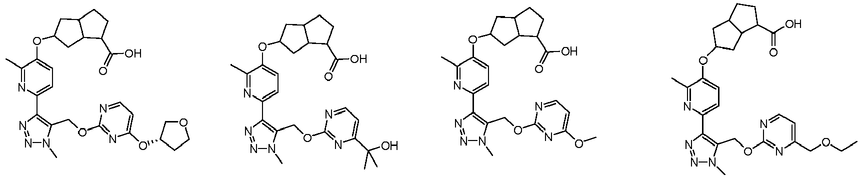

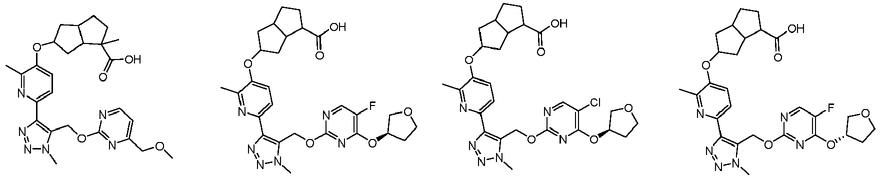

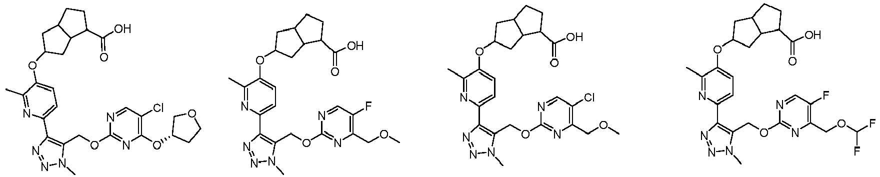

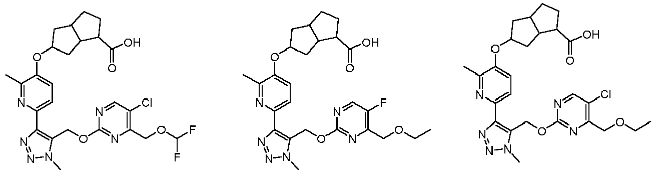

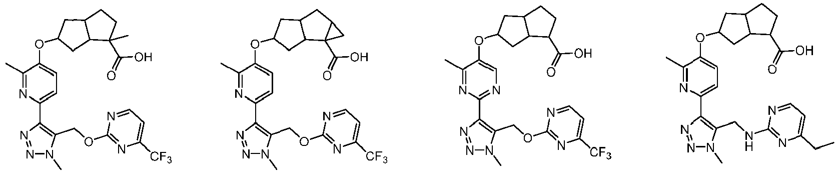

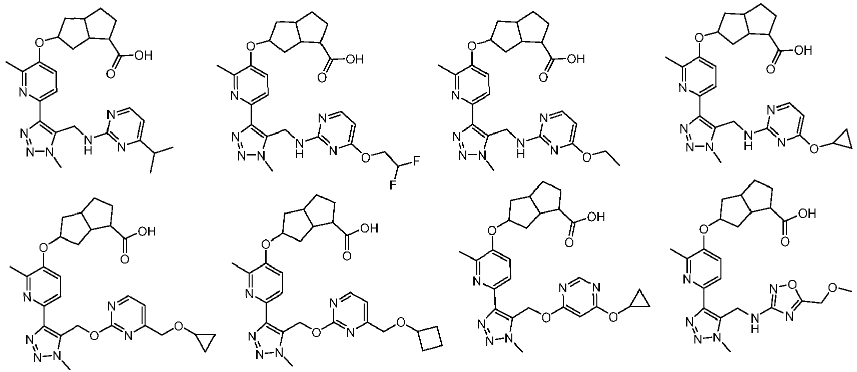

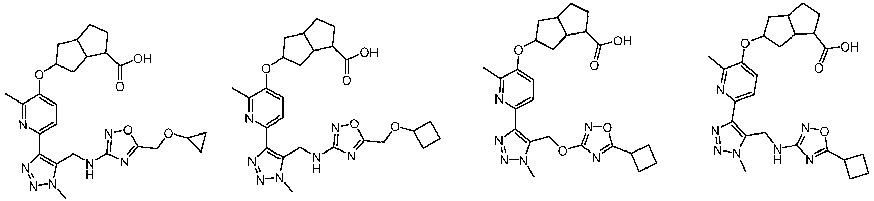

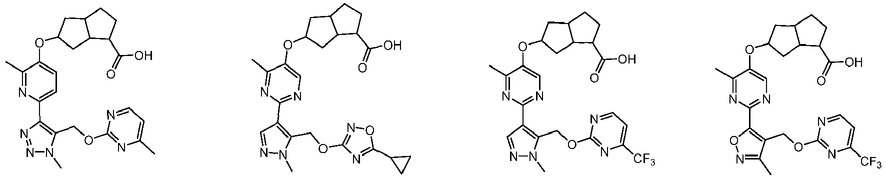

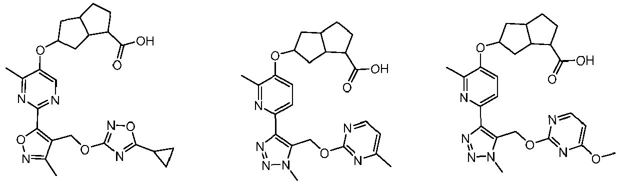

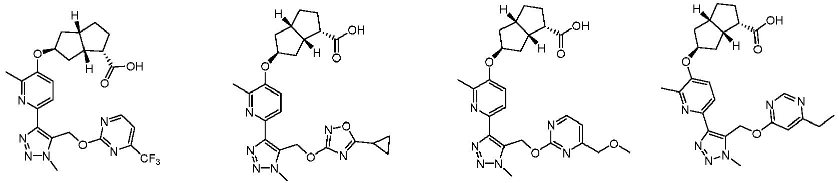

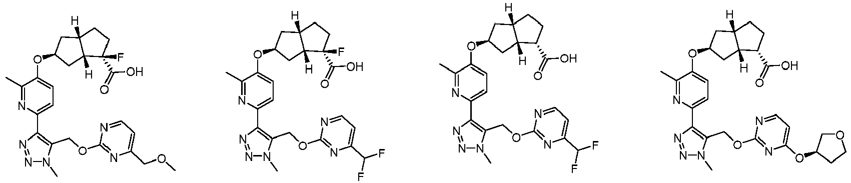

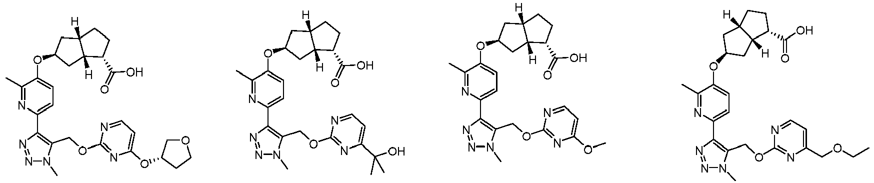

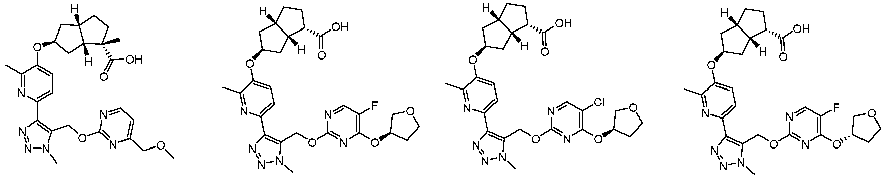

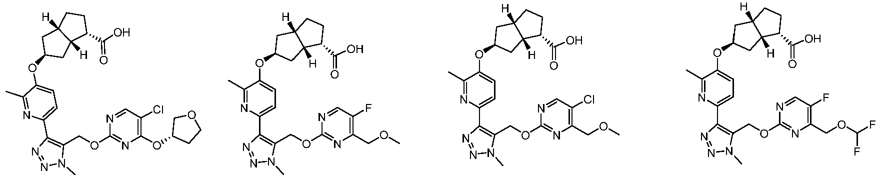

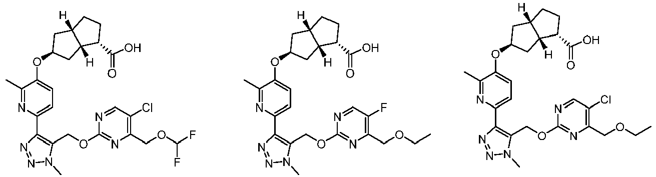

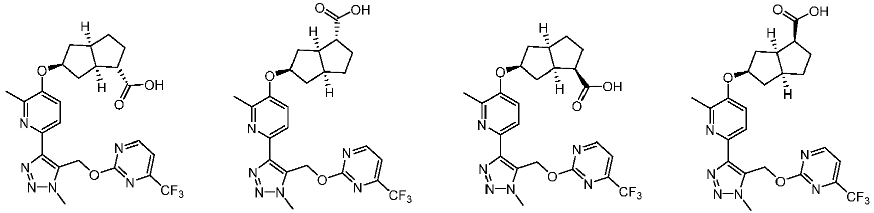

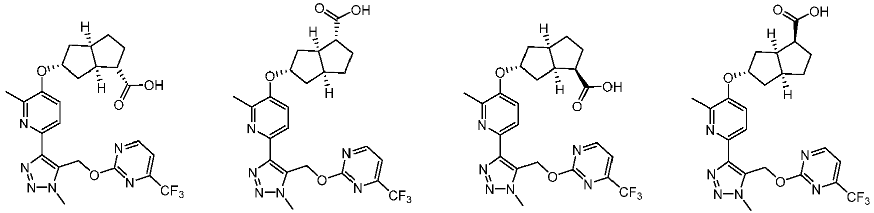

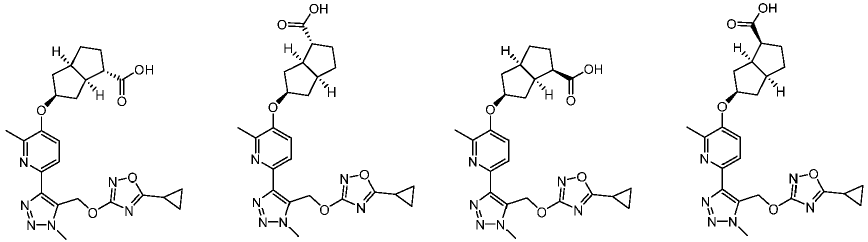

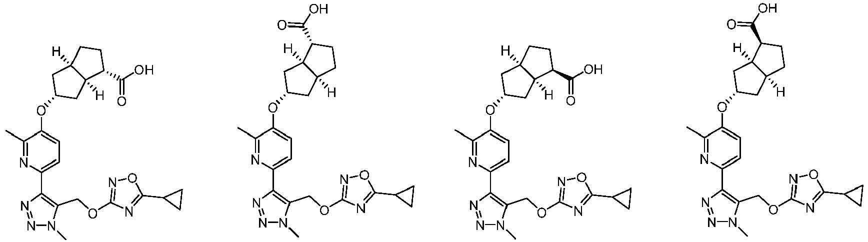

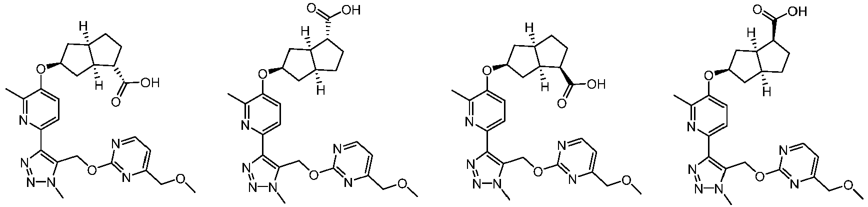

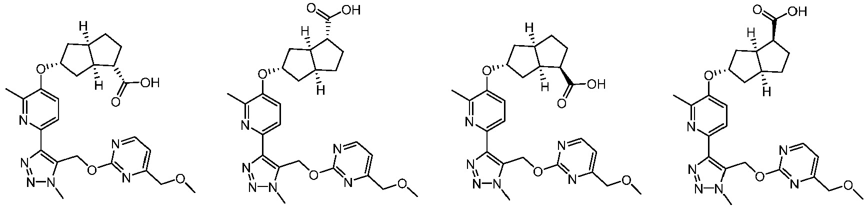

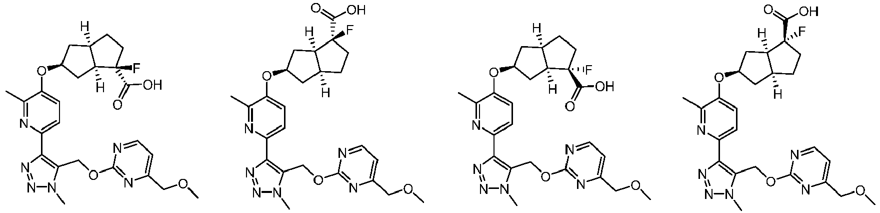

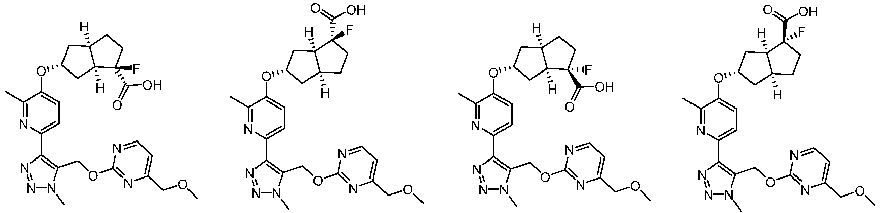

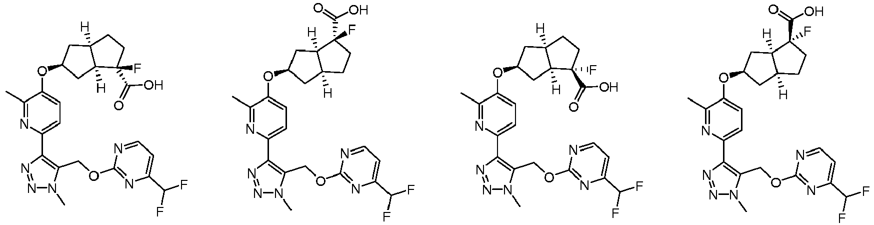

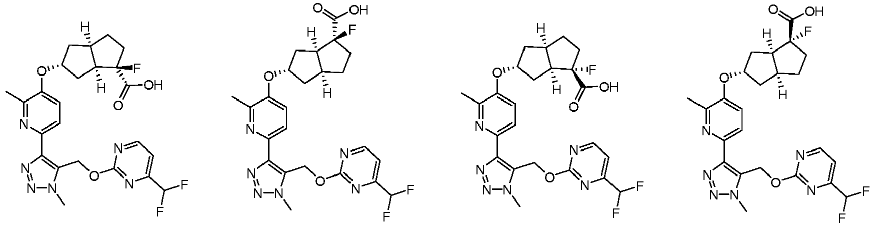

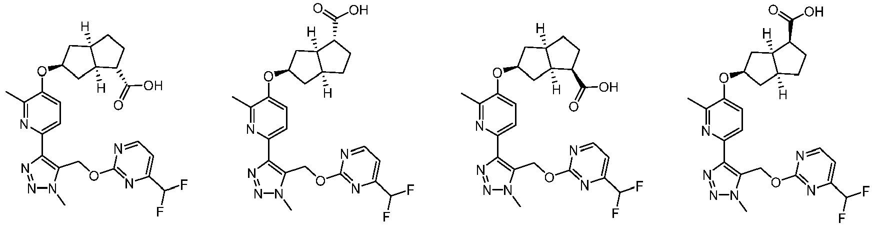

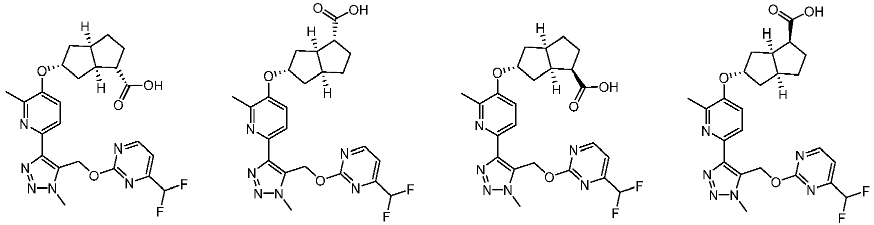

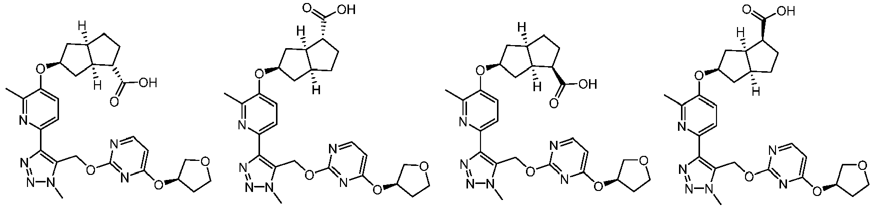

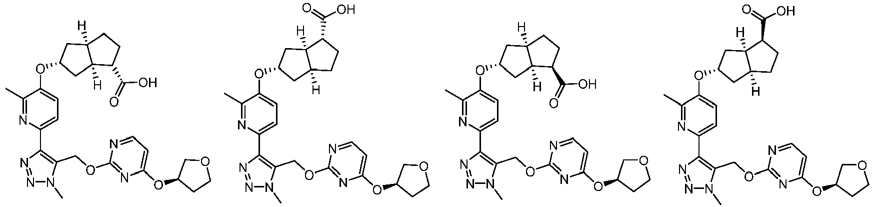

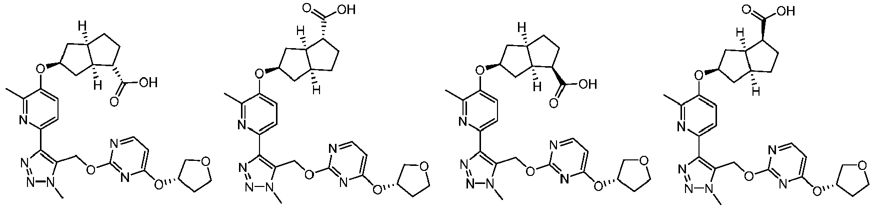

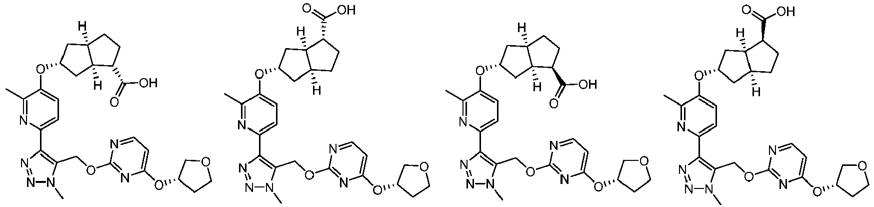

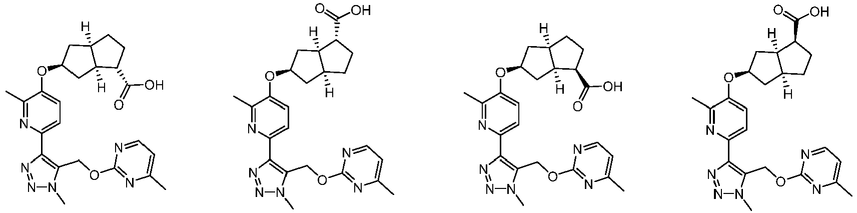

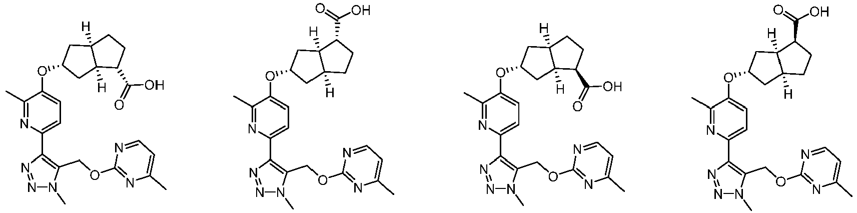

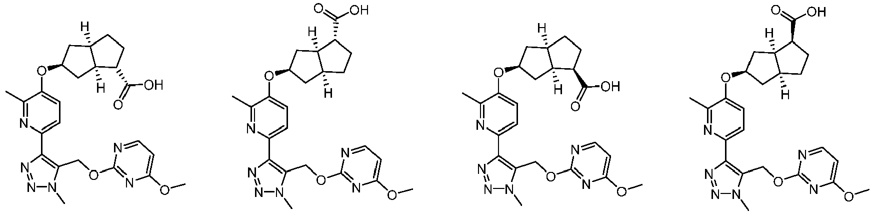

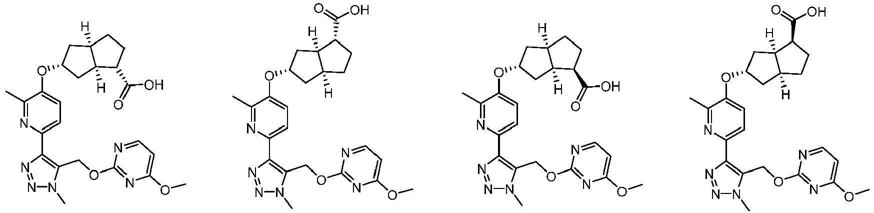

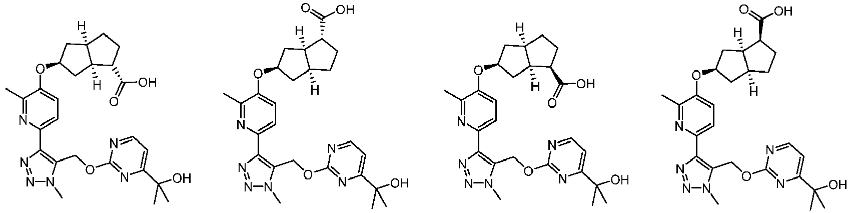

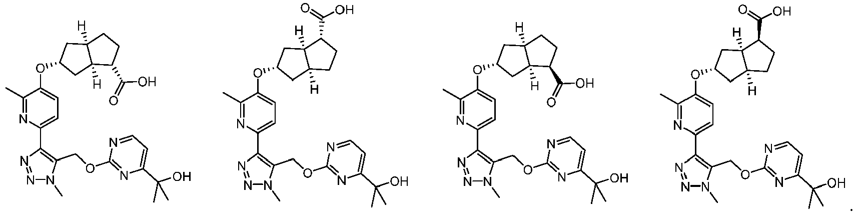

In a fifth aspect, the present disclosure further provides a compound as shown below or a pharmaceutically acceptable salt thereof 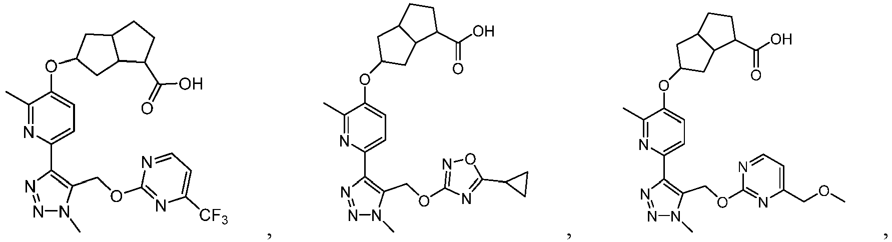 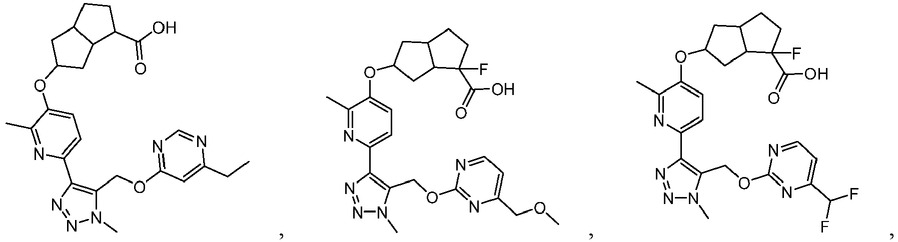 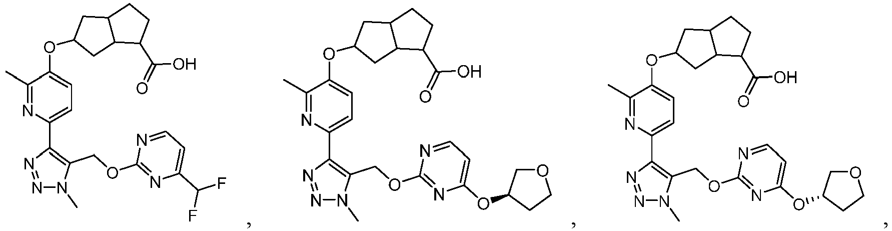 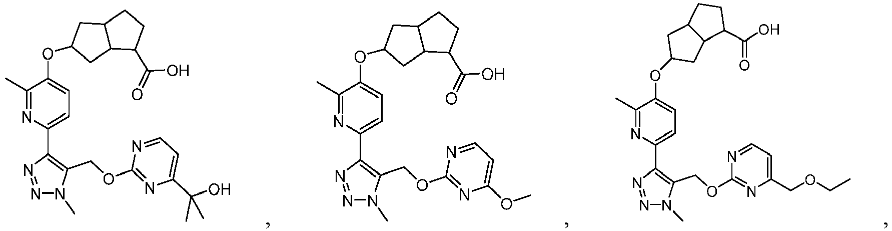 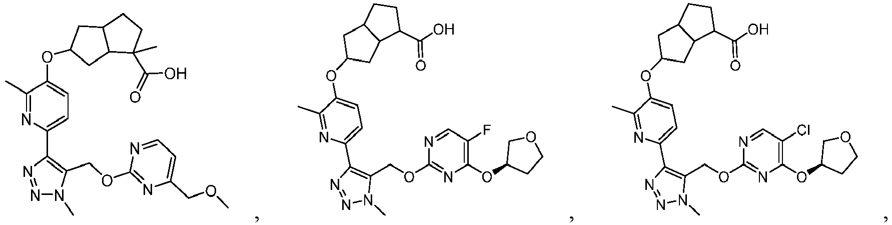 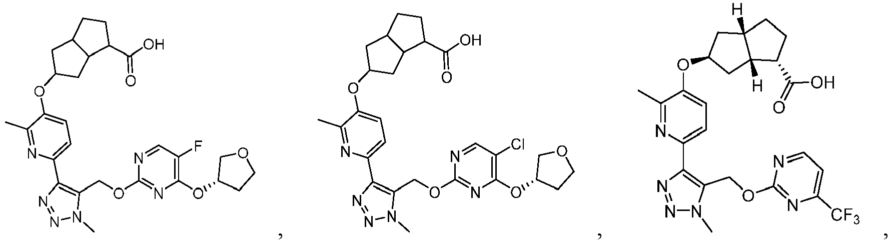 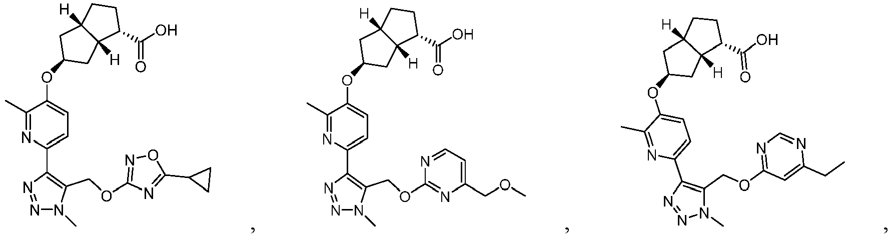 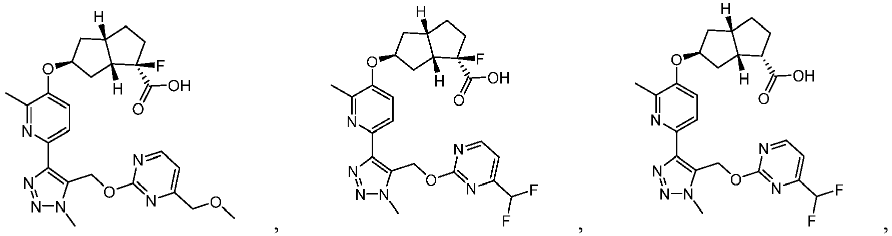 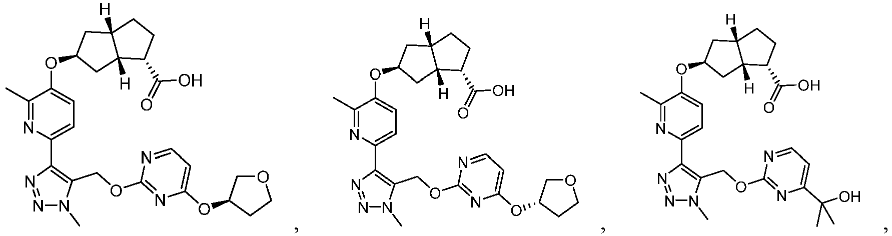 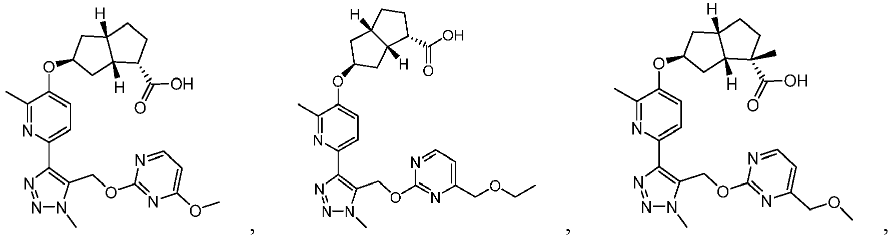 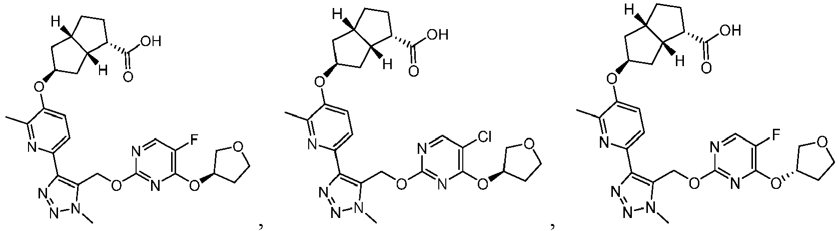 and

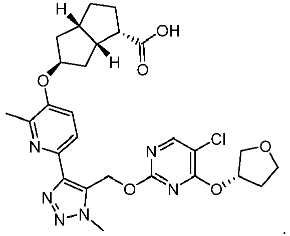

In a sixth aspect, the present disclosure further provides an isotopically substituted form of the compound set forth in the first, second, third, fourth or fifth aspect; preferably, the isotopic substitution is a substitution with a deuterium atom.

In a seventh aspect, the present disclosure further provides a pharmaceutical composition comprising the compound or the pharmaceutically acceptable salt thereof set forth in the first, second, third, fourth or fifth aspect, or the isotopically substituted form set forth in the sixth aspect, and a pharmaceutically acceptable excipient.

In some embodiments, a unit dose of the pharmaceutical composition is 0.001 mg-1000 mg.

In certain embodiments, the pharmaceutical composition comprises 0.01-99.99% of the aforementioned compound or pharmaceutically acceptable salt thereof based on the total weight of the composition. In certain embodiments, the pharmaceutical composition comprises 0.1-99.9% of the aforementioned compound or pharmaceutically acceptable salt thereof. In certain embodiments, the pharmaceutical composition comprises 0.5%-99.5% of the aforementioned compound or pharmaceutically acceptable salt thereof. In certain embodiments, the pharmaceutical composition comprises 1%-99% of the aforementioned compound or pharmaceutically acceptable salt thereof. In certain embodiments, the pharmaceutical composition comprises 2%-98% of the aforementioned compound or pharmaceutically acceptable salt thereof.

In certain embodiments, the pharmaceutical composition comprises 0.01%-99.99% of a pharmaceutically acceptable excipient based on the total weight of the composition. In certain embodiments, the pharmaceutical composition comprises 0.1%-99.9% of a pharmaceutically acceptable excipient. In certain embodiments, the pharmaceutical composition comprises 0.5%-99.5% of a pharmaceutically acceptable excipient. In certain embodiments, the pharmaceutical composition comprises 1%-99% of a pharmaceutically acceptable excipient. In certain embodiments, the pharmaceutical composition comprises 2%-98% of a pharmaceutically acceptable excipient.

The present disclosure further provides a method for preventing and/or treating an LPA1-related condition in a patient, comprising administering to the patient a therapeutically effective amount of the compound or the pharmaceutically acceptable salt thereof set forth in the first, second, third, fourth or fifth aspect, or the isotopically substituted form set forth in the sixth aspect, or the pharmaceutical composition set forth in the seventh aspect.

The present disclosure further provides a method for preventing and/or treating an organ fibrotic disease, a respiratory disease, a renal disease, a hepatic disease, an inflammatory disease, a neurological disease, cardiovascular and cerebrovascular diseases, a gastrointestinal disease, pain, a urological disease, an ophthalmic disease, a metabolic disease, cancer or transplant rejection in a patient, comprising administering to the patient a therapeutically effective amount of the compound or the pharmaceutically acceptable salt thereof set forth in the first, second, third, fourth or fifth aspect, or the isotopically substituted form set forth in the sixth aspect, or the pharmaceutical composition set forth in the seventh aspect.

The present disclosure further provides use of the compound or the pharmaceutically acceptable salt thereof set forth in the first, second, third, fourth or fifth aspect, or the isotopically substituted form set forth in the sixth aspect, or the pharmaceutical composition set forth in the seventh aspect in the preparation of a medicament for preventing and/or treating LPA1-related conditions.

The present disclosure further provides use of the compound or the pharmaceutically acceptable salt thereof set forth in the first, second, third, fourth or fifth aspect, or the isotopically substituted form set forth in the sixth aspect, or the pharmaceutical composition set forth in the seventh aspect in the preparation of a medicament for preventing and/or treating organ fibrotic diseases, respiratory diseases, renal diseases, hepatic diseases, inflammatory diseases, neurological diseases, cardiovascular and cerebrovascular diseases, gastrointestinal diseases, pain, urological diseases, ophthalmic diseases, metabolic diseases, cancer or transplant rejection.

The present disclosure further provides a compound or a pharmaceutically acceptable salt thereof as set forth in the first, second, third, fourth or fifth aspect, or an isotopically substituted form as set forth in the sixth aspect, or a pharmaceutical composition as set forth in the seventh aspect for use in a medicament for preventing and/or treating LPA1-related conditions.

The present disclosure further provides a compound or a pharmaceutically acceptable salt thereof as set forth in the first, second, third, fourth or fifth aspect, or an isotopically substituted form as set forth in the sixth aspect, or a pharmaceutical composition as set forth in the seventh aspect for use in a medicament for preventing and/or treating organ fibrotic diseases, respiratory diseases, renal diseases, hepatic diseases, inflammatory diseases, neurological diseases, cardiovascular and cerebrovascular diseases, gastrointestinal diseases, pain, urological diseases, ophthalmic diseases, metabolic diseases, cancer or transplant rejection.

The organ fibrotic diseases include, but are not limited to: pulmonary fibrosis (especially idiopathic pulmonary fibrosis), renal fibrosis, hepatic fibrosis, skin fibrosis, intestinal fibrosis, and ocular fibrosis.

The respiratory diseases include, but are not limited to: respiratory disorders including asthma, chronic obstructive pulmonary disease (COPD), bronchospasm, cough, chronic cough, and respiratory failure.

The renal diseases include, but are not limited to: acute kidney injury and chronic kidney disease.

The hepatic diseases include, but are not limited to: hepatic diseases such as alcoholic steatohepatitis, nonalcoholic steatohepatitis, acute and chronic hepatitis, cirrhosis of the liver, and hepatic hypofunction.

The inflammatory diseases include, but are not limited to: autoimmune diseases, inflammation, arthritis, rheumatoid arthritis, scleroderma, Raynaud's phenomenon, and chronic pruritus.

The neurological diseases include, but are not limited to: senile dementia, Parkinson's disease, neurodegeneration, traumatic brain injury, epilepsy, mental diseases, and sleep disorders.

The cardiovascular and cerebrovascular diseases include but are not limited to: collagen vascular diseases, myocardial infarction, stroke, thrombosis, atherosclerosis, heart failure, and hypertension.

The gastrointestinal diseases include, but are not limited to: colon syndrome, inflammatory bowel disease, digestive tract diseases, and gastrointestinal dysfunction. The pain includes, but is not limited to: cancer pain, neuropathic pain, inflammatory pain, surgical pain, visceral pain, dental pain, premenstrual pain, central pain, pain caused by burns, migraine, or cluster headache, and chronic pain.

The urological diseases include urinary incontinence, dysuria, cystitis, prostatic hyperplasia, urinary dysfunction associated with prostatic hyperplasia, bladder neck sclerosis, and underactive bladder.

The ophthalmic diseases include macular degeneration and diabetic retinopathy.

The cancer includes, but is not limited to: breast cancer, pancreatic cancer, ovarian cancer, prostate cancer, glioblastoma, bone cancer, colon cancer, intestinal cancer, head and neck cancer, melanoma, multiple myeloma, chronic lymphocytic leukemia, and tumor metastasis.

The pharmaceutically acceptable salt of the compound described herein may be selected from the group consisting of inorganic or organic salts.

The compounds of the present disclosure may exist in specific geometric or stereoisomeric forms. The present disclosure contemplates all such compounds, including cis and trans isomers, (-)- and (+)-enantiomers, (R)- and (S)-enantiomers, diastereomers, (D)-isomer, (L)-isomer, and racemic mixtures and other mixtures thereof, such as enantiomerically or diastereomerically enriched mixtures, all of which are within the scope of the present disclosure. Additional asymmetric carbon atoms may be present in substituents such as an alkyl group. All such isomers and mixtures thereof are included within the scope of the present disclosure. The compounds of the present disclosure containing asymmetric carbon atoms can be isolated in optically active pure form or in racemic form. The optically active pure form can be isolated from a racemic mixture or synthesized using chiral starting materials or chiral reagents.

Optically active (R)- and (S)-enantiomers, and D- and L-isomers may be prepared by chiral synthesis, chiral reagents or other conventional techniques. If one enantiomer of a certain compound of the present disclosure is desired, it may be prepared by asymmetric synthesis or derivatization with a chiral auxiliary, wherein the resulting mixture of diastereomers is separated and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, followed by resolution of diastereomers by conventional methods known in the art, and the pure enantiomers are obtained by recovery. In addition, separation of enantiomers and diastereomers is generally accomplished by chromatography using a chiral stationary phase, optionally in combination with chemical derivatization (e.g., carbamate formation from amines).

In the chemical structure of the compound of the present invention, a bond " represents an unspecified configuration-that is, if chiral isomers exist in the chemical structure, the bond " 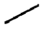 " may be " 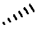 " or " 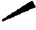 ", or includes both the configurations of " 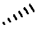 " and " 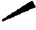 ". In the chemical structure of the compound of the present disclosure, a bond " 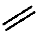 " does not specify a configuration-that is, the configuration for the bond " 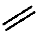 " can be an E configuration or a Z configuration, or includes both the E configuration and the Z configuration.

The compounds and intermediates of the present disclosure may also exist in different tautomeric forms, and all such forms are included within the scope of the present disclosure. The term "tautomer" or "tautomeric form" refers to structural isomers of different energies that can interconvert via a low energy barrier. For example, proton tautomers (also known as proton transfer tautomers) include interconversion via proton migration, such as keto-enol, imine-enamine, and lactam-lactim isomerization. An example of a lactam-lactim equilibrium is present between A and B as shown below:

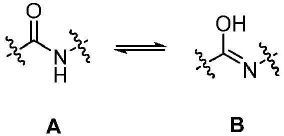

All the compounds in the present invention can be drawn as form A or form B. All tautomeric forms fall within the scope of the present invention. The names of the compounds do not exclude any tautomers.

The present disclosure further includes isotopically labeled compounds that are identical to those recited herein but have one or more atoms replaced by an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S , ¹⁸F, ¹²³I, ¹²⁵I and ³⁶Cl.

Unless otherwise specified, when a position is specifically assigned deuterium (D), the position should be construed as deuterium with an abundance that is at least 1000 times greater than the natural abundance of deuterium (which is 0.015%) (i.e., at least 10% deuterium incorporation). The compounds of examples comprise deuterium having an abundance that is greater than at least 1000 times the natural abundance, at least 2000 times the natural abundance, at least 3000 times the natural abundance, at least 4000 times the natural abundance, at least 5000 times the natural abundance, at least 6000 times the natural abundance, or higher times the natural abundance. The present disclosure further comprises various deuterated forms of the compounds. Each available hydrogen atom connected to a carbon atom may be independently replaced by a deuterium atom. Those skilled in the art can synthesize the compounds in deuterated form by reference to the relevant literature. Commercially available deuterated starting materials can be used in preparing the deuterated compounds, or they can be synthesized using conventional techniques with deuterated reagents including, but not limited to, deuterated borane, tri-deuterated borane in tetrahydrofuran, deuterated lithium aluminum hydride, deuterated iodoethane, deuterated iodomethane, and the like. "Optionally" or "optional" means that the event or circumstance subsequently described may, but does not necessarily, occur and that the description includes instances where the event or circumstance occurs or does not occur. For example, "C1-6 alkyl optionally substituted with halogen or cyano" means that halogen or cyano may, but does not necessarily, exist and that the description includes the instance where alkyl is substituted with halogen or cyano and the instance where alkyl is not substituted with halogen and cyano.

Terms and definitions:
"Pharmaceutical composition" refers to a mixture containing one or more of the compounds or the physiologically acceptable salts or pro-drugs thereof described herein, and other chemical components, for example, physiologically acceptable carriers and excipients. The pharmaceutical composition is intended to promote the administration to an organism, which facilitates the absorption of the active ingredient, thereby exerting biological activity.

"Pharmaceutically acceptable excipient" includes, but is not limited to, any adjuvant, carrier, excipient, glidant, sweetener, diluent, preservative, dye/colorant, flavoring agent, surfactant, wetting agent, dispersant, suspending agent, stabilizer, isotonic agent, solvent or emulsifier that has been approved by the U.S. food and drug administration as acceptable for use in humans or livestock animals.

"Effective amount" or "therapeutically effective amount" described herein includes an amount sufficient to ameliorate or prevent a symptom or condition of a medical disease. Effective amount also refers to an amount sufficient to allow or facilitate diagnosis. The effective amount for a particular patient or veterinary subject may vary with factors such as the condition to be treated, the general health of the patient, the method and route and dosage of administration, and the severity of side effects. Effective amount may be the maximum dose or administration regimen to avoid significant side effects or toxic effects.

"Alkyl" refers to a saturated aliphatic hydrocarbon group, including linear and branched groups of 1 to 20 carbon atoms, and alkyl containing 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, various branched isomers thereof, and the like. Alkyl may be substituted or unsubstituted, and when it is substituted, the substituent may be substituted at any accessible point of attachment, preferably one or more of the following groups, independently selected from the group consisting of halogen, hydroxy, oxo, cyano, amino, C₁₋₆ alkyl, C₁₋₆ alkoxy, 3-7 membered cycloalkyl and 3-7 membered heterocycloalkyl, wherein the alkyl, alkoxy, cycloalkyl or heterocycloalkyl is optionally substituted with halogen, hydroxy, nitro, cyano or amino.

The term "cycloalkyl" refers to a saturated or partially unsaturated, monocyclic or polycyclic hydrocarbon substituent. The cycloalkyl ring contains 3 to 20 carbon atoms, preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, and the like. Polycyclic cycloalkyl includes spirocycloalkyl, fused cycloalkyl and bridged cycloalkyl. Cycloalkyl may be substituted or unsubstituted, and when it is substituted, the substituent may be substituted at any accessible point of attachment, preferably one or more of the following groups, independently selected from the group consisting of halogen, hydroxy, oxo, cyano, amino, C₁₋₆ alkyl, C₁₋₆ alkoxy, 3-7 membered cycloalkyl and 3-7 membered heterocycloalkyl, wherein the alkyl, alkoxy, cycloalkyl or heterocycloalkyl is optionally substituted with halogen, hydroxy, nitro, cyano or amino.

The term "heterocyclyl" refers to a saturated or partially unsaturated, monocyclic or polycyclic hydrocarbon substituent, which contains 3 to 20 ring atoms, one or more of which are heteroatoms selected from the group consisting of nitrogen, oxygen and S(O)ₘ (where m is an integer of 0 to 2), but does not contain a ring moiety of -O-O-, -O-S- or -S-S-, and the other ring atoms are carbons. It preferably contains 3 to 12 ring atoms, 1 to 4 of which are heteroatoms; more preferably, it contains 3 to 6 ring atoms. Non-limiting examples of "heterocyclyl" include: 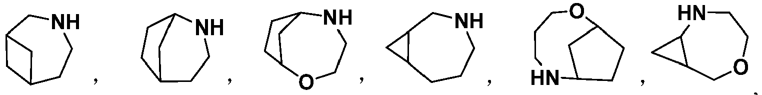 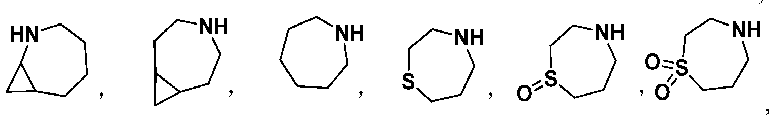 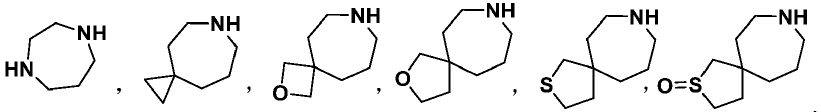 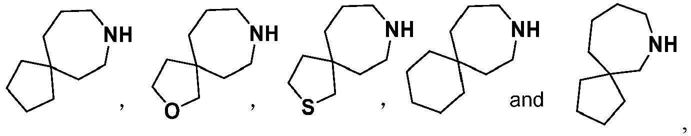 and the like.

The heterocyclyl ring may be fused to an aryl or heteroaryl ring, wherein the ring attached to the parent structure is heterocycloalkyl; its non-limiting examples include: 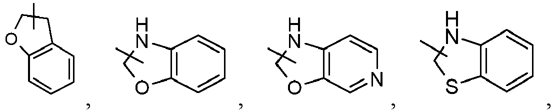 and the like.

Heterocyclyl may be optionally substituted or unsubstituted, and when it is substituted, the substituent is preferably one or more of the following groups independently selected from the group consisting of halogen, hydroxy, oxo, cyano, amino, C₁₋₆ alkyl, C₁₋₆ alkoxy, 3-6 membered cycloalkyl and 3-6 membered heterocycloalkyl, wherein the alkyl, alkoxy, cycloalkyl or heterocycloalkyl is optionally substituted with halogen, hydroxy, nitro, cyano or amino.

The term "alkoxy" refers to -O-(alkyl), wherein the alkyl is as defined above. Non-limiting examples of alkoxy include: methoxy, ethoxy, propoxy, and butoxy. Alkoxy may be optionally substituted or unsubstituted, and when it is substituted, the substituent is preferably one or more of the following groups independently selected from the group consisting of halogen, hydroxy, oxo, cyano, amino, C₁₋₆ alkyl, C₁₋₆ alkoxy, 3-7 membered cycloalkyl and 3-7 membered heterocycloalkyl, wherein the alkyl, alkoxy, cycloalkyl or heterocycloalkyl is optionally substituted with halogen, hydroxy, nitro, cyano or amino.

The term "monovalent group" refers to an atom or group obtained by "formally" removing a monovalent atom or group from a compound. "-ylene" refers to a group formed by "formally" removing two monovalent atoms or atomic groups or one divalent atom or atomic group from a compound. The term "alkylene" refers to the remainder of an alkane molecule after 2 hydrogen atoms are removed from the alkane molecule, including linear and branched -ylene groups of 1 to 20 carbon atoms. Non-limiting examples of alkylene containing 1 to 6 carbon atoms include methylene (-CH₂-) and ethylene (e.g., -CH₂CH₂- or -CH(CH₃)-). Unless otherwise specified, alkylene may be substituted or unsubstituted, and when it is substituted, the substituent may be substituted at any accessible point of attachment, preferably one or more of the following groups independently selected from the group consisting of halogen, hydroxy, cyano, amino, C₁₋₆ alkyl and C₁₋₆ alkoxy.

Likewise, the definitions of "alkyleneoxy", "alkenylene", "alkenyleneoxy", "cycloalkylene" and "heterocyclylene" are similar to that of "alkylene".

The term "aryl" refers to a 6-14 membered, preferably 6-12 membered, all-carbon monocyclic or fused polycyclic (i.e., rings sharing a pair of adjacent carbon atoms) group having a conjugated π-electron system, such as phenyl and naphthyl. The aryl ring may be fused to a heteroaryl, heterocycloalkyl or cycloalkyl ring, wherein the ring attached to the parent structure is an aryl ring; its non-limiting examples include:

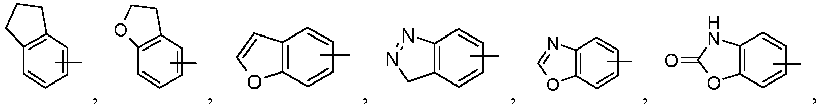

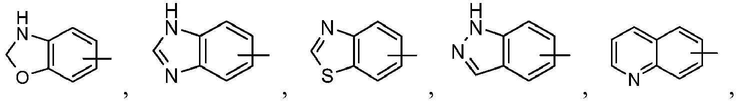

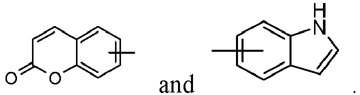

Aryl may be substituted or unsubstituted, and when it is substituted, the substituent is preferably one or more of the following groups independently selected from the group consisting of halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3-6 membered heterocycloalkoxy, C₃₋₈ cycloalkenyloxy, and 5-6 membered aryl or heteroaryl, wherein the C₁₋₆ alkyl, C₁₋₆ alkoxy, C₂₋₆ alkenyloxy, C₂₋₆ alkynyloxy, C₃₋₆ cycloalkoxy, 3-6 membered heterocycloalkoxy, C₃₋₈ cycloalkenyloxy, or 5-6 membered aryl or heteroaryl is optionally substituted with one or more groups selected from the group consisting of halogen, hydroxy, cyano, amino, C₁₋₆ alkyl and C₁₋₆ alkoxy.

The term "heteroaryl" refers to a heteroaromatic system containing 1 to 4 heteroatoms and 5 to 14 ring atoms, wherein the heteroatoms are selected from the group consisting of oxygen, sulfur and nitrogen. Heteroaryl is preferably 6- to 12-membered, and is more preferably 5- or 6-membered. For example, its non-limiting examples include: imidazolyl, furanyl, thienyl, thiazolyl, pyrazolyl, oxazolyl, isoxazolyl, pyrrolyl, tetrazolyl, pyridinyl, pyrimidinyl, thiadiazole, pyrazinyl, triazolyl, indazolyl, benzimidazolyl, 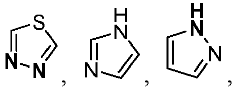 and the like.

The heteroaryl ring may be fused to an aryl, heterocycloalkyl or cycloalkyl ring, wherein the ring attached to the parent structure is a heteroaryl ring; its non-limiting examples include:

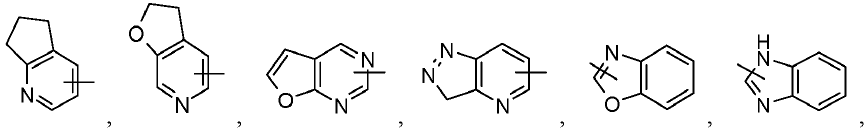

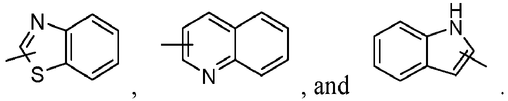

Heteroaryl may be optionally substituted or unsubstituted, and when it is substituted, the substituent is preferably one or more of the following groups independently selected from the group consisting of halogen, hydroxy, cyano, amino, C₁₋₆ alkyl and C₁₋₆ alkoxy. The term "spiro" refers to compounds in which two rings share one atom. Non-limiting examples of spirocycloalkyl include:

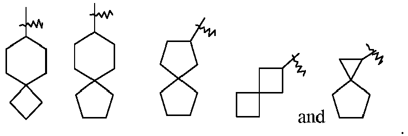

The term "fused" refers to compounds formed by fusing two or more rings by sharing two adjacent atoms. Non-limiting examples of fused cycloalkyl include:

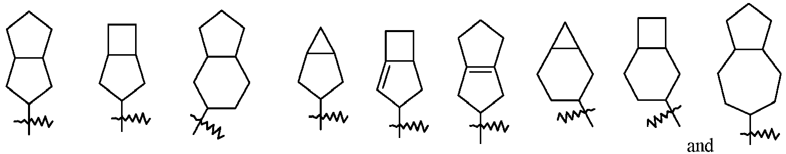

The term "bridged" refers to a structure formed by two or more cyclic structures sharing two non-adjacent ring atoms. According to the number of constituent rings, bridged cycloalkyl may be bicyclic, tricyclic, tetracyclic or polycyclic bridged cycloalkyl, preferably bicyclic, tricyclic or tetracyclic bridged cycloalkyl, and more preferably bicyclic or tricyclic bridged cycloalkyl. Non-limiting examples of bridged cycloalkyl include:

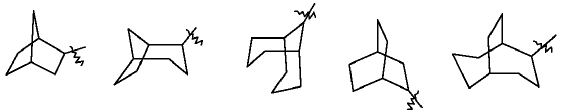

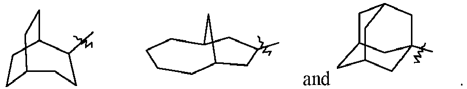

The term "hydroxy" refers to the -OH group.

The term "halogen" refers to fluorine, chlorine, bromine or iodine.

The term "cyano" refers to -CN.

The term "amino" refers to -NH₂.

The term "nitro" refers to -NO₂.

The term "oxo" refers to the =O substituent.

The term "carboxyl" refers to COOH.

"Substituted" means that one or more, preferably up to 5, more preferably 1 to 3 hydrogen atoms in the group are independently substituted with a corresponding number of substituents. When the substituent is ketone or oxo (i.e., =O), then two (2) hydrogens on the atom are replaced.

### DETAILED DESCRIPTION

The present disclosure is further described below with reference to examples, which are not intended to limit the scope of the present disclosure.

Experimental procedures without conditions specified in the examples of the present disclosure were generally conducted according to conventional conditions, or according to conditions recommended by the manufacturers of the starting materials or commercial products. Reagents without specified origins are commercially available conventional reagents.

The structures of the compounds were determined by nuclear magnetic resonance (NMR) spectroscopy and/or mass spectrometry (LCMS). NMR shifts (δ) are given in 10⁻⁶ (ppm). NMR analysis was performed on a Bruker AVANCE-400 nuclear magnetic resonance instrument, with dimethyl sulfoxide-D6 (DMSO-*d₆*), chloroform-D (CDCl₃) and methanol-D4 (CD₃OD) as solvents and tetramethylsilane (TMS) as an internal standard. The spatial configurations of the optical isomers (isomers) of the compounds can be further confirmed by determining single crystal parameters.

High performance liquid chromatography (HPLC) analysis was performed on Waters ACQUITY ultra high performance LC, Shimadzu LC-20A systems, Shimadzu LC-2010HT series, or Agilent 1200 LC high performance liquid chromatograph (ACQUITYUPLC BEH C18 1.7 µm 2.1 × 50 mm column, Ultimate XB-C18 3.0 × 150 mm column, or Xtimate C18 2.1 × 30 mm column).

Mass spectrometry (MS) analysis was performed on a Waters SQD2 mass spectrometer in the positive/negative ion scan mode with a mass scan range of 100-1200.

Chiral HPLC analysis was performed using Chiralpak IG-3 100 × 4.6 mm I.D., 3 µm; Chiralpak AD-3 150 × 4.6 mm I.D., 3 µm; Chiralpak IH-3 50 × 4.6 mm I.D., 3 µm; Chiralpak AS-3 150 × 4.6 mm I.D., 3 µm; Chiralpak AS-3 100 × 4.6 mm I.D., 3 µm; and Chiralcel OJ-3 100 × 4.6 mm I.D., 3 µm chromatography columns.

Yantai Huanghai HSGF254 or Qingdao GF254 silica gel plates, 0.15 mm-0.2 mm layer thickness, were adopted for thin-layer chromatography (TLC) analysis and 0.4 mm-0.5 mm layer thickness for TLC separation and purification.

The flash column chromatography system used was Combiflash Rf150 (TELEDYNE ISCO) or Isolara one (Biotage).

Normal-phase column chromatography generally used 100-200 mesh, 200-300 mesh or 300-400 mesh Yantai Huanghai silica gel as a carrier, or used a Changzhou Santai pre-fill ultrapure normal-phase silica gel column (40-63 µm, 60 g, 12 g, 25 g, 40 g, 80 g or other specifications).

Reversed-phase column chromatography generally used a Changzhou Santai pre-fill ultrapure C18 silica gel column (20-45 µm, 100 Å, 40 g, 80 g, 120 g, 220 g or other specifications).

High pressure column purification was performed on Waters AutoP equipped with a Waters XBridge BEH C18 OBD Prep Column, 130 Å, 5 µm, 19 mm × 150 mm or Atlantis T3 OBD Prep Column, 100 Å, 5 µm, 19 mm × 150 mm.

The preparative chiral column used was DAICEL CHIRALPAK AS (250 mm × 30 mm, 10 µm), DAICEL CHIRALPAK AD (250 mm × 30 mm, 10 µm), DAICEL CHIRALCEL OJ (250 mm × 30 mm, 10 µm), DAICEL CHIRALPAK IG (250 mm × 30 mm, 10 µm) or Waters SFC150AP ChiralPak IH (250 mm × 30 mm, 10 µm).

Known starting materials in the present disclosure may be synthesized using or according to methods known in the art, or may be purchased from Shanghai Titan Scientific, ABCR GmbH & Co. KG, Acros Organics, Aldrich Chemical Company, Accela ChemBio Inc., Darui Chemicals, and other companies.

In the examples, reactions can all be performed in a nitrogen atmosphere unless otherwise specified.

The argon atmosphere or nitrogen atmosphere means that the reaction flask is connected to a balloon containing about 1 L of argon or nitrogen gas.

The hydrogen atmosphere means that the reaction flask is connected to a balloon containing about 1 L of hydrogen gas.

Pressurized hydrogenation reactions were performed using a Parr 3916EKX hydrogenator and a Qinglan QL-500 hydrogenator, or an HC2-SS hydrogenator. Hydrogenation reactions generally involved 3 cycles of vacuumization and hydrogen filling.

Microwave reactions were performed on a CEM Discover-S 908860 microwave reactor. In the examples, the solutions were aqueous solutions unless otherwise specified.

In the examples, the reaction temperature was room temperature, i.e., 20 °C-30 °C, unless otherwise specified.

In the examples, the reaction processes were monitored by thin-layer chromatography (TLC).

### Example 1

### 5-((2-Methyl-6-(1-methyl-5-(((4-(trifluoromethyl)pyrimidin-2-yl)oxy)methyl)-1H-1,2,3 -triazol-4-yl)pyridin-3-yl)oxy)octahydropentalene-1-carboxylic acid (1)

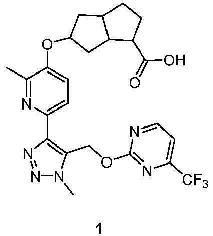

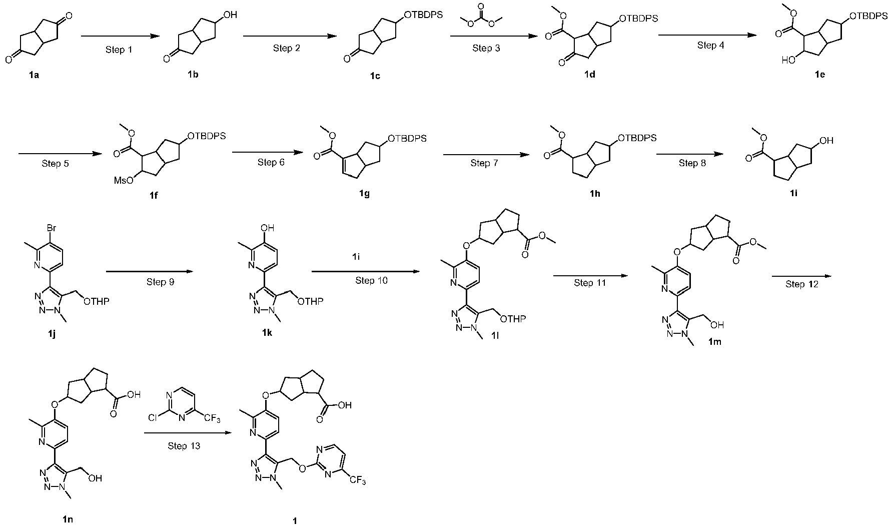

### Step 1: 5-hydroxyhexahydropentalen-2(1H)-one (1b)

The starting material cis-tetrahydropentalene-2,5(1*H*,3*H*)-dione **1a** (24 g, 173.7 mmol) was dissolved in ethanol (500 mL), and the solution was cooled to 0 °C. Sodium borohydride (1.47 g, 43.4 mmol) was added, and the mixture was stirred at 0 °C for 2 h. The reaction mixture was quenched with acetic acid (10 mL) and concentrated, and the residue was purified by silica gel flash column chromatography (petroleum ether/ethyl acetate) to give the title product **1b** (9 g, 37.0% yield).

¹H NMR: (400 MHz, CDCl₃) δ 4.55-4.37 (m, 1H), 2.90-2.75 (m, 2H), 2.64-2.48 (m, 2H), 2.36-2.14 (m, 4H), 1.65-1.49 (m, 3H).

### Step 2: 5-((tert-butyldiphenylsilyl)oxy)hexahydropentalen-2(1H)-one (1c)

**1b** (15 g, 107.0 mmol) was dissolved in dichloromethane (150 mL), and imidazole (21.8 g, 321.0 mmol) was added. After the mixture was cooled to 0 °C, tert-butyldiphenylchlorosilane (30.5 mL, 117.7 mmol) was added, and the mixture was stirred at 0 °C for 1 h. The reaction mixture was washed with 1 M hydrochloric acid solution (150 mL × 3) and saturated brine (300 mL), dried over anhydrous sodium sulfate, and filtered. The filtrate was concentrated, and the residue was purified by silica gel flash column chromatography (petroleum ether/ethyl acetate) to give the title product **1c** (41 g, crude). The product was directly used in the next step without further purification.

### Step 3: methyl 5-((tert-butyldiphenylsilyl)oxy)-2-oxooctahydropentalene-1-carboxylate (1d)

A solution of **1c** (41 g, 108.3 mmol) in tetrahydrofuran (500 mL) was cooled to 0 °C, and after 60% sodium hydride (17.3 g, 433.2 mmol) was added, dimethyl carbonate (19.5 g, 216.6 mmol) was added. The mixture was heated to 60 °C and stirred for 3 h. Water (150 mL) was added to quench the reaction. The reaction mixture was extracted with ethyl acetate (100 mL × 3). The organic phases were combined, washed with saturated brine (300 mL), dried over anhydrous sodium sulfate, and filtered. The filtrate was concentrated, and the residue was purified by silica gel flash column chromatography (petroleum ether/ethyl acetate) to give the title product **1d** (25 g, 52.9% yield).

MS (ESI): m/z = 459.1 [M+Na]⁺.

### Step 4: methyl 5-((tert-butyldiphenylsilyl)oxy)-2-hydroxyoctahydropentalene-1-carboxylate (1e)

**1d** (32 g, 73.3 mmol) was dissolved in tetrahydrofuran (350 mL), and the solution was cooled to 0 °C. Sodium borohydride (2.48 g, 73.3 mmol) was added, and the mixture was stirred at 0 °C for 3 h. The reaction mixture was quenched with 0.5 M hydrochloric acid solution (30 mL) and extracted with ethyl acetate (30 mL × 3). The organic phases were combined, washed with saturated brine (30 mL), dried over anhydrous sodium sulfate, and filtered. The filtrate was concentrated, and the residue was purified by silica gel flash column chromatography (petroleum ether/ethyl acetate) to give the title product **1e** (9 g, 28.0% yield).

MS (ESI): m/z = 461.2 [M+Na]⁺.

### Step 5: methyl 5-((tert-butyldiphenylsilyl)oxy)-2-((methylsulfonyl)oxy) octahydropentalene-1-carboxylate (1f)

**1e** (9 g, 20.5 mmol) was dissolved in dichloromethane (20 mL), and triethylamine (5.19 g, 51.3 mmol) was added. The mixture was cooled to 0 °C, and after methanesulfonic anhydride (5.36 g, 30.8 mmol) was added, the reaction mixture was warmed to room temperature and stirred for 3 h. The reaction mixture was washed with water (25 mL × 2) and saturated brine (30 mL), dried over anhydrous sodium sulfate, and filtered. The filtrate was concentrated, and the residue was purified by silica gel flash column chromatography (petroleum ether/ethyl acetate) to give the title product 1**f** (9 g, 84.9% yield).

MS (ESI): m/z = 539.1 [M+Na]⁺.

### Step 6: methyl 5-((tert-butyldiphenylsilyl)oxy)-3,3a,4,5,6,6a-hexahydropentalene-1-carboxylate (1g)

**If** (9 g, 17.4 mmol) was dissolved in toluene (100 mL), and 1,8-diazabicyclo[5.4.0]undec-7-ene (5.3 g, 34.8 mmol) was added. The mixture was heated to 90 °C and stirred for 2 h. The reaction mixture was concentrated, and the residue was purified by silica gel flash column chromatography (petroleum ether/ethyl acetate) to give the title product **1g** (6.5 g, 88.7% yield).

MS (ESI): m/z = 443.2 [M+Na]⁺.

### Step 7: methyl 5-((tert-butyldiphenylsilyl)oxy)octahydropentalene-1-carboxylate (1h)

**1g** (6.5 g, 15.5 mmol) was dissolved in methanol (65 mL), and 10% Pd/C (600 mg) was added in a nitrogen atmosphere. After the mixture was purged with hydrogen several times, the reaction mixture was stirred at room temperature for 3 h in a hydrogen atmosphere (15 psi). The reaction mixture was filtered, and the filtrate was concentrated to give the title product **1h** (crude, 6.5 g). The product was directly used in the next step. MS (ESI): m/z = 445.2 [M+Na]⁺.

### Step 8: methyl 5-hydroxyoctahydropentalene-1 -carboxylate (1i)

**1h** (6.3 g, 14.9 mmol) was dissolved in tetrahydrofuran (60 mL), and a 1 M solution of tetrabutylammonium fluoride in tetrahydrofuran (16.4 mL, 16.4 mmol) was added. The mixture was left to react at room temperature for 3 h. Water (30 mL) was added to the reaction mixture, and the reaction mixture was extracted with ethyl acetate (20 mL × 3). The organic phases were combined, washed with saturated brine (60 mL), dried over anhydrous sodium sulfate, and filtered. The filtrate was concentrated, and the residue was purified by silica gel flash column chromatography (petroleum ether/ethyl acetate) to give the title product **1i** (2.1 g, 76.5% yield).

¹H NMR (400 MHz, CDCl₃) δ 4.03 (tt, *J* = 9.8, 6.1 Hz, 1H), 3.65 (s, 3H), 2.75-2.61 (m, 2H), 2.50-2.38 (m, 1H), 2.26-2.15 (m, 1H), 2.02-1.88 (m, 2H), 1.83-1.74 (m, 2H), 1.66-1.49 (m, 2H), 1.22-1.07 (m, 2H).

### Step 9: 2-methyl-6-(1-methyl-5-(((tetrahydro-2H-pyran-2-yl)oxy)methyl)-1H-1,2,3-triazol-4-yl)pyridin-3-ol (1k)

3-Bromo-2-methyl-6-(1-methyl-5-((tetrahydro-2*H*-pyran-2-yl)oxy)methyl)-1*H*-1,2,3-tri azol-4-yl)pyridine **1j** (2.5 g, 6.81 mmol, prepared using the method disclosed in Example 1 in the specification of the patent application WO2017223016A1), bis(dibenzylideneacetone)palladium (391 mg, 0.68 mmol), 2-di-tert-butylphosphino-2',4',6'-triisopropylbiphenyl (578 mg, 1.36 mmol) and potassium hydroxide (1.15 g, 20.44 mmol) were dissolved in 1,4-dioxane (50 mL) and water (10 mL), and the mixture was left to react at 100 °C for 2 h in a nitrogen atmosphere. The pH was adjusted to 6-7 with 1 M hydrochloric acid solution, and water (10 mL) was added. The mixture was extracted with ethyl acetate (50 mL × 2), and the organic phases were combined, washed with saturated brine, dried over anhydrous sodium sulfate, filtered, and concentrated to give the crude product. The crude product was purified by silica gel flash column chromatography (ethyl acetate/petroleum ether) to give the title product **1k** (1.9 g, 92% yield).

MS (ESI) m/z = 305.6 [M+H]⁺.

### Step 10: methyl 5-((2-methyl-6-(1-methyl-5-(((tetrahydro-2H-pyran-2-yl)oxy)methyl)-1H-1,2,3-triazol-4-yl)pyridin-3-yl)oxy)octahydropentalene-1-carboxylate (1l)

**1k** (1 g, 3.29 mmol), triphenylphosphine (2.59 g, 9.86 mmol) and **1i** (605 mg, 3.29 mmol) were dissolved in tetrahydrofuran (50 mL), and the solution was heated to 50 °C. A solution of di-tert-butyl azodicarboxylate (2.25 g, 9.86 mmol) in tetrahydrofuran (10 mL) was slowly added dropwise, and the mixture was stirred at 50 °C for 2 h. The reaction mixture was concentrated under reduced pressure to give a crude product, and the crude product was purified through a reversed-phase C18 column (acetonitrile/water) to give the title product **1l** (1.2 g, 77% yield).

MS (ESI) m/z = 471.8 [M+H]⁺.

### Step 11: methyl 5-((6-(5-(hydroxymethyl)-1-methyl-1H-1,2,3-triazol-4-yl)-2-methylpyridin-3-yl)oxy)octahydropentalene-1-carboxylate (1m)

**1l** (1.2 g, 2.55 mmol) was dissolved in methanol (30 mL), and p-toluenesulfonic acid hydrate (194 mg, 1.02 mmol) was added. The reaction mixture was heated to 70 °C and left to react for 2 h in a nitrogen atmosphere. The reaction mixture was cooled and concentrated under reduced pressure to give the title product **1m** (crude, 900 mg). The product was directly used in the next step.

MS (ESI) m/z = 387.7 [M+H]⁺.

### Step 12: 5-((6-(5-(hydroxymethyl)-1-methyl-1H-1,2,3-triazol-4-yl)-2-methylpyridin-3-yl)oxy)octahydropentalene-1-carboxylic acid (1n)

**1m** (900 mg, 2.33 mmol) was dissolved in tetrahydrofuran (10 mL), methanol (10 mL) and water (10 mL), and lithium hydroxide (536 mg, 23.29 mmol) was then added. The mixture was left to react at room temperature for 3 h. The pH was adjusted to 5-6 with 1 M hydrochloric acid solution, and water (10 mL) was added. The mixture was extracted with ethyl acetate (30 mL × 2), and the organic phases were combined, washed with saturated brine, dried over anhydrous sodium sulfate, and filtered. The filtrate was concentrated to give the title product **1n** (800 mg, 82% yield).

MS (ESI) m/z = 373.6 [M+H]⁺.

### Step 13: 5-((2-methyl-6-(1-methyl-5-(((4-(trifluoromethyl)pyrimidin-2-yl)oxy)methyl)-1H-1,2,3-triazol-4-yl)pyridin-3-yl)oxy)octahydropentalene-1-carboxylic acid (1)

**1n** (800 mg, 2.15 mmol) was dissolved in N,N-dimethylacetamide (20 mL), and 60% sodium hydride (412 mg, 17.18 mmol) was added under ice bath conditions. After 30 min of reaction, 2-chloro-4-(trifluoromethyl)pyrimidine (588 mg, 3.22 mmol) was added, and the mixture was stirred at room temperature for 30 min. Water (10 mL) was added, and the pH was adjusted to 4-5 with 1 M hydrochloric acid. The mixture was extracted with ethyl acetate (20 mL × 2), and the organic phases were combined, washed with saturated brine, dried over anhydrous sodium sulfate, and filtered. The filtrate was concentrated to give a crude product, and the crude product was purified by reversed-phase flash column chromatography (acetonitrile/water) and then lyophilized to give the title product **1** (800 mg, 72% yield).

MS (ESI) m/z = 519.8 [M+H]⁺.

¹H NMR (400 MHz, DMSO-*d*₆) δ 9.03 (d, *J* = 4.9 Hz, 1H), 7.87 (d, *J* = 8.5 Hz, 1H), 7.70 (d, *J* = 4.9 Hz, 1H), 7.44 (d, *J* = 8.6 Hz, 1H), 6.10 (s, 2H), 5.02-4.94 (m, 1H), 4.16 (s, 3H), 2.94-2.84 (m, 1H), 2.78-2.64 (m, 2H), 2.15 (s, 3H), 2.13-2.04 (m, 1H), 1.91-1.81 (m, 1H), 1.73-1.55 (m, 3H), 1.52-1.34 (m, 3H).

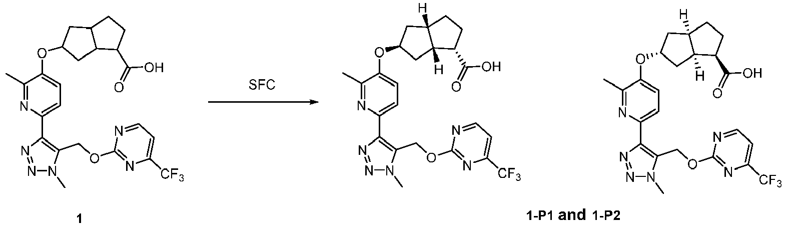

Compound **1** was resolved by preparative chiral SFC (resolution conditions: preparative chiral SFC column DAICEL CHIRALPAK AS (250 mm × 30 mm, 10 µm); mobile phases: A was CO₂, and B was isopropanol (0.1% ammonia water); flow rate: 70 mL/min). The corresponding fractions were collected and concentrated under reduced pressure to give compounds **1-P1** and **1-P2.**

Compound **1-P1** was the one with a shorter retention time: MS m/z (ESI): 519.4 [M+1]⁺; chiral SFC analysis: retention time 2.962 min, chiral purity 100% (column: Chiralpak AS-3, 0.46 cm I.D. × 100 mm, 3 µm; mobile phases: A was CO₂, and B was isopropanol (0.05% diethylamine).

¹H NMR (400 MHz, DMSO-*d*₆) δ 12.03 (s, 1H), 9.03 (d, *J* = 4.9 Hz, 1H), 7.86 (d, *J*= 8.5 Hz, 1H), 7.70 (d, *J* = 4.9 Hz, 1H), 7.42 (d, *J* = 8.6 Hz, 1H), 6.11 (s, 2H), 5.03-4.92 (m, 1H), 4.16 (s, 3H), 2.94-2.83 (m, 1H), 2.76-2.62 (m, 2H), 2.14 (s, 3H), 2.10-2.05 (m, 1H), 1.91-1.81 (m, 1H), 1.73-1.56 (m, 3H), 1.53-1.35 (m, 3H).

Compound **1-P2** was the one with a longer retention time: MS m/z (ESI): 519.4 [M+l]⁺; chiral SFC analysis: retention time 3.461 min, chiral purity 99.1% (column: Chiralpak AS-3, 0.46 cm I.D. × 100 mm, 3 µm; mobile phases: A was CO₂, and B was isopropanol (0.05% diethylamine).

¹H NMR (400 MHz, DMSO-*d*₆) δ 12.04 (s, 1H), 9.03 (d, *J* = 4.9 Hz, 1H), 7.86 (d, *J* = 8.5 Hz, 1H), 7.70 (d, *J* = 4.9 Hz, 1H), 7.42 (d, *J* = 8.7 Hz, 1H), 6.11 (s, 2H), 5.01-4.92 (m, 1H), 4.16 (s, 3H), 2.98-2.82 (m, 1H), 2.77-2.62 (m, 2H), 2.14 (s, 3H), 2.11-2.04 (m, 1H), 1.90-1.81 (m, 1H), 1.72-1.55 (m, 3H), 1.51-1.35 (m, 3H).

### Example 2

### 5-((6-(5-(((5-Cyclopropyl-1,2,4-oxadiazol-3-yl)oxy)methyl)-1-methyl-1H-1,2,3-triazol-4-yl)-2-methylpyridin-3-yl)oxy)octahydropentalene-1-carboxylic acid (2)

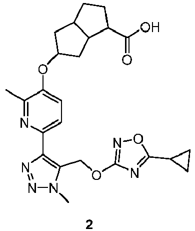

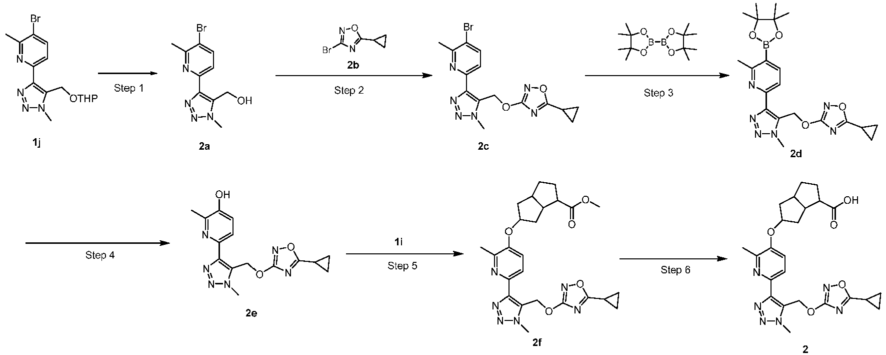

### Step 1: (4-(5-bromo-6-methylpyridin-2-yl)-1-methyl-1H-1,2,3-triazol-5-yl)methanol (2a)

**1j** (31 g, 84.4 mmol) was dissolved in methanol (300 mL), and pyridinium p-toluenesulfonate (31.8 g, 126.6 mmol) was added. The mixture was heated to 60 °C and stirred for 1 h. The reaction mixture was concentrated, and water (150 mL) was added. The mixture was extracted with ethyl acetate (300 mL × 3). The organic phases were combined, washed with saturated brine (100 mL), dried over anhydrous sodium sulfate, and filtered. The filtrate was concentrated to give the title product **2a** (crude, 24 g), and the product was directly used in the next step.

MS (ESI): m/z = 283.4 [M+H]⁺.

### Step 2: 3-((4-(5-bromo-6-methylpyridin-2-yl)-1-methyl-1H-1,2,3-triazol-5-yl)methoxy)-5-cyclo propyl-1,2,4-oxadiazole (2c)

**2a** (850 mg, 3.0 mmol) was dissolved in tetrahydrofuran (10 mL), and the solution was cooled to 0 °C. 60% sodium hydride (240 mg, 6 mmol) was added, and after the mixture was stirred at 0 °C for 30 min, 3-bromo-5-cyclopropyl-1,2,4-oxadiazole **2b** (567.4 mg, 3.0 mmol) was added. The reaction mixture was warmed to room temperature and stirred for 18 h. Water (5 mL) was added to quench the reaction, and the reaction mixture was extracted with ethyl acetate (10 mL × 3). The organic phases were combined, washed with saturated brine, dried over anhydrous sodium sulfate, and filtered. The filtrate was concentrated, and the residue was purified by silica gel flash column chromatography (petroleum ether/ethyl acetate) to give the title product **2c** (700 mg, 59.6% yield).

MS (ESI): m/z = 390.7 [M+H]⁺.

### Step 3: 5-cyclopropyl-3-((1-methyl-4-(6-methyl-5-(4,4,5,5-tetramethyl-1,3,2-dioxaborolan-2-yl )pyridin-2-yl)-1H-1,2,3-triazol-5-yl)methoxy)-1,2,4-oxadiazole (2d)

**2c** (460 mg, 1.18 mmol) and bis(pinacolato)diboron (358.3 mg, 1.41 mmol) were dissolved in dioxane (5 mL) in a nitrogen atmosphere, and [1,1'-bis(dibenzylphosphino)ferrocene]palladium dichloride (87.2 mg, 0.12 mmol) and potassium acetate (288.5 mg, 2.94 mmol) were added. The mixture was heated to 100 °C and left to react for 12 h. The reaction mixture was concentrated, and the residue was purified by silica gel flash column chromatography (petroleum ether/ethyl acetate) to give the title product **2d** (200 mg, 38.8% yield).

MS (ESI): m/z = 357.0 [M-82+H]⁺.

### Step 4: 6-(5-(((5-cyclopropyl-1,2,4-oxadiazol-3-yl)oxy)methyl)-1-methyl-1H-1,2,3-triazol-4-yl) -2-methylpyridin-3-ol (2e)

**2d** (50.0 mg, 0.11 mmol) was dissolved in ethyl acetate (1 mL), and 30% hydrogen peroxide solution (0.11 mL, 1.14 mmol) was added. The mixture was left to react at room temperature for 1 h. A saturated sodium sulfite solution (1 mL) was added to quench the reaction, and the reaction mixture was extracted with ethyl acetate (1 mL × 3). The organic phases were combined, washed with saturated brine, dried over anhydrous sodium sulfate, and filtered. The filtrate was concentrated, and the residue was purified by silica gel flash column chromatography (petroleum ether/ethyl acetate) to give the title product **2e** (20 mg, 53.4% yield).

MS (ESI): m/z = 328.9 [M+H]⁺.

### Step 5: methyl 5-((6-(5-(((5-cyclopropyl-1,2,4-oxadiazol-3-yl)oxy)methyl)-1-methyl-1H-1,2,3-triazol-4 -yl)-2-methylpyridin-3-yl)oxy)octahydropentalene-1 -carboxylate (2f)

**2e** (100 mg, 0.31 mmol) and **1i** (72.9 mg, 0.40 mmol) were dissolved in toluene (3 mL), and triphenylphosphine (184.9 mg, 0.91 mmol) and 1,1'-(azodicarbonyl)dipiperidine (230.5 mg, 0.91 mmol) were added. The reaction mixture was heated to 80 °C and stirred for 12 h. The reaction mixture was concentrated, and the residue was purified by silica gel flash column chromatography (petroleum ether/ethyl acetate) to give the title product **2f** (60 mg, 39.8% yield).

MS (ESI): m/z = 495.3 [M+H]⁺.

### Step 6: 5-((6-(5-(((5-cyclopropyl-1,2,4-oxadiazol-3-yl)oxy)methyl)-1-methyl-1H-1,2,3-triazol-4 -yl)-2-methylpyridin-3-yl)oxy)octahydropentalene-1-carboxylic acid (2)

**2f** (50 mg, 0.10 mmol) was dissolved in tetrahydrofuran (1 mL) and water (1 mL), and lithium hydroxide monohydrate (12.7 mg, 0.30 mmol) was added. The mixture was stirred at room temperature for 12 h. The pH of the reaction mixture was adjusted to around 7 with 1 M hydrochloric acid solution, and the reaction mixture was concentrated. The residue was purified by preparative HPLC (acetonitrile/water) to give the title compound **2** (6.2 mg, 12.8% yield).

MS (ESI): m/z = 481.2 [M+H]⁺.

¹H NMR (400 MHz, CD₃OD) δ 7.79 (d, *J* = 8.4 Hz, 1H), 7.36 (d, *J* = 8.5 Hz, 1H), 5.96 (s, 2H), 5.14-5.00 (m, 1H), 4.18 (s, 3H), 3.11-3.00 (m, 1H), 2.91-2.73 (m, 2H), 2.35 (s, 3H), 2.30-2.20 (m, 1H), 2.17-2.09 (m, 1H), 2.06-1.97 (m, 1H), 1.85-1.67 (m, 3H), 1.63-1.46 (m, 3H), 1.26-1.18 (m, 2H), 1.12-1.02 (m, 2H).

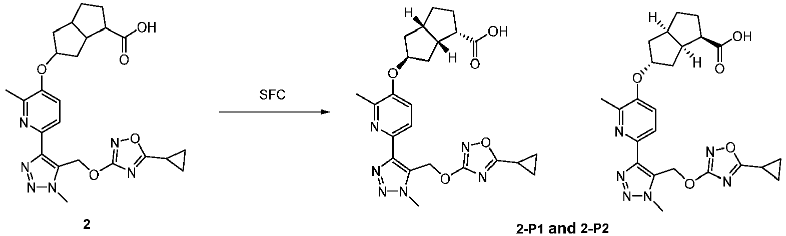

Compound **2** was resolved by preparative chiral SFC (resolution conditions: preparative chiral SFC column DAICEL CHIRALPAK AD (250 mm × 30 mm, 10 µm); mobile phases: A was CO₂, and B was ethanol (0.1% ammonia water); flow rate: 80 mL/min). The corresponding fractions were collected and concentrated under reduced pressure to give compounds **2-P1** and **2-P2.**

Compound **2-P1** was the one with a shorter retention time: MS m/z (ESI): 481.2 [M+H]⁺; chiral SFC analysis: retention time 3.427 min, chiral purity 99.2% (column: Chiralpak AD-3, 0.46 cm I.D. × 150 mm, 3 µm; mobile phases: A was CO₂, and B was ethanol (0.05% diethylamine).

¹H NMR (400 MHz, DMSO-*d*₆) δ 7.86 (d, *J* = 8.5 Hz, 1H), 7.43 (d, *J* = 8.6 Hz, 1H), 5.94 (s, 2H), 5.04-4.90 (m, 1H), 4.12 (s, 3H), 2.97-2.81 (m, 1H), 2.76-2.64 (m, 2H), 2.25 (s, 3H), 2.23-2.17 (m, 1H), 2.15-2.07 (m, 1H), 1.91-1.82 (m, 1H), 1.72-1.58 (m, 3H), 1.52-1.34 (m, 3H), 1.25-1.17 (m, 2H), 1.05-0.99 (m, 2H).

Compound **2-P2** was the one with a longer retention time: MS m/z (ESI): 481.2 [M+H]⁺; chiral SFC analysis: retention time 4.109 min, chiral purity 99.1% (column: Chiralpak AD-3, 0.46 cm I.D. × 150 mm, 3 µm; mobile phases: A was CO₂, and B was ethanol (0.05% diethylamine).

¹H NMR (400 MHz, DMSO-*d*₆) δ 7.86 (d, *J* = 8.5 Hz, 1H), 7.43 (d, *J* = 8.6 Hz, 1H), 5.94 (s, 2H), 5.05-4.93 (m, 1H), 4.12 (s, 3H), 2.97-2.84 (m, 1H), 2.76-2.65 (m, 2H), 2.25 (s, 3H), 2.23-2.17 (m, 1H), 2.15-2.06 (m, 1H), 1.90-1.82 (m, 1H), 1.73-1.57 (m, 3H), 1.52-1.35 (m, 3H), 1.25-1.17 (m, 2H), 1.05-0.99 (m, 2H).

### Example 3

### 5-((6-(5-(((4-(Methoxymethyl)pyrimidin-2-yl)oxy)methyl)-1-methyl-1H-1,2,3-triazol-4 -yl)-2-methylpyridin-3-yl)oxy)octahydropentalene-1-carboxylic acid (3)

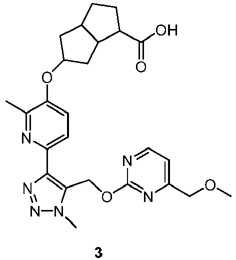

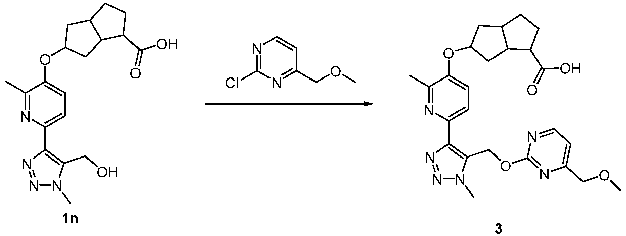

**1n** (350 mg, 0.94 mmol) was dissolved in N,N-dimethylacetamide (20 mL), and 60% sodium hydride (180 mg, 7.52 mmol) was added under ice bath conditions. After 30 min of reaction, 2-chloro-4-(methoxymethyl)pyrimidine (179 mg, 1.13 mmol) was added. The mixture was left to react at room temperature for 30 min. The reaction mixture was diluted with water (10 mL), and the pH was adjusted to 4-5 with 1 M hydrochloric acid. The mixture was extracted with ethyl acetate (20 mL × 2). The organic phases were combined, washed with saturated brine, dried over anhydrous sodium sulfate, and filtered. The filtrate was concentrated to give a crude product, and the crude product was purified by reversed-phase C18 column chromatography (acetonitrile/water) and then lyophilized to give the title compound **3** (200 mg, 43% yield).

MS (ESI) m/z = 495.5 [M+H]⁺.

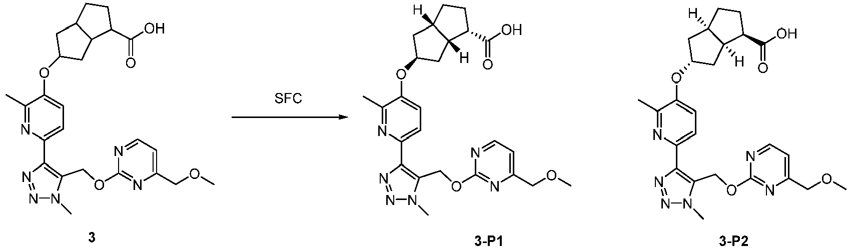

Compound **3** was resolved by preparative chiral SFC (resolution conditions: preparative chiral SFC column DAICEL CHIRALPAK AS (250 mm × 30 mm, 10 µm); mobile phases: A was CO₂, and B was ethanol (0.1% ammonia water)). The corresponding fractions were collected and concentrated under reduced pressure to give compounds **3-P1** and **3-P2.**

Compound **3-P1** was the one with a shorter retention time: MS m/z (ESI): 495.2 [M+H]⁺; chiral SFC analysis: retention time 2.717 min, chiral purity 100% (column: Chiralpak AS-3, 0.46 cm I.D. × 100 mm, 3 µm; mobile phases: A was CO₂, and B was ethanol (0.05% diethylamine).

¹H NMR (400 MHz, CDCl₃) δ 8.50 (d, *J* = 5.0 Hz, 1H), 7.90 (d, *J* = 8.5 Hz, 1H), 7.13 (d, *J* = 6.6 Hz, 2H), 6.16-6.01 (m, 2H), 4.86 (s, 1H), 4.36 (s, 2H), 4.18 (s, 3H), 3.45 (s, 3H), 3.15-2.98 (m, 1H), 2.91-2.77 (m, 2H), 2.34 (s, 3H), 2.30-2.21 (m, 1H), 2.09-2.00 (m, 1H), 1.87-1.77 (m, 2H), 1.75-1.64 (m, 1H), 1.57-1.40 (m, 3H).

Compound **3-P2** was the one with a longer retention time: MS m/z (ESI): 495.2 [M+H]⁺; chiral SFC analysis: retention time 3.031 min, chiral purity 97.4% (column: Chiralpak AS-3, 0.46 cm I.D. × 100 mm, 3 µm; mobile phases: A was CO₂, and B was ethanol (0.05% diethylamine).

¹H NMR (400 MHz, CDCl₃) δ 8.50 (d, *J* = 5.0 Hz, 1H), 7.91 (d, *J* = 8.5 Hz, 1H), 7.16-7.10 (m, 2H), 6.17-6.01 (m, 2H), 4.87 (s, 1H), 4.36 (s, 2H), 4.18 (s, 3H), 3.45 (s, 3H), 3.14-3.00 (m, 1H), 2.91-2.78 (m, 2H), 2.35 (s, 3H), 2.31-2.21 (m, 1H), 2.11-2.02 (m, 1H), 1.89-1.78 (m, 2H), 1.75-1.63 (m, 1H), 1.58-1.39 (m, 3H).

### Example 4

### 5-((6-(5-(((6-Ethylpyrimidin-4-yl)oxy)methyl)-1-methyl-1H-1,2,3-triazol-4-yl)-2-methy lpyridin-3-yl)oxy)octahydropentalene-1-carboxylic acid (4)

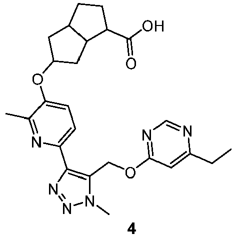

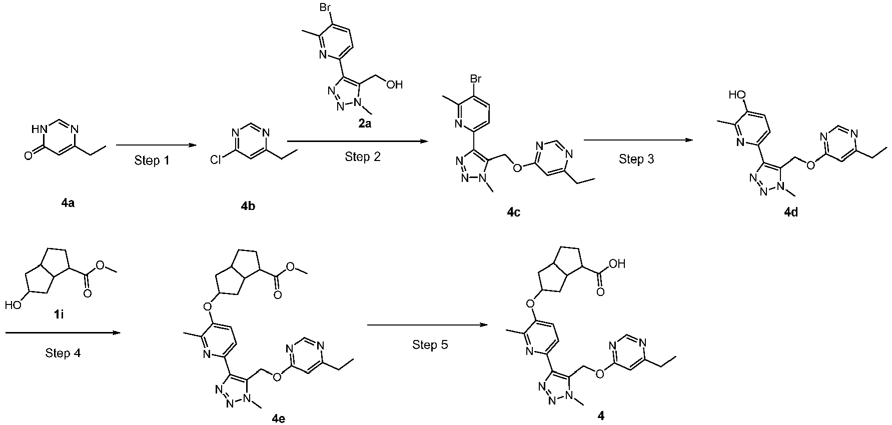

### Step 1: 4-chloro-6-ethylpyrimidine (4b)

6-Ethylpyrimidin-4-ol **4a** (300 mg, 2.4 mmol) was dissolved in phosphorus oxychloride (3 mL), and the solution was heated for 3 h in a nitrogen atmosphere. The remaining phosphorus oxychloride was removed under vacuum to give the title compound **4b** (crude, 300 mg), and the product was directly used in the next step.

MS (ESI) m/z = 143.3 [M+H]⁺.

### Step 2: 4-((4-(5-bromo-6-methylpyridin-2-yl)-1-methyl-1H-1,2,3-triazol-5-yl) methoxy)-6-ethylpyrimidine (4c)

**2a** (200 mg, 0.7 mmol) was dissolved in DMF (10 mL), and 60% sodium hydride (1.4 mmol, 56 mg) was added under ice bath conditions. After the reaction mixture was stirred in the ice bath for 20 min, **4b** (121 mg, 0.85 mmol) was added, and the mixture was stirred at room temperature for 2 h. Water (10 mL) was added, and the mixture was extracted with ethyl acetate (30 mL × 2). The organic phases were combined, washed with saturated brine, dried over anhydrous sodium sulfate, and filtered. The filtrate was concentrated to give a crude product, and the crude product was purified by flash column chromatography (ethyl acetate/petroleum ether) to give the title compound **4c** (200 mg, 73% yield).

MS (ESI) m/z = 389.5 [M+H]⁺.

### Step 3: 6-(5-(((6-ethylpyrimidin-4-yl)oxy)methyl)-1-methyl-1H-1,2,3-triazol-4-yl)-2-methylpyridin-3-ol (4d)

**4c** (200 mg, 0.51 mmol), [1,1'-bis(diphenylphosphino)ferrocene]palladium dichloride (38 mg, 0.05 mmol) and potassium hydroxide (86 mg, 1.5 mmol) were dissolved in 1,4-dioxane (5 mL) and water (1 mL) in a nitrogen atmosphere, and the solution was heated to 100 °C and stirred for 3 h. Water (10 mL) was added, and the mixture was extracted with ethyl acetate (30 mL × 2). The organic phases were combined, washed with saturated brine, dried over anhydrous sodium sulfate, and filtered. The filtrate was concentrated to give a crude product, and the crude product was purified by flash column chromatography (ethyl acetate/petroleum ether) to give the title compound **4d** (150 mg, 89% yield).

MS (ESI) m/z = 327.5 [M+H]⁺.

### Step 4: methyl 5-((6-(5-(((6-ethylpyrimidin-4-yl)oxy)methyl)-1-methyl-1H-1,2,3-triazol-4-yl)-2-methylpyridin-3-yl)oxy)octahydropentalene-1-carboxylate (4e)

**4d** (150 mg, 0.46 mmol), **1i** (127 mg, 0.67 mmol) and triphenylphosphine (301 mg, 1.15 mmol) were dissolved in dry tetrahydrofuran (20 mL) in a nitrogen atmosphere, and the solution was heated to 50 °C. A solution of di-tert-butyl azodicarboxylate (262 mg, 1.15 mmol) in tetrahydrofuran (5 mL) was slowly added dropwise, and after the addition, the mixture was stirred at 50 °C for 18 h. Water (10 mL) was added, and the mixture was extracted with ethyl acetate (50 mL × 2). The organic phases were combined, washed with saturated brine, dried over anhydrous sodium sulfate, and filtered. The filtrate was concentrated to give a crude product. The crude product was purified by flash column chromatography (ethyl acetate/petroleum ether) to give the title compound **4e** (150 mg, 60% yield).

MS (ESI) m/z = 493.7 [M+H]⁺.

### Step 5: 5-((6-(5-(((6-ethylpyrimidin-4-yl)oxy)methyl)-1-methyl-1H-1,2,3-triazol-4-yl)-2-methylpyridin-3-yl)oxy)octahydropentalene-1-carboxylic acid (4)

**4e** (150 mg, 0.3 mmol) was dissolved in tetrahydrofuran (5 mL) and methanol (5 mL), and lithium hydroxide monohydrate (128 mg, 3.05 mmol) and water (5 mL) were added. The mixture was left to react at room temperature. Water (10 mL) was added, and the pH was adjusted to 4-5 with 1 M hydrochloric acid. The mixture was extracted with ethyl acetate (30 mL × 2). The organic phases were combined, washed with saturated brine, dried over anhydrous sodium sulfate, and filtered. The filtrate was concentrated to give a crude product, and the crude product was purified by reversed-phase flash column chromatography (acetonitrile/water) and then lyophilized to give the title compound **4** (50 mg, 34% yield).

MS (ESI) m/z = 479.7 [M+H]⁺.

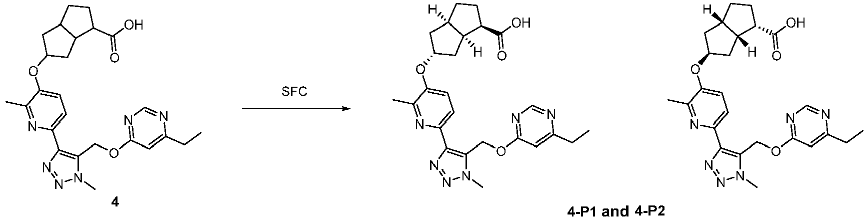

Compound **4** was resolved by preparative chiral SFC (resolution conditions: preparative chiral SFC column DAICEL CHIRALCEL OJ (250 mm × 30 mm, 10 µm); mobile phases: A was CO₂, and B was ethanol (0.1% ammonia water); flow rate: 70 mL/min). The corresponding fractions were collected and concentrated under reduced pressure to give compounds **4-P1** and **4-P2.**

Compound **4-P1** was the one with a shorter retention time: MS m/z (ESI): 479.2 [M+H]⁺; chiral SFC analysis: retention time 3.323 min, chiral purity 96.9% (column: Chiralcel OJ-3, 0.46 cm I.D. × 100 mm, 3 µm; mobile phases: A was CO₂, and B was ethanol (0.05% diethylamine); flow rate: 2.8 mL/min.

¹H NMR (400 MHz, CDCl₃) *δ* 8.72 (s, 1H), 7.93 (d, *J* = 8.5 Hz, 1H), 7.14 (d, *J* = 8.5 Hz, 1H), 6.63 (s, 1H), 6.13-6.03 (m, 2H), 4.93-4.87 (m, 1H), 4.14 (s, 3H), 3.14-3.02 (m, 1H), 2.91-2.80 (m, 2H), 2.71 (q, *J* = 7.6 Hz, 2H), 2.34 (s, 3H), 2.32-2.23 (m, 1H), 2.14-2.04 (m, 1H), 1.88-1.67 (m, 3H), 1.61-1.40 (m, 3H), 1.25 (t, *J* = 7.6 Hz, 3H).

Compound **4-P2** was the one with a longer retention time: MS m/z (ESI): 479.2 [M+H]⁺; chiral SFC analysis: retention time 4.303 min, chiral purity 100% (column: Chiralcel OJ-3, 0.46 cm I.D. × 100 mm, 3 µm; mobile phases: A was CO₂, and B was ethanol (0.05% diethylamine); flow rate: 2.8 mL/min.

¹H NMR (400 MHz, CDCl₃) *δ* 8.72 (s, 1H), 7.93 (d, *J* = 8.5 Hz, 1H), 7.14 (d, *J* = 8.6 Hz, 1H), 6.63 (d, *J* = 1.0 Hz, 1H), 6.14-5.99 (m, 2H), 4.91-4.86 (m, 1H), 4.14 (s, 3H), 3.14-3.01 (m, 1H), 2.91-2.79 (m, 2H), 2.71 (q, *J* = 7.6 Hz, 2H), 2.34 (s, 3H), 2.32-2.23 (m, 1H), 2.13-2.03 (m, 1H), 1.88-1.66 (m, 3H), 1.61-1.40 (m, 3H), 1.24 (t, *J* = 7.6 Hz, 3H).

### Example 5

### 1-Fluoro-5-((6-(5-(((4-(methoxymethyl)pyrimidin-2-yl)oxy)methyl)-1-methyl-1H-1,2,3 -triazol-4-yl)-2-methylpyridin-3-yl)oxy)octahydropentalene-1-carboxylic acid (5)

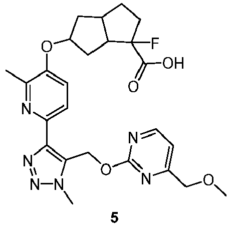

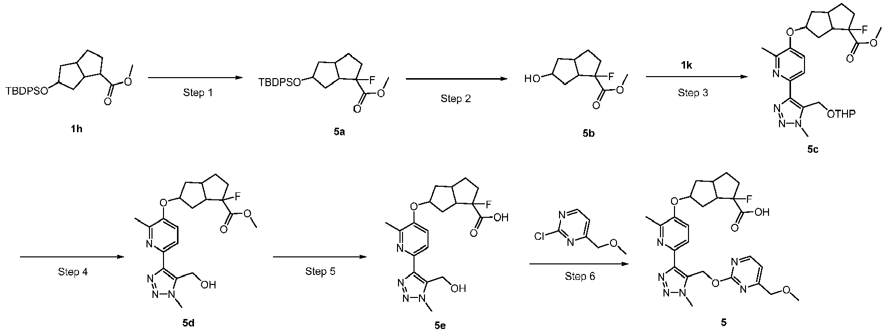

### Step 1: methyl 5-((tert-butyldiphenylsilyl)oxy)-1-fluoro-octahydropentalene-1-carboxylate (5a)

**1h** (1700 mg, 4.02 mmol) was dissolved in anhydrous tetrahydrofuran (50 mL), and the solution was cooled to -78 °C. LDA (4 mL, 2 M in THF) was added to the reaction mixture, and the mixture was left to react at that temperature for 30 min. N-fluorobisbenzenesulfonamide (2536 mg, 48.04 mmol) was added, and the mixture was gradually warmed to room temperature and left to react for 16 h. A saturated ammonium chloride solution was added to the reaction mixture, and the mixture was extracted with ethyl acetate (20 mL × 2). The organic phases were combined, washed with saturated brine, dried over anhydrous sodium sulfate, and filtered. The filtrate was concentrated to give a crude product, and the crude product was purified by reversed-phase flash column chromatography (acetonitrile/water) and then lyophilized to give **5a** (1400 mg, 79% yield).

MS (ESI) m/z = 441.3 [M+H]⁺.

### Step 2: methyl 1-fluoro-5-hydroxyoctahydropentalene-1-carboxylate (5b)

**5a** (1300 mg, 2.95 mmol) was dissolved in tetrahydrofuran (30 mL), and a tetrabutylammonium fluoride solution (4.42 mL, 1 M in THF) was added to the reaction mixture. The mixture was left to react at room temperature for 2 h. The reaction mixture was concentrated under reduced pressure to give a crude product, and the crude product was purified by flash column chromatography (petroleum ether/ethyl acetate) to give **5b** (400 mg, 67% yield).

Step 3: methyl 1-fluoro-5-((2-methyl-6-(1-methyl-5-(((tetrahydro-2*H*-pyran-2-yl)oxy) methyl)-1*H*-1,2,3-triazol-4-yl)pyridin-3-yl)oxy)octahydropentalene-1-carboxylate (5c) **5b** (399 mg, 1.97 mmol), **1k** (600 mg, 1.97 mmol) and triphenylphosphine (1293 mg, 4.93 mmol) were dissolved in tetrahydrofuran (50 mL), and the solution was heated to 50 °C. A solution of di-tert-butyl azodicarboxylate (1125 mg, 4.93 mmol) in tetrahydrofuran (10 mL) was added dropwise, and the mixture was left to react at 50 °C for 2 h. The reaction mixture was concentrated under reduced pressure to give a crude product, and the crude product was purified by reversed-phase flash column chromatography (acetonitrile/water) to give **5c** (600 mg, 62% yield) as a pale yellow oil. MS (ESI) m/z = 489.4 [M+H]⁺.

### Step 4: methyl 1-fluoro-5-((6-(5-(hydroxymethyl)-1-methyl-1H-1,2,3-triazol-4-yl)-2-methylpyridin-3-yl)oxy)octahydropentalene-1-carboxylate (5d)

**5c** (600 mg, 1.23 mmol) was dissolved in methanol (30 mL), and p-toluenesulfonic acid monohydrate (117 mg, 0.61 mmol) was added. The mixture was heated to 70 °C and left to react for 2 h in a nitrogen atmosphere. The reaction mixture was cooled to room temperature and concentrated under reduced pressure to give **5d** (crude, 450 mg), and the product was directly used in the next step.

MS (ESI) m/z = 405.7 [M+H]⁺.

### Step 5: 1-fluoro-5-((6-(5-(hydroxymethyl)-1-methyl-1H-1,2,3-triazol-4-yl)-2-methylpyridin-3-yl)oxy)octahydropentalene-1-carboxylic acid (5e)

**5d** (450 mg, 1.11 mmol) was dissolved in tetrahydrofuran (3 mL), methanol (3 mL) and water (3 mL), and lithium hydroxide (140 mg, 3.34 mmol) was added. The mixture was left to react at room temperature for 2 h. The reaction mixture was concentrated under reduced pressure to give a crude product, and the crude product was purified by reversed-phase flash column chromatography (water/acetonitrile) to give **5e** (380 mg, 87% yield).

MS (ESI) m/z = 391.3 [M+H]⁺.

### Step 6: 1-fluoro-5-((6-(5-(((4-(methoxymethyl)pyrimidin-2-yl)oxy)methyl)-1-methyl-1H-1,2,3-triazol-4-yl)-2-methylpyridin-3-yl)oxy)octahydropentalene-1-carboxyl ic acid (5)

**5e** (130 mg, 0.33 mmol) was dissolved in N,N-dimethylacetamide (10 mL), and 60% sodium hydride (107 mg, 2.66 mmol) was added under ice bath conditions. After 30 min of reaction, 2-chloro-4-(methoxymethyl)pyrimidine (106 mg, 0.67 mmol) was added, and the mixture was left to react at room temperature for 30 min. The reaction mixture was diluted with water (10 mL), and the pH was adjusted to 4-5 with 1 M hydrochloric acid. The mixture was extracted with ethyl acetate (20 mL × 2). The organic phases were combined, washed with saturated brine, dried over anhydrous sodium sulfate, and filtered. The filtrate was concentrated to give a crude product, and the crude product was purified by reversed-phase flash column chromatography (acetonitrile/water) to give compound **5** (80 mg, 47% yield).

MS (ESI) m/z = 513.9 [M+H]⁺.

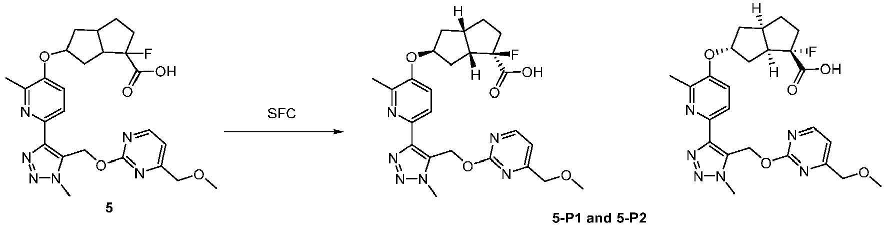

Compound **5** was resolved by preparative chiral SFC (resolution conditions: preparative chiral SFC column DAICEL CHIRALPAK AD (250 mm × 30 mm, 10 µm); mobile phases: A was CO₂, and B was ethanol (0.1% ammonia water); flow rate: 70 mL/min). The corresponding fractions were collected and concentrated under reduced pressure to give compounds **5-P1** and **5-P2.**

Compound **5-P1** was the one with a shorter retention time: MS m/z (ESI): 513.2 [M+H]⁺; chiral SFC analysis: retention time 5.535 min, chiral purity 99.3% (column: Chiralpak AD-3, 0.46 cm I.D. × 150 mm, 3 µm; mobile phases: A was CO₂, and B was ethanol (0.05% diethylamine); flow rate: 2.5 mL/min.

¹H NMR (400 MHz, DMSO-*d*₆) δ 8.62 (d, *J* = 5.0 Hz, 1H), 7.84 (d, *J* = 8.5 Hz, 1H), 7.44 (d, *J* = 8.7 Hz, 1H), 7.16 (d, *J* = 5.0 Hz, 1H), 6.00 (s, 2H), 5.09-4.89 (m, 1H), 4.35 (s, 2H), 4.13 (s, 3H), 3.36 (s, 3H), 2.93-2.68 (m, 2H), 2.20 (s, 3H), 2.17-2.06 (m, 2H), 1.97-1.82 (m, 3H), 1.70-1.47 (m, 3H).

Compound **5-P2** was the one with a longer retention time: MS m/z (ESI): 513.3 [M+H]⁺; chiral SFC analysis: retention time 6.103 min, chiral purity 98.6% (column: Chiralpak AD-3, 0.46 cm I.D. × 150 mm, 3 µm; mobile phases: A was CO₂, and B was ethanol (0.05% diethylamine); flow rate: 2.5 mL/min.

¹H NMR (400 MHz, DMSO-d6) *δ* 8.62 (d, J = 5.0 Hz, 1H), 7.84 (d, J = 8.5 Hz, 1H), 7.44 (d, J = 8.6 Hz, 1H), 7.16 (d, J = 5.0 Hz, 1H), 6.00 (s, 2H), 5.17-4.89 (m, 1H), 4.35 (s, 2H), 4.13 (s, 3H), 3.36 (s, 3H), 2.89-2.63 (m, 2H), 2.19 (s, 3H), 2.19-2.03 (m, 2H), 1.92-1.78 (m, 3H), 1.74-1.65 (m, 1H), 1.59-1.45 (m, 2H).

### Example 6

### 5-((6-(5-(((4-(Difluoromethyl)pyrimidin-2-yl)oxy)methyl)-1-methyl-1H-1,2,3-triazol-4-yl)-2-methylpyridin-3-yl)oxy)-1-fluorooctahydropentalene-1-carboxylic acid (6)

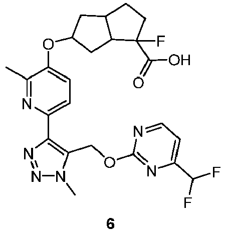

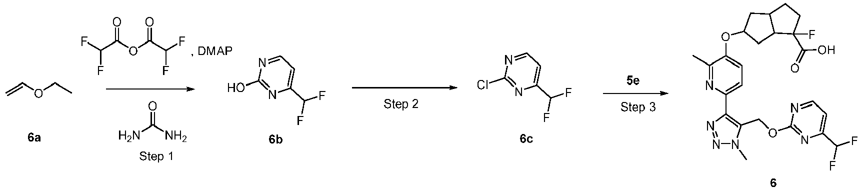

### Step 1: 4-(difluoromethyl)pyrimidin-2-ol (6b)

Difluoroacetic anhydride (4823 mg, 27.74 mmol) was dissolved in anhydrous dichloromethane (30 mL), and the solution was cooled to -20 °C. 4-Dimethylaminopyridine (17 mg, 0.14 mmol) was added, and then 6a (1000 mg, 13.87 mmol) was added. During the addition, the temperature was controlled to not exceed -10 °C. The mixture was then slowly warmed to room temperature and left to react for 16 h. The reaction mixture was washed with a saturated sodium bicarbonate solution. The dichloromethane layer was washed with saturated brine, dried over anhydrous sodium sulfate, and filtered. The filtrate was concentrated to give an oil. The oil was dissolved in ethanol (50 mL), and urea (1666 mg, 27.74 mmol) and concentrated hydrochloric acid (12 mL) were added under ice bath conditions. After dropwise addition, the mixture was warmed to room temperature and left to react for 16 h. The reaction mixture was concentrated under reduced pressure to remove the solvent and extracted with ethyl acetate (20 mL × 2). The organic phases were combined, washed with saturated brine, dried over anhydrous sodium sulfate, and filtered. The filtrate was concentrated to give a crude product, and the crude product was purified by reversed-phase flash column chromatography (acetonitrile/water) to give **6b** (1500 mg, 74% yield).

MS (ESI) m/z = 147.3 [M+H]⁺.

### Step 2: 2-chloro-4-(difluoromethyl)pyrimidine (6c)

**6b** (1500 mg, 10.27 mmol) was dissolved in phosphorus oxychloride (10 mL), and the solution was heated to 105 °C and left to react for 2 h. The reaction mixture was concentrated under reduced pressure to remove the solvent and extracted with ethyl acetate (20 mL × 2). The organic phases were combined, washed with saturated brine, dried over anhydrous sodium sulfate, and filtered. The filtrate was concentrated to give **6c** (crude, 700 mg, 41% yield).

MS (ESI) m/z = 165.2 [M+H]⁺.

### Step 3: 5-((6-(5-(((4-(difluoromethyl)pyrimidin-2-yl)oxy)methyl)-1-methyl-1H-1,2,3-triazol-4-yl)-2-methylpyridin-3-yl)oxy)-1-fluorooctahydropentalene-1-carboxylic acid (6)

**5e** (130 mg, 0.33 mmol) was dissolved in N,N-dimethylacetamide (10 mL), and 60% sodium hydride (107 mg, 2.66 mmol) was added under ice bath conditions. After 30 min of reaction, **6c** (71 mg, 0.43 mmol) was added, and the mixture was left to react at room temperature for 30 min. The reaction mixture was diluted with water (10 mL), and the pH was adjusted to 4-5 with 1 M hydrochloric acid. The mixture was extracted with ethyl acetate (20 mL × 2). The organic phases were combined, washed with saturated brine, dried over anhydrous sodium sulfate, and filtered. The filtrate was concentrated to give a crude product, and the crude product was purified by reversed-phase flash column chromatography (acetonitrile/water) to give the title compound 6 (20 mg, 11% yield).

MS (ESI) m/z = 519.8 [M+H]⁺.

¹H NMR (400 MHz, CDCl₃) *δ* 8.71 (d, *J* = 4.9 Hz, 1H), 7.91 (d, *J* = 8.6 Hz, 1H), 7.29-7.26 (m, 1H), 7.11 (d, *J* = 8.6 Hz, 1H), 6.43-6.09 (m, 3H), 4.92-4.82 (m, 1H), 4.19 (s, 3H), 3.08-2.92 (m, 2H), 2.38-2.26 (m, 4H), 2.17-1.96 (m, 3H), 1.80-1.68 (m, 1H), 1.65-1.47 (m, 2H), 1.35-1.25 (m, 1H).

### Example 7

### 5-((6-(5-(((4-(Difluoromethyl)pyrimidin-2-yl)oxy)methyl)-1-methyl-1H-1,2,3-triazol-4-yl)-2-methylpyridin-3-yl)oxy)octahydropentalene-1-carboxylic acid (7)

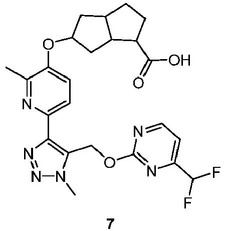

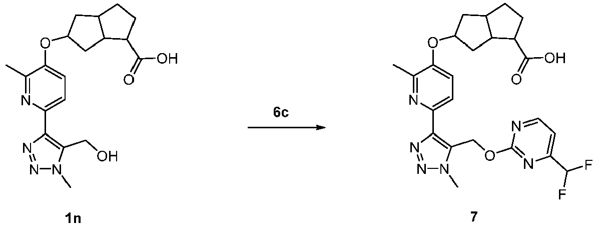

**1n** (100 mg, 0.27 mmol) was dissolved in N,N-dimethylacetamide (10 mL), and 60% sodium hydride (86 mg, 2.15 mmol) was added under ice bath conditions. After 30 min of reaction, **6c** (88 mg, 0.54 mmol) was added, and the mixture was left to react at room temperature for 30 min. The reaction mixture was diluted with water (10 mL), and the pH was adjusted to 4-5 with 1 M hydrochloric acid. The mixture was extracted with ethyl acetate (20 mL × 2). The organic phases were combined, washed with saturated brine, dried over anhydrous sodium sulfate, and filtered. The filtrate was concentrated to give a crude product, and the crude product was purified by reversed-phase flash column chromatography (acetonitrile/water) to give the title compound 7 (10 mg, 7.4% yield).

MS (ESI) m/z = 501.5 [M+H]⁺.

¹H NMR (400 MHz, CDCl₃) *δ* 8.71 (d, *J* = 4.9 Hz, 1H), 7.93 (d, *J* = 8.5 Hz, 1H), 7.29-7.27 (m, 1H), 7.14 (d, *J* = 8.5 Hz, 1H), 6.41-6.11 (m, 3H), 4.92-4.86 (m, 1H), 4.19 (s, 3H), 3.14-3.02 (m, 1H), 2.93-2.80 (m, 2H), 2.34 (s, 3H), 2.32-2.23 (m, 1H), 2.13-2.04 (m, 1H), 1.89-1.80 (m, 3H), 1.76-1.62 (m, 3H).

### Example 8

### 5-((2-Methyl-6-(1-methyl-5-(((4-(((R)-tetrahydrofuran-3-yl)oxy)pyrimidin-2-yl)oxy)me thyl)-1H-1,2,3-triazol-4-yl)pyridin-3-yl)oxy)octahydropentalene-1-carboxylic acid (8)

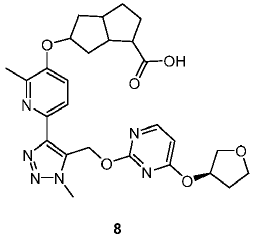

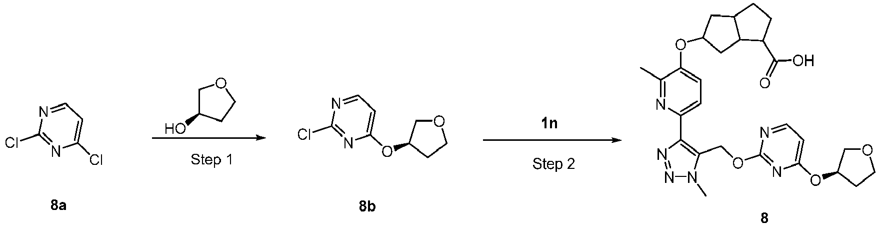

### Step 1: (R)-2-chloro-4-((tetrahydrofuran-3-yl)oxy)pyrimidine (8b) 2,4-Dichloropyrimidine 8a (1000 mg, 6.71 mmol) and (3R)-3-hydroxytetrahydrofuran

(710 mg, 8.05 mmol) were dissolved in N,N-dimethylformamide (10 mL), and cesium carbonate (4374 mg, 13.43 mmol) was added to the reaction mixture. The reaction mixture was heated to 70 °C and left to react for 2 h. Water was added to the reaction mixture, and the mixture was extracted with ethyl acetate (20 mL × 2). The organic phases were combined, washed with saturated brine, dried over anhydrous sodium sulfate, and filtered. The filtrate was concentrated to give a crude product, and the crude product was purified by flash column chromatography (petroleum ether/ethyl acetate) to give **8b** (1200 mg, 89% yield).

MS (ESI) m/z = 201.2 [M+H]⁺.

### Step 2: 5-((2-methyl-6-(1-methyl-5-(((4-(((R)-tetrahydrofuran-3-yl)oxy)pyrimidin-2-yl)oxy)met hyl)-1H-1,2,3-triazol-4-yl)pyridin-3-yl)oxy)octahydropentalene-1-carboxylic acid (8)

**1n** (110 mg, 0.3 mmol) was dissolved in N,N-dimethylacetamide (10 mL), and 60% sodium hydride (94 mg, 2.36 mmol) was added under ice bath conditions. After 30 min of reaction, **8b** (76 mg, 0.38 mmol) was added, and the mixture was left to react at room temperature for 30 min. The reaction mixture was diluted with water (10 mL), and the pH was adjusted to 4-5 with 1 M hydrochloric acid. The mixture was extracted with ethyl acetate (20 mL × 2). The organic phases were combined, washed with saturated brine, dried over anhydrous sodium sulfate, and filtered. The filtrate was concentrated to give a crude product, and the crude product was purified by reversed-phase flash column chromatography (acetonitrile/water) to give the title compound **8** (75 mg, 47% yield).

MS (ESI) m/z = 537.6 [M+H]⁺.

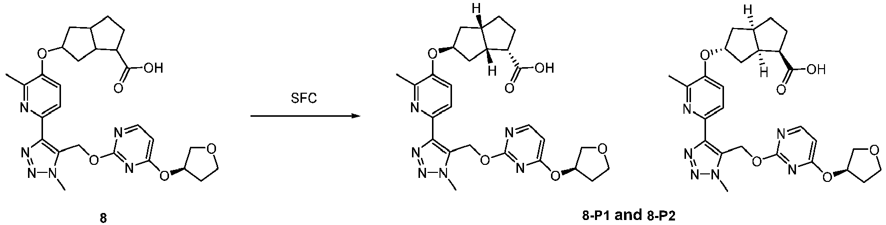

Compound **8** was resolved by preparative chiral SFC (resolution conditions: preparative chiral SFC column DAICEL CHIRALPAK AS (250 mm × 30 mm, 10 µm); mobile phases: A was CO₂, and B was ethanol (0.1% ammonia water)). The corresponding fractions were collected and concentrated under reduced pressure to give compounds **8-P1** and **8-P2.**

Compound **8-P1** was the one with a shorter retention time: MS m/z (ESI): 537.3 [M+H]⁺; chiral SFC analysis: retention time 4.707 min, chiral purity 97.4% (column: Chiralpak AS-3, 0.46 cm I.D. × 150 mm, 3 µm; mobile phases: A was CO₂, and B was ethanol (0.05% diethylamine); flow rate: 2.5 mL/min.

¹H NMR (400 MHz, DMSO-*d*₆) *δ* 8.34 (d, *J* = 5.7 Hz, 1H), 7.86 (d, *J* = 8.5 Hz, 1H), 7.43 (d, *J* = 8.6 Hz, 1H), 6.60 (d, *J* = 5.7 Hz, 1H), 6.00 (s, 2H), 5.46-5.37 (m, 1H), 5.04-4.91 (m, 1H), 4.11 (s, 3H), 3.87-3.64 (m, 4H), 2.94-2.82 (m, 1H), 2.76-2.63 (m, 2H), 2.22 (s, 3H), 2.17-2.04 (m, 2H), 1.99-1.81 (m, 2H), 1.76-1.54 (m, 3H), 1.52-1.35 (m, 3H).

Compound **8-P2** was the one with a longer retention time: MS m/z (ESI): 537.3 [M+H]⁺; chiral SFC analysis: retention time 5.541 min, chiral purity 97.1% (column: Chiralpak AS-3, 0.46 cm I.D. × 150 mm, 3 µm; mobile phases: A was CO₂, and B was ethanol (0.05% diethylamine); flow rate: 2.5 mL/min.

¹H NMR (400 MHz, DMSO-*d*₆) δ 8.34 (d, *J* = 5.7 Hz, 1H), 7.86 (d, *J* = 8.5 Hz, 1H), 7.43 (d, *J* = 8.6 Hz, 1H), 6.60 (d, *J* = 5.7 Hz, 1H), 6.01 (s, 2H), 5.45-5.39 (m, 1H), 5.01-4.90 (m, 1H), 4.11 (s, 3H), 3.86-3.67 (m, 4H), 2.95-2.82 (m, 1H), 2.77-2.64 (m, 2H), 2.22 (s, 3H), 2.17-2.05 (m, 2H), 2.01-1.91 (m, 1H), 1.90-1.81 (m, 1H), 1.74-1.57 (m, 3H), 1.53-1.36 (m, 3H).

### Example 9

### 5-((2-Methyl-6-(1-methyl-5-(((4-(((S)-tetrahydrofuran-3-yl)oxy)pyrimidin-2-yl)oxy)met hyl)-1H-1,2,3-triazol-4-yl)pyridin-3-yl)oxy)octahydropentalene-1-carboxylic acid (9)

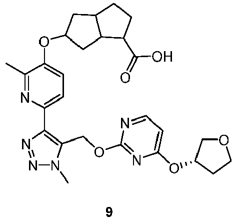

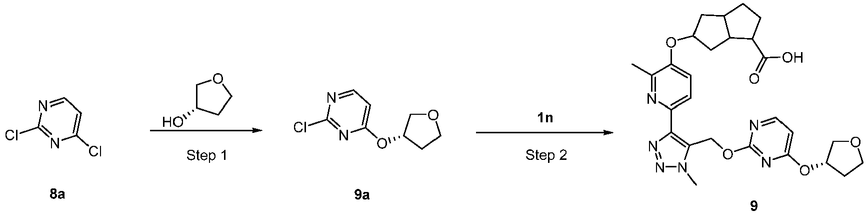

### Step 1: (S)-2-chloro-4-((tetrahydrofuran-3-yl)oxy)pyrimidine (9a)

2,4-Dichloropyrimidine **8a** (1000 mg, 6.71 mmol) and (3S)-3-hydroxytetrahydrofuran (710 mg, 8.05 mmol) were dissolved in N,N-dimethylformamide (10 mL), and cesium carbonate (4374 mg, 13.43 mmol) was added to the reaction mixture. The reaction mixture was heated to 70 °C and left to react for 2 h. Water was added to the reaction mixture, and the mixture was extracted with ethyl acetate (20 mL × 2). The organic phases were combined, washed with saturated brine, dried over anhydrous sodium sulfate, and filtered. The filtrate was concentrated to give a crude product, and the crude product was purified by flash column chromatography (petroleum ether/ethyl acetate) to give **9a** (1200 mg, 89% yield).

MS (ESI) m/z = 201.2 [M+H]⁺.

### Step 2: 5-((2-methyl-6-(1-methyl-5-(((4-(((S)-tetrahydrofuran-3-yl)oxy)pyrimidin-2-yl)oxy)methyl)-1H-1,2,3-triazol-4-yl)pyridin-3-yl)oxy)octahydropentalene-1-carboxyl ic acid (9)

**1n** (110 mg, 0.3 mmol) was dissolved in N,N-dimethylacetamide (10 mL), and 60% sodium hydride (94 mg, 2.36 mmol) was added under ice bath conditions. After 30 min of reaction, **9a** (76 mg, 0.38 mmol) was added, and the mixture was left to react at room temperature for 30 min. The reaction mixture was diluted with water (10 mL), and the pH was adjusted to 4-5 with 1 M hydrochloric acid. The mixture was extracted with ethyl acetate (20 mL × 2). The organic phases were combined, washed with saturated brine, dried over anhydrous sodium sulfate, and filtered. The filtrate was concentrated to give a crude product, and the crude product was purified by reversed-phase flash column chromatography (acetonitrile/water) to give the title compound **9** (75 mg, 47% yield).

MS (ESI) m/z = 537.5 [M+H]⁺.

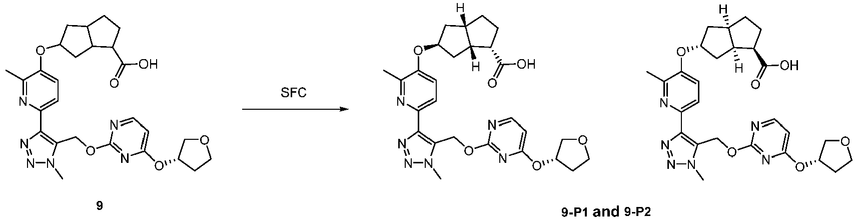

Compound **9** was resolved by preparative chiral SFC (resolution conditions: preparative chiral SFC column DAICEL CHIRALPAK AS (250 mm × 30 mm, 10 µm); mobile phases: A was CO₂, and B was ethanol (0.1% ammonia water)). The corresponding fractions were collected and concentrated under reduced pressure to give compounds **9-P1** and **9-P2.**

Compound **9-P1** was the one with a shorter retention time: MS m/z (ESI): 537.3 [M+H]⁺; chiral SFC analysis: retention time 3.704 min, chiral purity 98.2% (column: Chiralpak AS-3, 0.46 cm I.D. × 150 mm, 3 µm; mobile phases: A was CO₂, and B was ethanol (0.05% diethylamine); flow rate: 2.5 mL/min.

¹H NMR (400 MHz, DMSO-*d*₆) *δ* 8.34 (d, *J* = 5.7 Hz, 1H), 7.86 (d, *J* = 8.5 Hz, 1H), 7.43 (d, *J* = 8.7 Hz, 1H), 6.60 (d, *J* = 5.7 Hz, 1H), 6.09-5.90 (m, 2H), 5.47-5.39 (m, 1H), 5.01-4.95 (m, 1H), 4.11 (s, 3H), 3.84-3.66 (m, 4H), 2.94-2.83 (m, 1H), 2.77-2.63 (m, 2H), 2.22 (s, 3H), 2.18-2.05 (m, 2H), 1.99-1.91 (m, 1H), 1.90-1.81 (m, 1H), 1.73-1.55 (m, 3H), 1.51-1.36 (m, 3H).

Compound **9-P2** was the one with a longer retention time: MS m/z (ESI): 537.3 [M+H]⁺; chiral SFC analysis: retention time 4.1801 min, chiral purity 95.6% (column: Chiralpak AS-3, 0.46 cm I.D. × 150 mm, 3 µm; mobile phases: A was CO₂, and B was ethanol (0.05% diethylamine); flow rate: 2.5 mL/min.

¹H NMR (400 MHz, DMSO-*d*₆) *δ* 8.34 (d, *J* = 5.7 Hz, 1H), 7.86 (d, *J* = 8.6 Hz, 1H), 7.43 (d, *J* = 8.7 Hz, 1H), 6.60 (d, *J* = 5.6 Hz, 1H), 6.00 (s, 2H), 5.46-5.38 (m, 1H), 5.01-4.92 (m, 1H), 4.11 (s, 3H), 3.84-3.64 (m, 4H), 2.96-2.81 (m, 1H), 2.76-2.62 (m, 2H), 2.22 (s, 3H), 2.18-2.05 (m, 2H), 2.00-1.90 (m, 1H), 1.90-1.80 (m, 1H), 1.72-1.54 (m, 3H), 1.52-1.36 (m, 3H).

### Example 10

### 5-((6-(5-(((4-(2-Hydroxypropan-2-yl)pyrimidin-2-yl)oxy)methyl)-1-methyl-1H-1,2,3-tri azol-4-yl)-2-methylpyridin-3-yl)oxy)octahydropentalene-1-carboxylic acid (10)

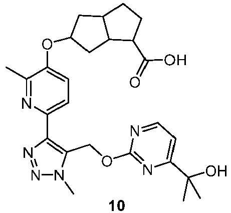

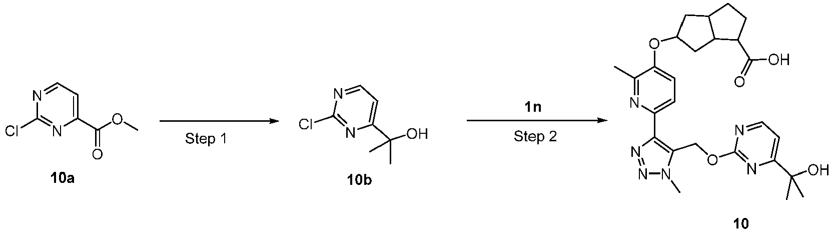

### Step 1: 2-(2-chloropyrimidin-4-yl)propan-2-ol (10b)

Methyl 2-chloropyrimidine-4-carboxylate **10a** (400 mg, 3.32 mmol) was dissolved in anhydrous tetrahydrofuran (30 mL), and the solution was cooled to 0 °C. Methylmagnesium bromide (1.9 mL, 5.80 mmol) was added, and the mixture was left to react for 1 h. Saturated ammonium chloride was added to the reaction mixture, and the mixture was extracted with ethyl acetate (20 mL × 2). The organic phases were combined, washed with saturated brine, dried over anhydrous sodium sulfate, and filtered. The filtrate was concentrated to give a crude product, and the crude product was purified by flash column chromatography (petroleum ether/ethyl acetate) to give **10b** (350 mg, 87% yield).

MS (ESI) m/z = 173.3 [M+H]⁺.

### Step 2: 5-((6-(5-(((4-(2-hydroxypropan-2-yl)pyrimidin-2-yl)oxy)methyl)-1-methyl-1H-1,2,3-tri azol-4-yl)-2-methylpyridin-3-yl)oxy)octahydropentalene-1-carboxylic acid (10)

**1n** (100 mg, 0.27 mmol) was dissolved in N,N-dimethylacetamide (10 mL), and 60% sodium hydride (86 mg, 2.15 mmol) was added under ice bath conditions. After 30 min of reaction, **10b** (70 mg, 0.40 mmol) was added, and the mixture was left to react at room temperature for 30 min. The reaction mixture was diluted with water (10 mL), and the pH was adjusted to 4-5 with 1 M hydrochloric acid. The mixture was extracted with ethyl acetate (20 mL × 2). The organic phases were combined, washed with saturated brine, dried over anhydrous sodium sulfate, and filtered. The filtrate was concentrated to give a crude product, and the crude product was purified by reversed-phase flash column chromatography (acetonitrile/water) to give the title compound **10** (80 mg, 58% yield).

MS (ESI) m/z = 509.7 [M+H]⁺.

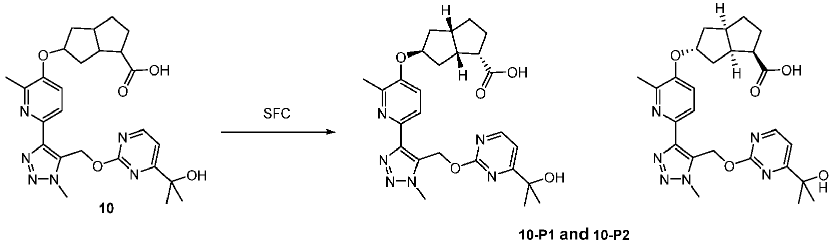

Compound **10** was resolved by preparative chiral SFC (resolution conditions: preparative chiral SFC column DAICEL CHIRALPAK AS (250 mm × 30 mm, 10 µm); mobile phases: A was CO₂, and B was ethanol (0.1% ammonia water); flow rate: 70 mL/min). The corresponding fractions were collected and concentrated under reduced pressure to give compounds **10-P1** and **10-P2.**

Compound **10-P1** was the one with a shorter retention time: MS m/z (ESI): 509.2 [M+H]⁺; chiral SFC analysis: retention time 2.971 min, chiral purity 97.9% (column: Chiralpak AS-3, 0.46 cm I.D. × 100 mm, 3 µm; mobile phases: A was CO₂, and B was ethanol (0.05% diethylamine); flow rate: 2.8 mL/min.

¹H NMR (400 MHz, CD₃OD) *δ* 8.52 (d, *J* = 5.2 Hz, 1H), 7.81 (d, *J* = 8.5 Hz, 1H), 7.43-7.33 (m, 2H), 6.13 (s, 2H), 5.06-4.96 (m, 1H), 4.22 (s, 3H), 3.11-3.00 (m, 1H), 2.91-2.76 (m, 2H), 2.35 (s, 3H), 2.31-2.20 (m, 1H), 2.08-1.98 (m, 1H), 1.91-1.68 (m, 3H), 1.65-1.49 (m, 3H), 1.33 (s, 6H).

Compound **10-P2** was the one with a longer retention time: MS m/z (ESI): 509.3 [M+H]⁺; chiral SFC analysis: retention time 3.310 min, chiral purity 95.4% (column: Chiralpak AS-3, 0.46 cm I.D. × 100 mm, 3 µm; mobile phases: A was CO₂, and B was ethanol (0.05% diethylamine); flow rate: 2.8 mL/min.

¹H NMR (400 MHz, CD₃OD) *δ* 8.52 (d, *J* = 5.2 Hz, 1H), 7.81 (d, *J* = 8.5 Hz, 1H), 7.40-7.35 (m, 2H), 6.13 (s, 2H), 5.03-4.96 (m, 1H), 4.22 (s, 3H), 3.11-2.98 (m, 1H), 2.90-2.76 (m, 2H), 2.35 (s, 3H), 2.29-2.20 (m, 1H), 2.08-1.97 (m, 1H), 1.89-1.67 (m, 3H), 1.64-1.49 (m, 3H), 1.33 (s, 6H).

### Example 11

### 5-((6-(5-(((4-Methoxypyrimidin-2-yl)oxy)methyl)-1-methyl-1H-1,2,3-triazol-4-yl)-2-m ethylpyridin-3-yl)oxy)octahydropentalene-1-carboxylic acid (11)

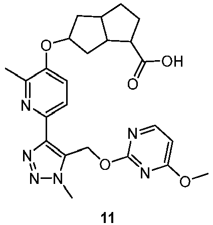

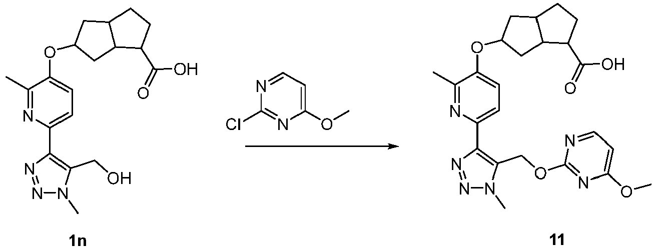

**1n** (100 mg, 0.27 mmol) was dissolved in N,N-dimethylacetamide (10 mL), and 60% sodium hydride (86 mg, 2.15 mmol) was added under ice bath conditions. After 30 min of reaction, 2-chloro-4-methoxypyrimidine (51 mg, 0.35 mmol) was added, and the mixture was left to react at room temperature for 30 min. The reaction mixture was diluted with water (10 mL), and the pH was adjusted to 4-5 with 1 M hydrochloric acid. The mixture was extracted with ethyl acetate (20 mL × 2). The organic phases were combined, washed with saturated brine, dried over anhydrous sodium sulfate, and filtered. The filtrate was concentrated to give a crude product, and the crude product was purified by reversed-phase flash column chromatography (acetonitrile/water) to give the title compound **11** (80 mg, 62% yield).

MS (ESI) m/z = 481.5 [M+H]⁺.

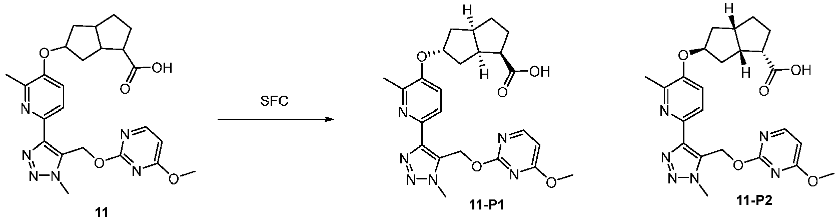

Compound **11** was resolved by preparative chiral SFC (resolution conditions: preparative chiral SFC column DAICEL CHIRALPAK IG (250 mm × 30 mm, 10 µm); mobile phases: A was CO₂, and B was ethanol (0.1% ammonia water)). The corresponding fractions were collected and concentrated under reduced pressure to give compounds **11-P1** and **11-P2.**

Compound **11-P1** was the one with a shorter retention time: MS m/z (ESI): 481.2 [M+H]⁺; chiral SFC analysis: retention time 0.731 min, chiral purity 100% (column: Chiralcel OJ-3, 0.46 cm I.D. × 100 mm, 3 µm; mobile phases: A was CO₂, and B was ethanol (0.05% diethylamine); flow rate: 2.8 mL/min.

¹H NMR (400 MHz, DMSO-*d*₆) δ 8.33 (d, *J* = 5.7 Hz, 1H), 7.86 (d, *J* = 8.5 Hz, 1H), 7.43 (d, *J* = 8.6 Hz, 1H), 6.61 (d, *J* = 5.7 Hz, 1H), 6.01 (s, 2H), 5.01-4.92 (m, 1H), 4.12 (s, 3H), 3.80 (s, 3H), 2.92-2.82 (m, 1H), 2.77-2.65 (m, 2H), 2.22 (s, 3H), 2.14-2.05 (m, 1H), 1.90-1.81 (m, 1H), 1.72-1.54 (m, 3H), 1.51-1.36 (m, 3H).

Compound **11-P2** was the one with a longer retention time: MS m/z (ESI): 481.2 [M+H]⁺; chiral SFC analysis: retention time 0.969 min, chiral purity 99.6% (column: Chiralcel OJ-3, 0.46 cm I.D. × 100 mm, 3 µm; mobile phases: A was CO₂, and B was ethanol (0.05% diethylamine); flow rate: 2.8 mL/min.

¹H NMR (400 MHz, DMSO-*d*₆) *δ* 8.33 (d, *J* = 5.7 Hz, 1H), 7.86 (d, *J* = 8.5 Hz, 1H), 7.43 (d, *J* = 8.7 Hz, 1H), 6.61 (d, *J* = 5.7 Hz, 1H), 6.01 (s, 2H), 5.00-4.94 (m, 1H), 4.12 (s, 3H), 3.80 (s, 3H), 2.94-2.81 (m, 1H), 2.76-2.63 (m, 2H), 2.22 (s, 3H), 2.17-2.06 (m, 1H), 1.90-1.81 (m, 1H), 1.74-1.55 (m, 3H), 1.52-1.36 (m, 3H).

### Example 12

### (1S,3 S)-3-((2-Methyl-6-(1-methyl-5-(((4-(trifluoromethyl)pyrimidin-2-yl)oxy)methyl)-1H-1,2,3-triazol-4-yl)pyridin-3-yl)oxy)cyclohexane-1-carboxylic acid (12)

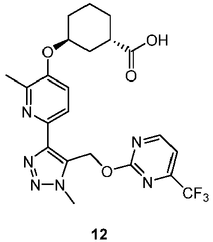

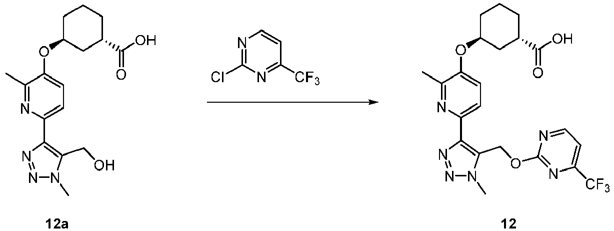

**12a** (550 mg, 1.59 mmol) (obtained by the method of synthesis of Intermediate 43 in WO2019126093 A1) was dissolved in N,N-dimethylacetamide (20 mL), and 60% sodium hydride (305 mg, 12.7 mmol) was added under ice bath conditions. After 30 min of reaction, 2-chloro-4-trifluoromethylpyrimidine (434 mg, 2.38 mmol) was added, and the mixture was left to react at room temperature. The reaction mixture was diluted with water (10 mL), and the pH was adjusted to 5-6 with 1 M hydrochloric acid. The mixture was extracted with ethyl acetate (20 mL × 3). The organic phases were combined, washed with saturated brine, dried over anhydrous sodium sulfate, and filtered. The filtrate was concentrated to give a crude product, and the crude product was purified by reversed-phase flash column chromatography (acetonitrile/water) to give the title compound **12** (523 mg, 67% yield).

MS (ESI) m/z = 493.4 [M+H]⁺.

¹H NMR (400 MHz, DMSO-*d*₆) δ 12.18 (s, 1H), 9.03 (d, *J* = 4.9 Hz, 1H), 7.87 (d, *J* = 8.5 Hz, 1H), 7.70 (d, *J* = 4.9 Hz, 1H), 7.47 (d, *J* = 8.6 Hz, 1H), 6.12 (s, 2H), 4.76 (s, 1H), 4.16 (s, 3H), 2.68-2.55 (m, 1H), 2.20 (s, 3H), 2.05-1.94 (m, 1H), 1.89-1.70 (m, 3H), 1.67-1.40 (m, 4H).

### Example 13

### 5-((6-(5-(((4-(Ethoxymethyl)pyrimidin-2-yl)oxy)methyl)-1-methyl-1H-1,2,3-triazol-4-y l)-2-methylpyridin-3-yl)oxy)octahydropentalene-1-carboxylic acid (13)

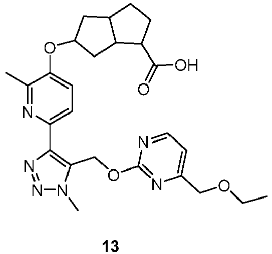

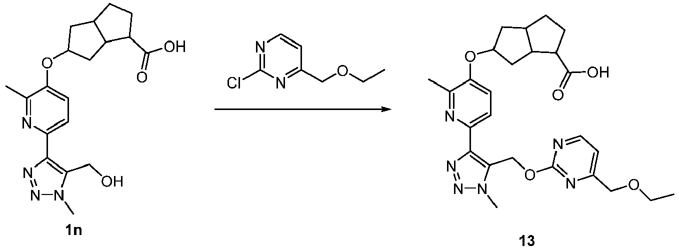

The title compound **13** was synthesized by replacing the starting material "2-chloro-4-(methoxymethyl)pyrimidine" in Example 3 with "2-chloro-4-(ethoxymethyl)pyrimidine".

MS (ESI) m/z = 509.2 [M+H]⁺.

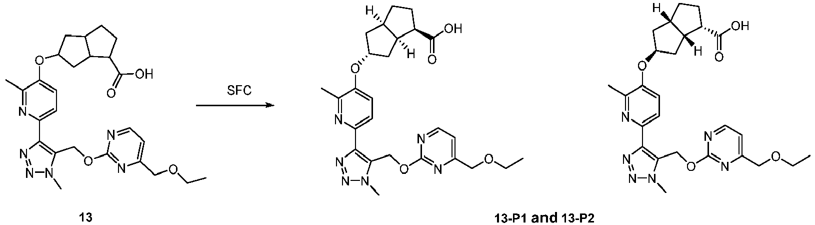

Compound **13** was resolved by preparative chiral SFC (resolution conditions: preparative chiral SFC column DAICEL CHIRALPAK IG (250 mm × 30 mm, 10 µm); mobile phases: A was CO₂, and B was ethanol (0.1% ammonia water)). The corresponding fractions were collected and concentrated under reduced pressure to give compounds **13-P1** and **13-P2.**

Compound **13-P1** was the one with a shorter retention time: MS m/z (ESI): 509.2

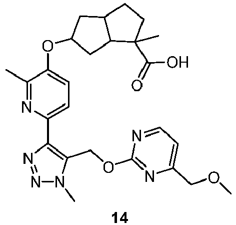

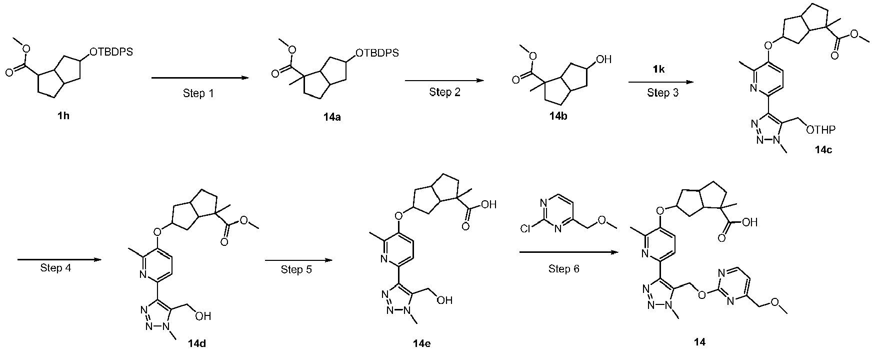

### Step 1: methyl 5-((tert-butyldiphenylsilyl)oxy)-1-methyloctahydropentalene-1-carboxylate (14a)

**1h** (1.2 g, 2.84 mmol) was dissolved in dry tetrahydrofuran (30 mL), and the solution was cooled to -78 °C. 2 M lithium diisopropylamide (4 mL, 8 mmol) was added

[M+H]⁺; chiral SFC analysis: retention time 4.544 min, chiral purity 100% (column: Chiralpak IG-3, 0.46 cm I.D. × 100 mm, 3 µm; mobile phases: A was CO₂, and B was isopropanol (0.05% diethylamine); flow rate: 2.8 mL/min.

¹H NMR (400 MHz, CD₃OD) δ 8.52 (d, *J* = 5.0 Hz, 1H), 7.78 (d, *J* = 8.5 Hz, 1H), 7.38 (d, *J* = 8.5 Hz, 1H), 7.21 (d, *J* = 5.1 Hz, 1H), 6.06 (s, 2H), 4.99 (s, 1H), 4.34 (s, 2H), 4.23 (s, 3H), 3.59 (q, *J* = 7.0 Hz, 2H), 3.10-2.99 (m, 1H), 2.89-2.76 (m, 2H), 2.35 (s, 3H), 2.29-2.21 (m, 1H), 2.07-1.98 (m, 1H), 1.88-1.69 (m, 3H), 1.66-1.49 (m, 3H), 1.25 (t, *J* = 7.0 Hz, 3H).

Compound **13-P2** was the one with a longer retention time: MS m/z (ESI): 509.2 [M+H]⁺; chiral SFC analysis: retention time 5.903 min, chiral purity 100% (column: Chiralpak IG-3, 0.46 cm I.D. × 100 mm, 3 µm; mobile phases: A was CO₂, and B was isopropanol (0.05% diethylamine); flow rate: 2.8 mL/min.

¹H NMR (400 MHz, CD₃OD) δ 8.52 (d, *J* = 5.1 Hz, 1H), 7.78 (d, *J* = 8.5 Hz, 1H), 7.38 (d, *J* = 8.6 Hz, 1H), 7.21 (d, *J* = 5.0 Hz, 1H), 6.06 (s, 2H), 4.99 (s, 1H), 4.35 (s, 2H), 4.23 (s, 3H), 3.59 (q, *J* = 7.0 Hz, 2H), 3.11-3.01 (m, 1H), 2.89-2.78 (m, 2H), 2.35 (s, 3H), 2.30-2.20 (m, 1H), 2.07-1.98 (m, 1H), 1.87-1.49 (m, 6H), 1.25 (t, *J=* 7.0 Hz, 3H).

### Example 14

### 5-((6-(5-(((4-(Methoxymethyl)pyrimidin-2-yl)oxy)methyl)-1-methyl-1H-1,2,3-triazol-4 -yl)-2-methylpyridin-3-yl)oxy)-1-methyloctahydropentalene-1-carboxylic acid (14) dropwise, and after the mixture was stirred at 0 °C for half an hour, iodomethane (1.2 g, 8.52 mmol) was added, and the mixture was left to react at room temperature. An aqueous ammonium chloride solution was added to quench the reaction. The reaction mixture was concentrated, and the crude product was purified by reversed-phase flash column chromatography (acetonitrile/water) to give 14a (900 mg, 72% yield).

MS (ESI) m/z = 437.4 [M+H]⁺.

### Step 2: methyl 5-hydroxy-1-methyloctahydropentalene-1-carboxylate (14b)

**14a** (1 g, 2.29 mmol) was dissolved in tetrahydrofuran (100 mL), and a 1 M solution of tetrabutylammonium fluoride in tetrahydrofuran (4.6 mL, 4.6 mmol) was added. The mixture was left to react at room temperature for 5 h. The reaction mixture was concentrated, and the residue was purified by silica gel flash column chromatography (petroleum ether/ethyl acetate) to give **14b** (200 mg, 44% yield).

Step 3: methyl 1-methyl-5-((2-methyl-6-(1-methyl-5-(((tetrahydro-2*H*-pyran-2-yl)oxy) methyl)-1H-1,2,3-triazol-4-yl)pyridin-3-yl)oxy)octahydropentalene-1-carboxylate (**14c**) **1k** (230 mg, 0.76 mmol), triphenylphosphine (597 g, 2.27 mmol) and **14b** (150 mg, 0.76 mmol) were dissolved in tetrahydrofuran (100 mL), and the solution was heated to 50 °C. A solution of di-tert-butyl azodicarboxylate (522 mg, 2.27 mmol) in tetrahydrofuran (10 mL) was slowly added dropwise, and the mixture was stirred at 50 °C for 2 h. The reaction mixture was concentrated under reduced pressure to give a crude product, and the crude product was purified by reversed-phase C18 column chromatography (acetonitrile/water) to give **14c** (180 mg, 49% yield).

MS (ESI) m/z = 485.3 [M+H]⁺.

### Step 4: methyl 5-((6-(5-(hydroxymethyl)-1-methyl-1H-1,2,3-triazol-4-yl)-2-methylpyridin-3-yl)oxy)-1-methyloctahydropentalene-1-carboxylate (14d)

**14c** (230 mg, 0.48 mmol) was dissolved in methanol (30 mL), and p-toluenesulfonic acid hydrate (45 mg, 0.24 mmol) was added. The reaction mixture was heated to 70 °C and left to react for 2 h in a nitrogen atmosphere. The reaction mixture was cooled and concentrated under reduced pressure to give the title product **14d** (crude, 160 mg). The product was directly used in the next step.

MS (ESI) m/z = 401.3 [M+H]⁺.

### Step 5: 5-((6-(5-(hydroxymethyl)-1-methyl-1H-1,2,3-triazol-4-yl)-2-methylpyridin-3-yl)oxy)-1-methyloctahydropentalene-1-carboxylic acid (14e)

**14d** (160 mg, 0.4 mmol) was dissolved in tetrahydrofuran (3 mL), methanol (3 mL) and water (3 mL), and lithium hydroxide (84 mg, 1.99 mmol) was then added. The mixture was left to react at room temperature. The reaction mixture was concentrated to give a crude product, and the crude product was purified by reversed-phase C18 column chromatography (acetonitrile/water) to give **14e** (130 mg, 84% yield).

MS (ESI) m/z = 387.2 [M+H]⁺.

### Step 6: 5-((6-(5-(((4-(methoxymethyl)pyrimidin-2-yl)oxy)methyl)-1-methyl-1H-1,2,3-triazol-4-yl)-2-methylpyridin-3-yl)oxy)-1-methyloctahydropentalene-1-carboxylic acid (14)

**14e** (130 mg, 0.33 mmol) was dissolved in N,N-dimethylacetamide (10 mL), and 60% sodium hydride (108 mg, 2.69 mmol) was added under ice bath conditions. After 30 min of reaction, 2-chloro-4-(methoxymethyl)pyrimidine (107 mg, 0.67 mmol) was added, and the mixture was stirred at room temperature for 30 min. Water (10 mL) was added, and the pH was adjusted to 5-6 with 1 M hydrochloric acid. The mixture was extracted with ethyl acetate (20 mL × 3). The organic phases were combined, washed with saturated brine, dried over anhydrous sodium sulfate, and filtered. The filtrate was concentrated to give a crude product, and the crude product was purified by reversed-phase flash column chromatography (acetonitrile/water) and lyophilized to give the title product **14** (38 mg, 22% yield).

MS (ESI) m/z = 509.5 [M+H]⁺.

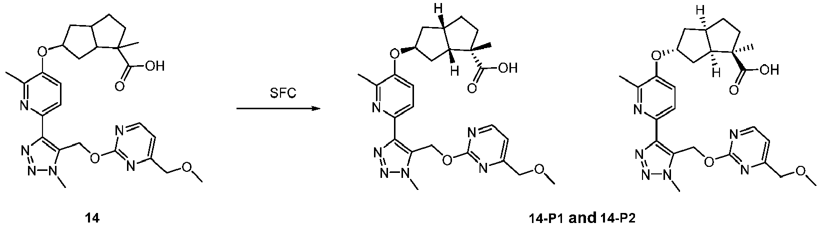

Compound **14** was resolved by preparative chiral SFC (resolution conditions: preparative chiral SFC column DAICEL CHIRALPAK AD (250 mm × 30 mm, 10 µm); mobile phases: A was CO₂, and B was ethanol (0.1% ammonia water)). The corresponding fractions were collected and concentrated under reduced pressure to give compounds **14-P1** and **14-P2.**

Compound **14-P1** was the one with a shorter retention time: MS m/z (ESI): 509.2 [M+H]⁺; chiral SFC analysis: retention time 3.190 min, chiral purity 99.9% (column: Chiralpak AD-3, 0.46 cm I.D. × 150 mm, 3 µm; mobile phases: A was CO₂, and B was ethanol (0.05% diethylamine); flow rate: 2.5 mL/min.

¹H NMR (400 MHz, DMSO-*d*₆) δ 8.62 (d, *J* = 5.0 Hz, 1H), 7.84 (d, *J* = 8.5 Hz, 1H), 7.41 (d, *J* = 8.7 Hz, 1H), 7.16 (d, *J* = 5.0 Hz, 1H), 6.00 (s, 2H), 4.95 (s, 1H), 4.36 (s, 2H), 4.13 (s, 3H), 3.37 (s, 3H), 2.69-2.65 (m, 2H), 2.34-2.31 (m, 2H), 2.20 (s, 3H), 2.15-1.83 (m, 3H), 1.58-1.31 (m, 3H), 1.18 (s, 3H).

Compound **14-P2** was the one with a longer retention time: MS m/z (ESI): 509.2 [M+H]⁺; chiral SFC analysis: retention time 4.538 min, chiral purity 99.3% (column: Chiralpak AD-3, 0.46 cm I.D. × 150 mm, 3 µm; mobile phases: A was CO₂, and B was ethanol (0.05% diethylamine); flow rate: 2.5 mL/min.

¹H NMR (400 MHz, DMSO-*d*₆) δ 8.62 (d, *J* = 5.0 Hz, 1H), 7.84 (d, *J* = 8.6 Hz, 1H), 7.42 (d, *J* = 8.5 Hz, 1H), 7.16 (d, *J* = 5.0 Hz, 1H), 6.00 (s, 2H), 4.95 (s, 1H), 4.36 (s, 2H), 4.13 (s, 3H), 3.37 (s, 3H), 2.69-2.66 (m, 2H), 2.36-2.31 (m, 2H), 2.20 (s, 3H), 2.13-1.80 (m, 3H), 1.57-1.30 (m, 3H), 1.18 (s, 3H).

### Example 15

### 5-((6-(5-(((5-Fluoro-4-(((R)-tetrahydrofuran-3-yl)oxy)pyrimidin-2-yl)oxy)methyl)-1-me thyl-1H-1,2,3-triazol-4-yl)-2-methylpyridin-3-yl)oxy)octahydropentalene-1-carboxylic acid (15)

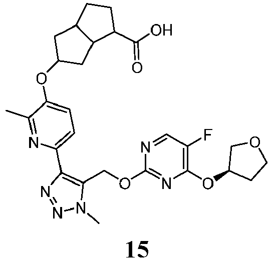

Compound 15 was obtained by referring to the preparation method in Example 8. Compound **15** was resolved by preparative chiral SFC (resolution conditions: preparative chiral SFC column ChiralPak IH (250 mm × 30 mm, 10 µm); mobile phases: A was CO₂, and B was isopropanol (0.1% ammonia water)). The corresponding fractions were collected and concentrated under reduced pressure to give compounds **15-P1** and **15-P2.**

Compound **15-P1** was the one with a shorter retention time: LCMS (ESI) m/z = 555.3 [M+H]⁺; chiral SFC analysis: retention time 1.278 min, chiral purity 100% (column: Chiralpak IH-3, 0.46 cm I.D. × 50 mm, 3 µm; mobile phases: A was CO₂, and B was isopropanol (0.1% isopropylamine); flow rate: 3.4 mL/min; column temperature: 35 °C).

¹H NMR (400 MHz, CD₃OD) δ 8.21 (d, J = 2.7 Hz, 1H), 7.81 (d, J = 8.5 Hz, 1H), 7.39 (d, J = 8.6 Hz, 1H), 6.12-5.97 (m, 2H), 5.46-5.39 (m, 1H), 4.99 (s, 1H), 4.20 (s, 3H), 3.98-3.77 (m, 4H), 3.14-2.98 (m, 1H), 2.88-2.76 (m, 2H), 2.37 (s, 3H), 2.28-1.98 (m, 4H), 1.88-1.66 (m, 3H), 1.67-1.47 (m, 3H).

Compound **15-P2** was the one with a longer retention time: LCMS (ESI) m/z = 555.2 [M+H]⁺; chiral SFC analysis: retention time 1.507 min, chiral purity 100% (column: Chiralpak IH-3, 0.46 cm I.D. × 50 mm, 3 µm; mobile phases: A was CO₂, and B was isopropanol (0.1% isopropylamine); flow rate: 3.4 mL/min; column temperature: 35 °C).

¹H NMR (400 MHz, CD₃OD) δ 8.20 (d, *J* = 2.4 Hz, 1H), 7.82 (d, *J* = 8.1 Hz, 1H), 7.38 (d, *J* = 8.0 Hz, 1H), 6.10-5.97 (m, 2H), 5.45 (s, 1H), 4.97 (s, 1H), 4.21 (s, 3H), 3.97-3.74 (m, 4H), 3.11-3.00 (m, 1H), 2.88-2.76 (m, 2H), 2.38 (s, 3H), 2.27-1.97 (m, 4H), 1.86-1.44 (m, 6H).

### Example 16

### 5-((6-(5-(((5-Chloro-4-(((R)-tetrahydrofuran-3-yl)oxy)pyrimidin-2-yl)oxy)methyl)-1-m ethyl-1H-1,2,3-triazol-4-yl)-2-methylpyridin-3-yl)oxy)octahydropentalene-1-carboxylic acid (16)

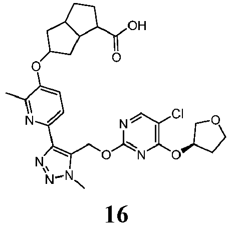

Compound 16 was obtained by referring to the preparation method in Example 8. Compound **16** was resolved by preparative chiral SFC (resolution conditions: preparative chiral SFC column ChiralPak IH (250 mm × 30 mm, 10 µm); mobile phases: A was CO₂, and B was isopropanol (0.1% ammonia water)). The corresponding fractions were collected and concentrated under reduced pressure to give compounds **16-P1** and **16-P2.**

Compound **16-P1** was the one with a shorter retention time: LCMS (ESI) m/z = 571.2 [M+H]⁺; chiral SFC analysis: retention time 1.321 min, chiral purity 100% (column: Chiralpak IH-3, 0.46 cm I.D. × 50 mm, 3 µm; mobile phases: A was CO₂, and B was isopropanol (0.1% isopropylamine); flow rate: 3.4 mL/min; column temperature: 35 °C).

¹H NMR (400 MHz, CD₃OD) δ 8.31 (s, 1H), 7.81 (d, J = 8.5 Hz, 1H), 7.38 (d, J = 8.6 Hz, 1H), 6.06 (s, 2H), 5.50-5.37 (m, 1H), 4.98 (s, 1H), 4.20 (s, 3H), 3.98-3.79 (m, 4H), 3.12-2.99 (m, 1H), 2.88-2.76 (m, 2H), 2.36 (s, 3H), 2.26-1.96 (m, 4H), 1.84-1.47 (m, 6H).

Compound **16-P2** was the one with a shorter retention time: LCMS (ESI) m/z = 571.3 [M+H]⁺; chiral SFC analysis: retention time 1.601 min, chiral purity 99.8% (column: Chiralpak IH-3, 0.46 cm I.D. × 50 mm, 3 µm; mobile phases: A was CO₂, and B was isopropanol (0.1% isopropylamine); flow rate: 3.4 mL/min; column temperature: 35 °C).

¹H NMR (400 MHz, CD₃OD) δ 8.31 (s, 1H), 7.82 (d, J = 8.6 Hz, 1H), 7.38 (d, J = 8.6 Hz, 1H), 6.13-5.99 (m, 2H), 5.50-5.40 (m, 1H), 4.98 (s, 1H), 4.20 (s, 3H), 3.98-3.76 (m, 4H), 3.11-2.97 (m, 1H), 2.87-2.76 (m, 2H), 2.36 (s, 3H), 2.27-1.96 (m, 4H), 1.85-1.46 (m, 6H).

### Example 17

### 5-((6-(5-(((5-Fluoro-4-(((S)-tetrahydrofuran-3-yl)oxy)pyrimidin-2-yl)oxy)methyl)-1-me thyl-1H-1,2,3-triazol-4-yl)-2-methylpyridin-3-yl)oxy)octahydropentalene-1-carboxylic acid (17)

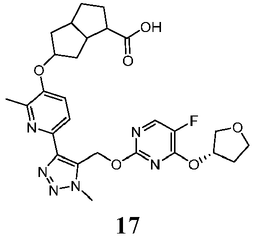

Compound **17** was obtained by referring to the preparation method in Example 8. Compound **17** was resolved by preparative chiral SFC (resolution conditions: preparative chiral SFC column ChiralPak IH (250 mm × 30 mm, 10 µm); mobile phases: A was CO₂, and B was isopropanol (0.1% ammonia water)). The corresponding fractions were collected and concentrated under reduced pressure to give compounds **17-P1** and **17-P2.**

Compound **17-P1** was the one with a shorter retention time, LCMS (ESI) m/z = 555.3 [M+H]⁺; chiral SFC analysis: retention time 1.282 min, chiral purity 95.8% (column: Chiralpak IH-3, 0.46 cm I.D. × 50 mm, 3 µm; mobile phases: A was CO₂, and B was isopropanol (0.1% isopropylamine); flow rate: 3.4 mL/min; column temperature: 35 °C).

¹H NMR (400 MHz, CD₃OD) δ 8.21 (d, J = 2.7 Hz, 1H), 7.81 (d, J = 8.5 Hz, 1H), 7.38 (d, J = 8.6 Hz, 1H), 6.03 (d, J = 2.8 Hz, 2H), 5.51-5.40 (m, 1H), 4.98 (s, 1H), 4.20 (s, 3H), 4.03-3.76 (m, 4H), 3.12-2.98 (m, 1H), 2.88-2.74 (m, 2H), 2.36 (s, 3H), 2.28-1.97 (m, 4H), 1.86-1.46 (m, 6H).

Compound **17-P2** was the one with a longer retention time, LCMS (ESI) m/z = 555.3 [M+H]⁺; chiral SFC analysis: 1.504 min, chiral purity 99.9% (column: Chiralpak IH-3, 0.46 cm I.D. × 50 mm, 3 µm; mobile phases: A was CO₂, and B was isopropanol (0.1% isopropylamine); flow rate: 3.4 mL/min; column temperature: 35 °C).

¹H NMR (400 MHz, CD₃OD) δ 8.21 (d, *J* = 2.7 Hz, 1H), 7.81 (d, *J* = 8.5 Hz, 1H), 7.39 (d, *J* = 8.6 Hz, 1H), 6.03 (s, 2H), 5.47-5.40 (m, 1H), 4.98 (s, 1H), 4.20 (s, 3H), 3.97-3.77 (m, 4H), 3.11-3.00 (m, 1H), 2.84-2.76 (m, 2H), 2.37 (s, 3H), 2.29-1.98 (m, 4H), 1.89-1.47 (m, 6H).

### Example 18

### 5-((6-(5-(((5-Chloro-4-(((S)-tetrahydrofuran-3-yl)oxy)pyrimidin-2-yl)oxy)methyl)-1-me thyl-1H-1,2,3-triazol-4-yl)-2-methylpyridin-3-yl)oxy)octahydropentalene-1-carboxylic acid (18)

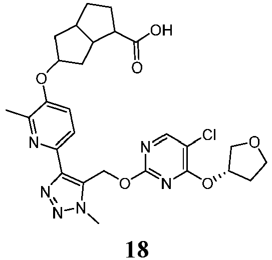

Compound **18** was obtained by referring to the preparation method in Example 8. Compound **18** was resolved by preparative chiral SFC (resolution conditions: preparative chiral SFC column ChiralPak IH (250 mm × 30 mm, 10 µm); mobile phases: A was CO₂, and B was isopropanol (0.1% ammonia water)). The corresponding fractions were collected and concentrated under reduced pressure to give compounds **18-P1** and **18-P2.**

Compound **18-P1** was the one with a shorter retention time, chiral SFC analysis: LCMS (ESI) m/z = 571.2 [M+H]⁺; retention time 1.328 min, chiral purity 99.9% (column: Chiralpak IH-3, 0.46 cm I.D. × 50 mm, 3 µm; mobile phases: A was CO₂, and B was isopropanol (0.1% isopropylamine); flow rate: 3.4 mL/min; column temperature: 35 °C).

¹H NMR (400 MHz, CD₃OD) δ 8.31 (s, 1H), 7.82 (d, *J* = 8.5 Hz, 1H), 7.38 (d, *J* = 8.6 Hz, 1H), 6.06 (s, 2H), 5.50-5.39 (m, 1H), 4.98 (s, 1H), 4.20 (s, 3H), 3.97-3.77 (m, 4H), 3.10-2.98 (m, 1H), 2.87-2.77 (m, 2H), 2.36 (s, 3H), 2.29-1.98 (m, 4H), 1.86-1.68 (m, 3H), 1.65-1.48 (m, 3H).

Compound **18-P2** was the one with a longer retention time, LCMS (ESI) m/z = 571.2 [M+H]⁺; chiral SFC analysis: retention time 1.597 min, chiral purity 99.8% (column: Chiralpak IH-3, 0.46 cm I.D. × 50 mm, 3 µm; mobile phases: A was CO₂, and B was isopropanol (0.1% isopropylamine); flow rate: 3.4 mL/min; column temperature: 35 °C).

¹H NMR (400 MHz, CD₃OD) δ 8.31 (s, 1H), 7.82 (d, *J* = 8.5 Hz, 1H), 7.38 (d, *J* = 8.6 Hz, 1H), 6.11-6.00 (m, 2H), 5.47-5.42 (m, 1H), 4.99-4.96 (m, 1H), 4.20 (s, 3H), 3.96-3.77 (m, 4H), 3.09-2.98 (m, 1H), 2.88-2.79 (m, 2H), 2.36 (s, 3H), 2.27-1.98 (m, 4H), 1.87-1.66 (m, 3H), 1.64-1.47 (m, 3H).

### Biological Assays

### Test Example 1: Testing of Antagonist Properties of Compounds

The antagonist properties of the compounds of the present disclosure were tested using the FLIPR (fluorometric imaging plate reader) method. The compounds are inhibitors of intracellular calcium elevation induced by activation of hLPAR1 (human lysophosphatidic acid receptor 1) expressed in CHO-K1 cells (Chinese hamster ovary K1 cells, HDB).
1. Experimental reagents and instrument consumables

| Reagents and instrument consumables | Supplier | Cat. No. |
|---|---|---|
| CHO-K1 cells | HDB | |
| Nutrient mixture F-12 Ham | Gibco | 21700 |
| Fetal bovine serum (FBS) | biosera | FB-1058/500 |
| Hygromycin B | CALBIOCHEM | 400052-20mL |
| 0.25% pancreatin | Invitrogen | 25200 |
| Calcium ion fluorescent probe Fluo-8 | AAT Bioquest | 21080 |
| Probenecid | Invitrogen | P36400 |
| Tartrazine | Sigma | T0388-100G |
| Acid red | ALDRICH | 210634-25G |
| HBSS (containing Ca/Mg) | Sigma | H1387 |
| HEPES, pH 7.4 | gibco | 15630-080 |
| Bovine serum albumin, free of fatty acids | Proliant | 69700 |
| FLIPR | Molecular Devices | HD-4HYSG2600 |
| Oleoyl-L-α-lysophosphatidic acid sodium salt (LPA) | Sigma | L7260 |

2. Reagent preparation
2.1. Reaction buffer: HBSS + 20 mM HEPES + 0.1% fatty acid-free BSA + 0.001% F-127
2.2. 50× Red dye: (a red dye for blocking background signals in cells) prepared by: weighing out 4 g of tartrazine and 10.2 g of acid red and dissolving them in 100 mL of H₂O.
2.3. Fluo-8 staining mixture: 4 mL of reaction buffer, 32 µL of fluo-8, 320 µL of 50× Red dye, and 40 µL of probenecid.
3. Compound preparation
3.1. LPA: dissolved in DPBS (containing 0.1% fatty acid-free BSA) to prepare a 0.8 µM stock solution, and the solution was aliquoted and stored at -20 °C.
3.2. Test compound preparation: The test compounds were dissolved in DMSO to prepare 10 mM stock solutions, and the solutions were stored at -20 °C. In the experiment, the compounds were first gradient-diluted with DMSO (initial concentration of 50 µM, 3-fold dilution, 10 points) to prepare 200× compound solutions. The compound solutions were then diluted with reaction buffer to give 5× compound solutions, and the solutions were transferred to a 384-well plate (Cat. No. 6008590) using Bravo.
4. Experimental procedure
4.1. CHO-K1/LPA1R cells were cultured in a cell culture medium (F-12 + 10% FBS + 400 µg/mL hygromycin B).
4.2. When the cell confluence reached 80%, the cells were digested with 0.25% pancreatin.
4.3. When the cells became round, the digestion was stopped with the medium F-12 (10% FBS), and the cells were counted. Then the cell culture was diluted with F-12 (10% FBS) to give a cell suspension with a density of 6.7 × 10⁵ cells/mL.
4.4. The cell suspension was added to a 384-well black cell culture plate at 30 µL/well using a Multidrop auto-dispenser, and the plate was incubated in a 37 °C, 5% CO₂ incubator for 20-24 h. The medium was changed to a serum-free F-12 medium, and the cells were starved for 24 h.
4.5. Dose experiment for testing LPA. Gradient dilutions of 6× LPA solution (initial concentration of 60 µM, 3-fold dilution, 10 points) were prepared with reaction buffer. The medium in the 384-well black cell culture plate was discarded and changed to reaction buffer. 10 µL of 5% DMSO reaction buffer was added, and 10 µL of Fluo-8 staining mixture was then added. The plate was incubated in the dark in a 37 °C, 5% CO₂ incubator for 0.5 h.
4.6. FLIPR was correspondingly programmed, the 6× LPA gradient solutions were added, and readings were taken. Two-minute data were collected.
4.7. A reaction curve was obtained from the numerical values of LPA, and the concentration of LPA at EC₈₀ was calculated. A 6× LPA solution was prepared in a 384-well plate (Cat. No. 6008590), and HPE and ZPE were added to corresponding wells. HPE (one hundred per cent effect) was 60 µM LPA, and ZPE (zero per cent effect) was reaction buffer.
4.8. Testing of compounds. The medium in the 384-well black cell culture plate was discarded and changed to reaction buffer. 10 µL of 5× compound solution was transferred using Bravo, and then 10 µL of Fluo-8 staining mixture was added immediately. The plate was incubated in the dark in a 37 °C, 5% CO₂ incubator for 0.5 h.
4.9. FLIPR was correspondingly programmed, the 6× LPA solution was added, and readings were taken. Two-minute data were collected.
4.10. Finally, the output fluorescence counts were analyzed, and IC50 was calculated for the compounds.
5. Experimental results

**Table 1. The IC₅₀ of the compounds of the present disclosure obtained against the receptor LPAR1**

| Compound | LPAR1 IC₅₀ (nM) |
|---|---|
| BMS986278 (shown in Example 104 of the patent application WO2017/223016 A1) | 60.6 |
| **1** | 16.7 (n=2) |
| **1-P1** | 13.3 (n=4) |
| **1-P2** | 659 |
| **2-P1** | 26 |
| **2-P2** | 2689 |
| **3-P1** | 8.7 |
| **3-P2** | 1380 |
| **4-P1** | 746 |
| **4-P2** | 35.8 |
| **5-P1** | 15.6 |
| **5-P2** | 917 |
| **6** | 39.6 |
| **7** | 93.9 |
| **8-P1** | 28 |
| **8-P2** | 502 |
| **9-P1** | 33.4 |
| **9-P2** | 852.8 |
| **10-P1** | 88.7 |
| **10-P2** | 1860 |
| **11-P1** | >10000 |
| **11-P2** | 50.6 |
| **12** | 75 |
| **13-P1** | 1093 |
| **13-P2** | 27.9 |
| **14-P1** | 41.5 |
| **14-P2** | >10000 |

| | |
|---|---|
| Conclusion: The results show that the compounds described above have excellent inhibitory activity against LPAR1, and as LPAR1 antagonists, can be applied in the treatment of diseases related to the LPAR1 target. | |

## Claims

1. A compound represented by formula I-a or a pharmaceutically acceptable salt thereof, 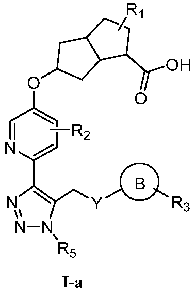 wherein:
R₁ is selected from the group consisting of hydrogen, C₁₋₆ alkyl, 3-6 membered cycloalkyl, C₁₋₆ alkoxy, halogen, cyano, nitro, hydroxy, 3-6 membered heterocyclyl, 5-6 membered heteroaryl, -O-3-6 membered heterocyclyl and -O-5-6 membered heteroaryl, wherein the C₁₋₆ alkyl, 3-6 membered cycloalkyl, C₁₋₆ alkoxy, 3-6 membered heterocyclyl, 5-6 membered heteroaryl, -O-3-6 membered heterocyclyl or -O-5-6 membered heteroaryl is optionally substituted with one or more R₄ₐ;
R₂ is selected from the group consisting of hydrogen, C₁₋₆ alkyl, C₁₋₆ alkoxy, hydroxy, halogen, nitro, cyano, amino, carboxyl, 3-6 membered cycloalkyl, 3-6 membered heterocyclyl, 5-6 membered heteroaryl, -O-3-6 membered heterocyclyl and -O-5-6 membered heteroaryl; the C₁₋₆ alkyl, C₁₋₆ alkoxy, 3-6 membered cycloalkyl, 3-6 membered heterocyclyl, 5-6 membered heteroaryl, -O-3-6 membered heterocyclyl or -O-5-6 membered heteroaryl is optionally substituted with one or more R_{4b};
R₅ is selected from the group consisting of C₁₋₆ alkyl, C₁₋₆ alkoxy, methylenecyclopropyl, halogen, nitro and cyano, wherein the C₁₋₆ alkyl, C₁₋₆ alkoxy or methylenecyclopropyl is optionally substituted with one or more substituents selected from the group consisting of halogen, hydroxy, cyano and nitro;
Y is selected from the group consisting of an oxygen atom and a nitrogen atom;
ring B is selected from the group consisting of 5-6 membered heteroaryl; the 5-6 membered heteroaryl is optionally substituted with 1-3 substituents selected from the group consisting of C₁₋₆ alkyl, C₁₋₆ alkoxy, methylenecyclopropyl, halogen, nitro and cyano, wherein the C₁₋₆ alkyl, C₁₋₆ alkoxy or methylenecyclopropyl is optionally substituted with one or more substituents selected from the group consisting of halogen, hydroxy, cyano and nitro;
R₃ is selected from the group consisting of hydrogen, C₁₋₆ alkyl, C₁₋₆ alkoxy, 3-6 membered cycloalkyl, 3-6 membered heterocyclyl, 5-6 membered heteroaryl, -O-3-6 membered heterocyclyl, -O-5-6 membered heteroaryl, hydroxy, halogen, nitro, cyano, amino and carboxyl; the C₁₋₆ alkyl, C₁₋₆ alkoxy, 3-6 membered cycloalkyl, 3-6 membered heterocyclyl, 5-6 membered heteroaryl, -O-3-6 membered heterocyclyl or -O-5-6 membered heteroaryl is optionally substituted with one or more R_{4c};
each R₄ₐ is independently selected from the group consisting of halogen, methyl, ethyl, methoxy, ethoxy, cyclopropoxy and cyclobutoxy;
each R_{4b} is independently selected from the group consisting of halogen, hydroxy, oxo, nitro, cyano and amino;
each R_{4c} is independently selected from the group consisting of halogen, hydroxy, oxo, nitro, cyano, amino, C₁₋₆ alkyl, C₁₋₆ haloalkyl, C₁₋₆ alkoxy, C₁₋₆ haloalkoxy, C₂₋₆ alkenyloxy, C₂₋₆ alkynyloxy, 3-6 membered cycloalkyl, 3-6 membered heterocyclyl, 3-6 membered heterocycloalkoxy, C₃₋₈ cycloalkenyloxy, 5-6 membered aryl and 3-6 membered heteroaryl.

2. The compound represented by formula I-a or the pharmaceutically acceptable salt thereof according to claim 1, wherein
R₁ is selected from the group consisting of hydrogen, C₁₋₆ alkyl, 3-6 membered cycloalkyl, C₁₋₆ alkoxy, halogen, cyano, nitro and hydroxy, wherein the C₁₋₆ alkyl, 3-6 membered cycloalkyl or C₁₋₆ alkoxy is optionally substituted with one or more R₄ₐ; preferably, R₁ is selected from the group consisting of hydrogen, methyl, trifluoromethyl and halogen;
R₄ₐ is as defined in claim 1.

3. The compound represented by formula I-a or the pharmaceutically acceptable salt thereof according to claim 1, wherein
R₂ is selected from the group consisting of hydrogen, C₁₋₆ alkyl, C₁₋₆ alkoxy, hydroxy, halogen, nitro, cyano, amino, carboxyl and 3-6 membered cycloalkyl; the C₁₋₆ alkyl, C₁₋₆ alkoxy or 3-6 membered cycloalkyl is optionally substituted with one or more R_{4b}; preferably, R₂ is selected from the group consisting of hydrogen, C₁₋₆ alkyl and halogen; the C₁₋₆ alkyl is optionally substituted with one or more R_{4b};
more preferably, R₂ is selected from C₁₋₆ alkyl; the C₁₋₆ alkyl is optionally substituted with one or more R_{4b};
R_{4b} is as defined in claim 1.

4. The compound represented by formula I-a or the pharmaceutically acceptable salt thereof according to claim 1, being a compound represented by formula I'-a or a pharmaceutically acceptable salt thereof, 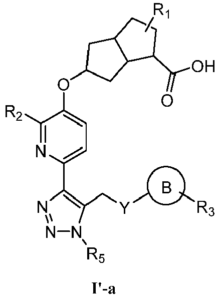 wherein R₁, R₂, R₅, Y, ring B and R₃ are as defined in claim 1.

5. The compound represented by formula I-a or the pharmaceutically acceptable salt thereof according to any of claims 1-4, wherein Y is an oxygen atom.

6. The compound represented by formula I-a or the pharmaceutically acceptable salt thereof according to any of claims 1-5, wherein:
ring B is selected from the group consisting of 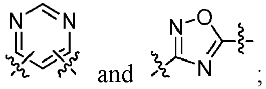 the 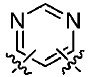 is optionally substituted with 1-3 substituents selected from the group consisting of C₁₋₆ alkyl, C₁₋₆ alkoxy, methylenecyclopropyl, halogen, nitro and cyano, wherein the C₁₋₆ alkyl, C₁₋₆ alkoxy or methylenecyclopropyl is optionally substituted with one or more substituents selected from the group consisting of halogen, hydroxy, cyano and nitro.

7. The compound represented by formula I-a or the pharmaceutically acceptable salt thereof according to any of claims 1-6, wherein:
ring B is selected from the group consisting of: 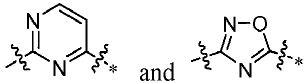 , and R₃ is attached to the * end;
preferably, ring B is selected from 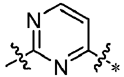 , and R₃ is attached to the * end.

8. The compound represented by formula I-a or the pharmaceutically acceptable salt thereof according to any of claims 1-7, wherein:
R₃ is selected from the group consisting of hydrogen, C₁₋₆ alkyl, C₁₋₆ alkoxy, 3-6 membered cycloalkyl, 3-6 membered heterocyclyl, 5-6 membered heteroaryl, -O-3-6 membered heterocyclyl and -O-5-6 membered heteroaryl; the C₁₋₆ alkyl, C₁₋₆ alkoxy, 3-6 membered cycloalkyl, 3-6 membered heterocyclyl, 5-6 membered heteroaryl, -O-3-6 membered heterocyclyl or -O-5-6 membered heteroaryl is optionally substituted with one or more R_{4c};
preferably, R₃ is selected from the group consisting of hydrogen, C₁₋₆ alkyl, C₁₋₆ alkoxy and 3-6 membered cycloalkyl; the C₁₋₆ alkyl, C₁₋₆ alkoxy or 3-6 membered cycloalkyl is optionally substituted with one or more R_{4c};
R_{4c} is as defined in claim 1.

9. The compound represented by formula I-a or the pharmaceutically acceptable salt thereof according to any of claims 1-8, wherein R₃ is selected from C₁₋₆ alkyl; the C₁₋₆ alkyl is substituted with one or more R_{4c}; preferably, R₃ is selected from methyl; the methyl is substituted with one or more R_{4c};
R_{4c} is as defined in claim 1.

10. The compound represented by formula I-a or the pharmaceutically acceptable salt thereof according to claim 1, wherein R₃ is selected from the group consisting of trifluoromethyl, difluoromethyl, ethyl, cyclopropyl, -CH₂OCH₃, -CH₂OCHF₂, -CH₂OCH₂CH₃,

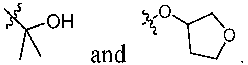

11. The compound represented by formula I-a or the pharmaceutically acceptable salt thereof according to claim 1, wherein:
R₅ is selected from C₁₋₆ alkyl, wherein the C₁₋₆ alkyl is optionally substituted with one or more halogens;
preferably, R₅ is selected from methyl, wherein the methyl is optionally substituted with 1-3 halogens.

12. The compound represented by formula I-a or the pharmaceutically acceptable salt thereof according to claim 1, wherein
each R_{4c} is independently selected from the group consisting of halogen, hydroxy, oxo, nitro, cyano and amino;
or each R_{4c} is independently selected from the group consisting of C₁₋₆ alkyl, C₁₋₆ alkoxy, 3-6 membered cycloalkyl, 3-6 membered heterocyclyl and 3-6 membered heterocycloalkoxy;
or each R_{4c} is independently selected from the group consisting of halogen, methyl, ethyl, methoxy, ethoxy, cyclopropoxy and cyclobutoxy;
or R_{4c} is C₁₋₆ haloalkoxy or C₁₋₆ haloalkyl.

13. The compound represented by formula I-a or the pharmaceutically acceptable salt thereof according to claim 1, wherein the compound represented by formula I-a is selected from the group consisting of

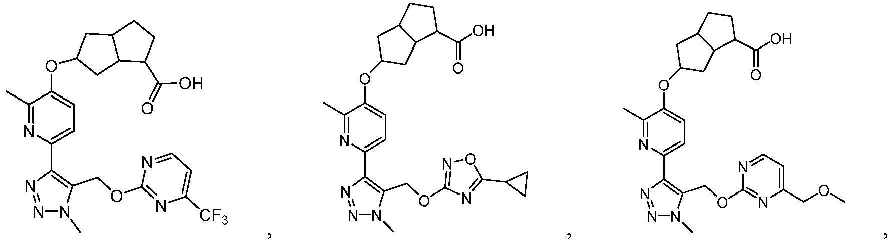

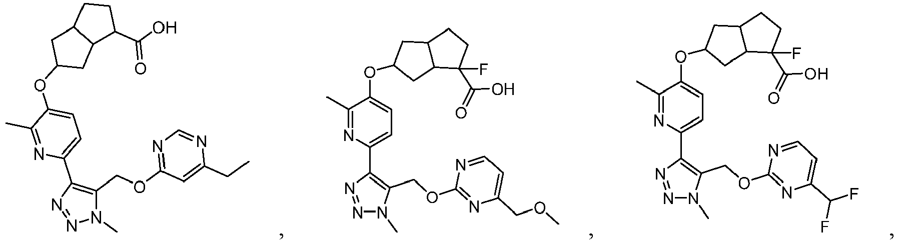

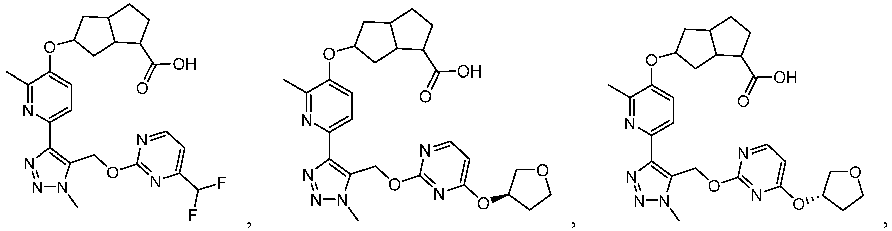

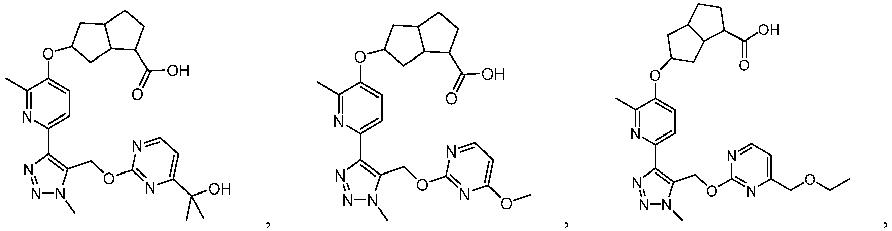

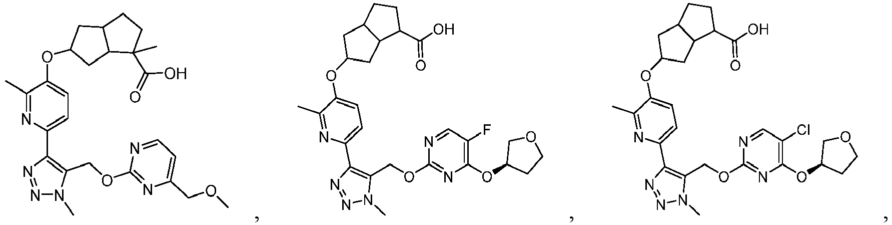

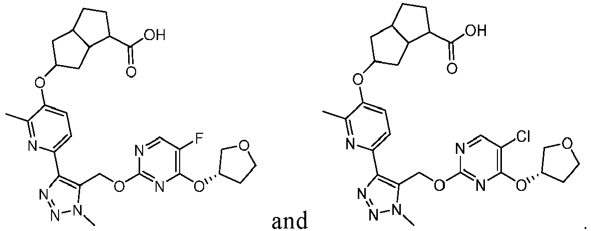

14. The compound represented by formula I-a or the pharmaceutically acceptable salt thereof according to claim 1, wherein the compound represented by formula I-a is selected from the group consisting of:

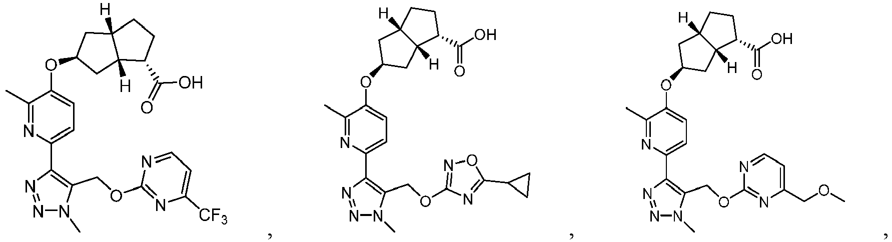

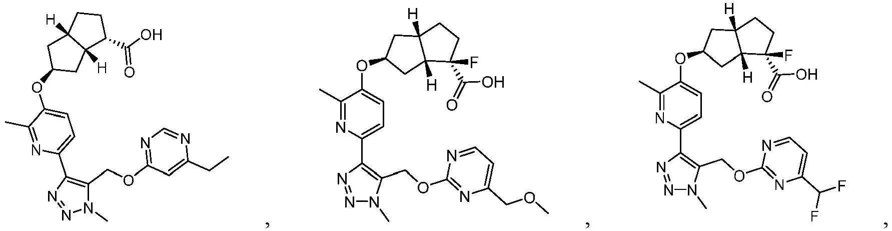

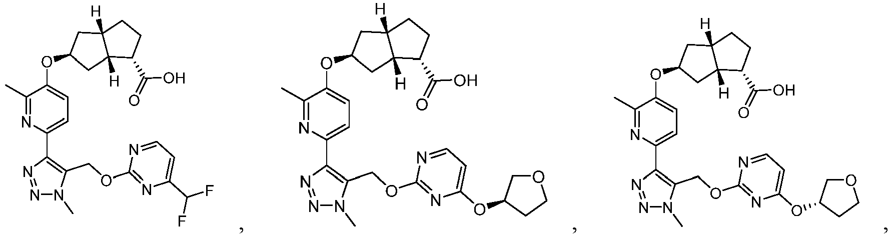

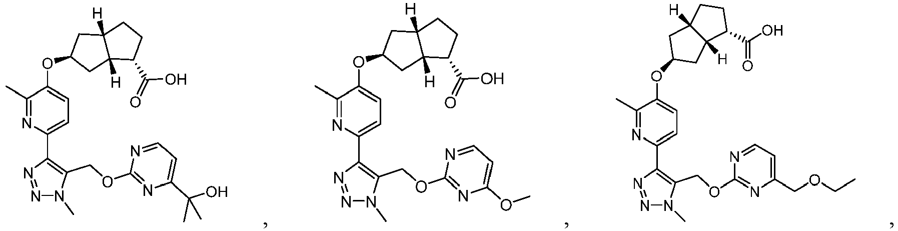

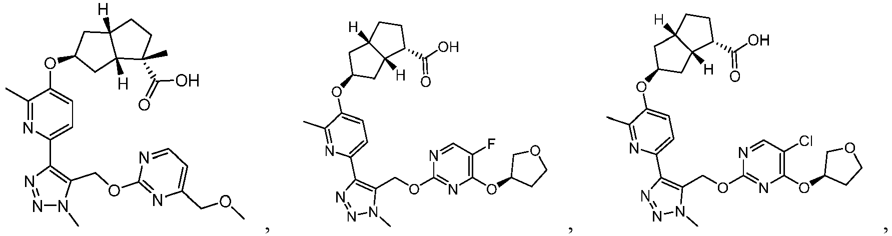

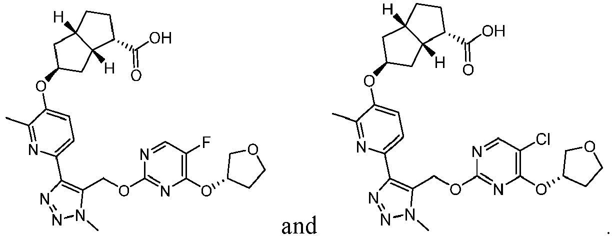

15. An isotopically substituted form of the compound represented by formula I-a according to any of claims 1-14, wherein preferably, the isotopic substitution is a substitution with a deuterium atom.

16. A pharmaceutical composition comprising a therapeutically effective amount of at least one of the compound represented by formula I-a or the pharmaceutically acceptable salt thereof according to any of claims 1-14 or the isotopically substituted form according to claim 15, and a pharmaceutically acceptable excipient.

17. Use of the compound represented by formula I-a or the pharmaceutically acceptable salt thereof according to any of claims 1-14 or the isotopically substituted form according to claim 15 or the pharmaceutical composition according to claim 16 in the preparation of a medicament for preventing and/or treating LPA1-related diseases.

18. Use of the compound represented by formula I-a or the pharmaceutically acceptable salt thereof according to any of claims 1-14 or the isotopically substituted form according to claim 15 or the pharmaceutical composition according to claim 16 in the preparation of a medicament for preventing and/or treating organ fibrotic diseases, respiratory diseases, renal diseases, hepatic diseases, inflammatory diseases, neurological diseases, cardiovascular and cerebrovascular diseases, gastrointestinal diseases, pain, urological diseases, ophthalmic diseases, metabolic diseases, cancer or transplant rejection.
